(19) Europäisches Patentamt
European Patent Office
Office européen des brevets

(11) EP 4 703 472 A1

(12) **EUROPEAN PATENT APPLICATION**
published in accordance with Art. 153(4) EPC

(43) Date of publication:
**04.03.2026 Bulletin 2026/10**

(21) Application number: **24797170.8**

(22) Date of filing: **26.04.2024**

(51) International Patent Classification (IPC):
***C12N 15/11** (2006.01)* ***A61K 31/712** (2006.01)*
***A61K 31/7125** (2006.01)* ***A61K 48/00** (2006.01)*
***C12N 15/10** (2006.01)*

(52) Cooperative Patent Classification (CPC):
**A61K 31/712; A61K 31/7125; A61K 48/00; C12N 15/10; C12N 15/11**

(86) International application number:
**PCT/JP2024/016395**

(87) International publication number:
**WO 2024/225425 (31.10.2024 Gazette 2024/44)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **26.04.2023 JP 2023071927**

(71) Applicant: **Eurus Therapeutics Inc.**
**Fujisawa-shi, Kanagawa 251-8555 (JP)**

(72) Inventors:
- **NOUMI, Takato**
**Kamakura-shi, Kanagawa, 248-0012 (JP)**
- **HAYAMI, Keiko**
**Kamakura-shi, Kanagawa, 248-0024 (JP)**
- **INOUE, Naoki**
**Yokohama-shi, Kanagawa, 244-0003 (JP)**

(74) Representative: **Vossius & Partner Patentanwälte Rechtsanwälte mbB**
**Siebertstraße 3**
**81675 München (DE)**

Remarks:
The complete document including Reference Table(s) and the Sequence Listing(s) can be downloaded from the EPO website

# (54) NON-NATURAL POLYNUCLEOTIDES FOR MODIFICATION OF TARGET NUCLEOTIDE SEQUENCE

(57) The present invention is a non-naturally occurring polynucleotide capable of specifically binding to a target nucleotide sequence for altering one or more nucleotides contained in the target nucleotide sequence in an intracellular double-stranded DNA, wherein the non-naturally occurring polynucleotide contains one or more mismatch nucleotides against the sequence, and has a bridged nucleic acid shown in at least any of the following (A) and (B), and further has features of (C) and (D): (A) one or more nucleotides adjacent to a 5' upstream side of the mismatch nucleotide are a bridged nucleic acid; (B) one or more nucleotides adjacent to a 3' downstream side of the mismatch nucleotide are a bridged nucleic acid; (C) a nucleotide at a 5' terminus is a bridged nucleic acid; and (D) the chain length is from 22 to 95 nt.

EP 4 703 472 A1

## Description

### TECHNICAL FIELD

The present invention relates to a non-naturally occurring polynucleotide capable of specifically binding to a target nucleotide sequence for altering one or more nucleotides contained in the target nucleotide sequence in an intracellular double-stranded DNA.

### BACKGROUND ART

CRISPR-Cas is a genome-editing technology applying an acquired immune mechanism of eubacteria or archaea, which is used as a tool of genetic engineering. CRISPR-Cas9 using CRISPR (Clustered Regularly interspaced short palindromic repeats) sequence which is a DNA sequence and Cas9 which is a DNA-cleaving enzyme of *Streptococcus pyogenes* (Patent Publication 1) is characterized by RNA recognizing a target DNA sequence to introduce a cleavage of a target double-stranded DNA, and a most frequently used genome-editing technology because of its easiness, rapidness and high efficiency. On the other hand, CRISPR-Cas9 has a disadvantage of off-target effects due to a misrecognition of genome sequence by a guide RNA sequence, an occurrence of unexpected mutagenesis at portions other than the target DNA sequence ascribing to a cleavage of a double-stranded DNA, and the like.

Various solutions have been proposed for the off-target effects of CRISPR-Cas. For example, CRISPR-Cas3 allowed mutagenesis with higher specificity by utilizing the fact that the recognition sequence of Cas9 is 20 nucleotides, while the recognition sequence of Cas3 derived from *E. coli* is 27 nucleotides (Patent Publication 2). A method using a complex containing a guide RNA having a DNA sequence-recognizing capability linked to a deaminase converting a nucleic acid base and a mutant Cas nuclease having a deactivated cleavage activity of either one of a double-stranded DNA did not induce a cleavage of a double-stranded DNA and realized genome editing with high safety and specificity as compared to CRISPR-Cas9 (Patent Publication 3).

However, even if the disadvantage of off-target effects could be improved, disadvantages of unexpected risk by an introduction of foreign gene still remains, because the CRISPR-Cas is a technology for introducing a Cas nuclease derived from bacteria or a gene encoding the Cas nuclease into a cell. As genome-editing technology without using Cas nuclease protein, genome-editing technology using a synthetic single-stranded DNA containing a modified nucleic acid has been known.

As an example of a method of altering a target nucleotide sequence in a double-stranded DNA using the synthetic single-stranded DNA in which a part of a nucleotide sequence is replaced with a locked nucleic acid (LNA), a method using at least one mismatch nucleotide and at least two LNA in an acellular experiment has been known, wherein each LNA is an oligonucleotide placed at a distance of at least one nucleotide from at least one mismatch nucleotide (Patent Publication 4).

As other examples of genome-editing technologies not using a Cas nuclease protein, it has been reported that mutations of 1 to 3 nucleotides can be introduced into a target nucleotide sequence by introducing a synthetic single-stranded DNA in which a part of nucleotide sequence is LNA into a mouse ES cell (Non-Patent Publication 1). It has been suggested that the technology of Non-Patent Publication 1 can improve the disadvantage of off-target effects as compared to the genome-editing technology using a Cas nuclease protein. The authors of Non-Patent Publication 1 have analyzed nucleotide sequence of 335 bp region around the target nucleotide sequence for 33 cells in which the intentional alteration of the target nucleotide sequence was confirmed by analysis of nucleotide sequence, and have confirmed that unintentional alteration did not occur at portions other than the target nucleotide sequence. From the results, it has been explained that the synthetic single-stranded DNA used by authors of Non-Patent Publication 1 could achieve very exact genome editing in ES cells derived from a mouse in which a mismatch repair mechanism functions.

Authors of Non-Patent Publication 1 concluded that it is important that a mismatch nucleotide is an LNA for avoiding an intracellular mismatch repair mechanism and altering a target nucleotide sequence, and carried out experiments with an emphasis, and actually carried out experiments using total 60 kinds or more of synthetic single-stranded DNAs in Non-Patent Publication 1, in which the mismatch nucleotide was at least LNA in 41 kinds of synthetic single-stranded DNAs.

Non-Patent Publication 2 is a subsequent report by authors identical to those of Non-Patent Publication 1 which was carried out based on a discovery of Non-Patent Publication 1 that an intracellular mismatch repair mechanism can be avoided when the mismatch nucleotide contained in the synthetic single-stranded DNA was LNA. The subject of Non-Patent Publication 2 is an elucidation of a mechanism of how the synthetic single-stranded DNA having a mismatch nucleotide which is LNA alters a genome of a mammalian cell.

### PRIOR ART REFERENCES

PATENT PUBLICATIONS

Patent Publication 1: Japanese Patent Gazette No. 6343605
Patent Publication 2: Japanese Patent Gazette No. 6480647
Patent Publication 3: Japanese Patent Gazette No. 6206893
Patent Publication 4: Japanese Patent Gazette No. 5405121

NON-PATENT PUBLICATIONS

Non-Patent Publication 1: Thomas W. van Ravesteyn, Marleen Dekker, Alexander Fish, Titia K. Sixma, Astrid Wolters, Rob J. Dekker, and Hein P. J. te Riele (2016) LNA modification of single-stranded DNA oligonucleotides allows subtle gene modification in mismatch-repair-proficient cells, PNAS Vol.113, no.15, 4122-4127 Non-Patent Publication 2: Thomas W. van Ravesteyn, Marcos Arranz Dols, Wietske Pieters, Marleen Dekker, Hein te Riele (2020) Extensive trimming of short single-stranded DNA oligonucleotides during replication-coupled gene editing in mammalian cells. PLoS Genet 16(10): e1009041. https://doi.org/10.1371/journal.pgen.1009041

SUMMARY OF THE INVENTION

PROBLEMS TO BE SOLVED BY THE INVENTION

As genome-editing technology without using Cas nuclease, alterations of a target nucleotide sequence using a synthetic single-stranded DNA containing at least one or more LNA have been reported, but the reports are not said to be sufficient in the point of editing efficiency.

The problems of the present invention are to provide a new non-naturally occurring polynucleotide having structural features which allow an improvement of an editing efficiency in genome-editing technology by a non-naturally occurring polynucleotide regarding a synthetic single-stranded DNA of which a part of a nucleotide sequence is a bridged nucleic acid.

MEANS TO SOLVE THE PROBLEMS

As a result of studying to solve the above problems, the present inventors found that an editing efficiency (yield) of a target nucleotide sequence is improved by using a non-naturally occurring polynucleotide capable of specifically binding to the target nucleotide sequence for altering one or more nucleotides contained in the target nucleotide sequence in an intracellular double-stranded DNA, wherein the non-naturally occurring polynucleotide contains one or more mismatch nucleotides against the target nucleotide sequence, nucleotides other than the mismatch nucleotide contain nucleotides complementary to the target nucleotide sequence, at least one nucleotide adjacent to the mismatch nucleotide or a nucleotide at a 5' terminus is a bridged nucleic acid, and the non-naturally occurring polynucleotide is within the specified range of chain length, and completed the present invention.

In other words, the present invention relates to the following [1] to [38]:

[1] A non-naturally occurring polynucleotide capable of specifically binding to a target nucleotide sequence for altering one or more nucleotides contained in the target nucleotide sequence in an intracellular double-stranded DNA, wherein the non-naturally occurring polynucleotide:

contains one or more mismatch nucleotides against the target nucleotide sequence,
has a bridged nucleic acid shown in at least any of the following (A) and (B), and further has features of (C) and (D):

(A) one or more nucleotides adjacent to a 5' upstream side of the mismatch nucleotide are a bridged nucleic acid;
(B) one or more nucleotides adjacent to a 3' downstream side of the mismatch nucleotide are a bridged nucleic acid;
(C) a nucleotide at a 5' terminus is a bridged nucleic acid; and
(D) the chain length is from 22 to 95 nucleotides.

[2] The non-naturally occurring polynucleotide according to the above [1], wherein the non-naturally occurring polynucleotide further has the following feature of (E):

(E) a nucleotide at a 3' terminus is a bridged nucleic acid.
[3] The non-naturally occurring polynucleotide according to the above [1] or [2], wherein the non-naturally occurring polynucleotide further has the following feature of (F):
(F) one or more nucleotides adjacent to a nucleotide at a 5' terminus are a bridged nucleic acid.
[4] The non-naturally occurring polynucleotide according to any one of the above [1] to [3], wherein the non-naturally occurring polynucleotide further has the following feature of (G):
(G) one or more nucleotides adjacent to a nucleotide at a 3' terminus are a bridged nucleic acid.
[5] The non-naturally occurring polynucleotide according to any one of the above [1] to [4], wherein the non-naturally occurring polynucleotide further has the following feature of (J):
(J) one or more phosphodiester bond moieties between the nucleotides are substituted to a phosphate part modification bond.
[6] The non-naturally occurring polynucleotide according to the above [5], wherein the phosphate part modification bond contains at least one selected from the group consisting of a phosphorothioate bond, a methyl phosphate bond, a boranophosphate bond and a mesyl phosphoramidate bond.
[7] The non-naturally occurring polynucleotide according to any one of the above [1] to [6], wherein the non-naturally occurring polynucleotide further has the following feature of (M):
(M) one or more nucleotides placed between a nucleotide adjacent to a 5' upstream side of the mismatch nucleotide and a nucleotide at a 5' terminus, and placed at a distance of at least 1 nucleotide from both of the nucleotide adjacent to a 5' upstream side of the mismatch nucleotide and the nucleotide at a 5' terminus are a bridged nucleic acid.
[8] The non-naturally occurring polynucleotide according to any one of the above [1] to [7], wherein the non-naturally occurring polynucleotide further has the following feature of (N):
(N) one or more nucleotides placed between a nucleotide adjacent to a 3' downstream side of the mismatch nucleotide and a nucleotide at a 3' terminus, and placed at a distance of at least 1 nucleotide from both of the nucleotide adjacent to a 3' downstream side of the mismatch nucleotide and the nucleotide at a 3' terminus are a bridged nucleic acid.
[9] The non-naturally occurring polynucleotide according to any one of the above [1] to [8], wherein the non-naturally occurring polynucleotide further has the following features of (O) and/or (X2):

(O) a pentose in one or more nucleotides adjacent to the bridged nucleic acid at a 3' terminus is a ribose; and/or
(X2) a pentose in one or more nucleotides adjacent to the bridged nucleic acid at a 5' terminus is a ribose.

[10] The non-naturally occurring polynucleotide according to any one of the above [1] to [9], wherein the non-naturally occurring polynucleotide further has the following feature of (P):
(P) the nucleotide at a 3' terminus is phosphorylated.
[11] The non-naturally occurring polynucleotide according to any one of the above [1] to [10], wherein the bridged nucleic acid contains at least one or more selected from the group consisting of LNA, AmNA, BNA N-H, BNA N-Me and ENA.
[12] The non-naturally occurring polynucleotide according to any one of the above [1] to [11], wherein the non-naturally occurring polynucleotide further has the following feature of (X1):
(X1) a nucleotide at a 3' terminus and/or one or more nucleotides adjacent to the nucleotide at a 3' terminus is a nucleic acid modified at a 2' site.
[13] The non-naturally occurring polynucleotide according to the above [12], wherein the nucleic acid modified at a 2' site contains at least one selected from the group consisting of 2'-F, 2'-OMe, 2'-MOE and 2'-O-(2-carbamoylethyl).
[14] The non-naturally occurring polynucleotide according to any one of the above [1] to [13], wherein the non-naturally occurring polynucleotide further has the following feature of (X4):
(X4) an adapter is added to a 3' terminus.
[15] The non-naturally occurring polynucleotide according to the above [14], wherein the adapter has a function of inhibiting a mismatch repair of a host cell.
[16] The non-naturally occurring polynucleotide according to the above [14] or [15], wherein the adapter has a function of preserving the non-naturally occurring polynucleotide from nuclease digestion.
[17] The non-naturally occurring polynucleotide according to any one of the above [14] to [16], wherein the adapter has a function of giving higher editing efficiency than a non-naturally occurring polynucleotide defined by SEQ ID NO: 9 (described as "GEO-8").
[18] The non-naturally occurring polynucleotide according to any one of the above [14] to [17], wherein the adapter is a nucleotide containing a mismatch nucleotide.
[19] The non-naturally occurring polynucleotide according to any one of the above [14] to [18], wherein the adapter is a nucleotide forming a stem structure which may have a loop.
[20] The non-naturally occurring polynucleotide according to any one of the above [14] to [17], wherein the adapter is a compound other than a nucleotide modifying a terminus (described as "modification compound").

[21] The non-naturally occurring polynucleotide according to the above [20], wherein the molecular weight of the modification compound is 2,000 or less.

[22] The non-naturally occurring polynucleotide according to any one of the above [1] to [21], wherein the non-naturally occurring polynucleotide further has the following feature of (X5):

(X5) one or more nucleotides are inserted between the mismatch nucleotide and the nucleotide at a 3' terminus.

[23] A non-naturally occurring polynucleotide capable of specifically binding to a target nucleotide sequence for altering one or more nucleotides contained in the target nucleotide sequence in an intracellular double-stranded DNA, wherein the non-naturally occurring polynucleotide:

contains one or more mismatch nucleotides against the target nucleotide sequence; and
has a bridged nucleic acid shown in at least any of the following (A) and (B), has a feature of (D), and further has one or more features selected from the group consisting of (H2), (Y1) and (Y3):

(A) one or more nucleotides adjacent to a 5' upstream side of the mismatch nucleotide are a bridged nucleic acid;
(B) one or more nucleotides adjacent to a 3' downstream side of the mismatch nucleotide are a bridged nucleic acid;
(D) the chain length is from 22 to 95 nucleotides;
(H2) a phosphodiester bond moiety between a nucleotide at a 5' terminus and one or more nucleotides adjacent to the nucleotide at a 5' terminus is substituted to a phosphate part modification bond;
(Y1) the nucleotide at a 5' terminus is a mismatch nucleotide, and one or more nucleotides adjacent to the nucleotide at a 5' terminus are a mismatch nucleotide; and
(Y3) an adapter is added to a 5' terminus.

[24] The non-naturally occurring polynucleotide according to the above [23], wherein the adapter has a function of inhibiting a mismatch repair of a host cell.

[25] The non-naturally occurring polynucleotide according to the above [23] or [24], wherein the adapter has a function of preserving the non-naturally occurring polynucleotide from nuclease digestion.

[26] The non-naturally occurring polynucleotide according to any one of the above [23] to [25], wherein the adapter has a function of giving a higher editing efficiency than a non-naturally occurring polynucleotide defined by SEQ ID NO: 9 (GEO-8).

[27] The non-naturally occurring polynucleotide according to any one of the above [23] to [26], wherein the adapter is a modification compound, and the molecular weight of the modification compound is 2,000 or less.

[28] The non-naturally occurring polynucleotide according to any one of the above [23] to [27], wherein the non-naturally occurring polynucleotide further has the following feature of (Y2):

(Y2) a pentose in the nucleotide at a 5' terminus is a ribose, and a pentose in one or more nucleotides adjacent to the nucleotide at a 5' terminus is a ribose.

[29] A non-naturally occurring polynucleotide capable of specifically binding to a target nucleotide sequence for altering one or more nucleotides contained in the target nucleotide sequence in an intracellular double-stranded DNA, wherein the non-naturally occurring polynucleotide:

contains one or more mismatch nucleotides against the target nucleotide sequence, and
has a bridged nucleic acid shown in at least any of the following (A) and (B), and further has features of (D) and (X7):

(A) one or more nucleotides adjacent to a 5' upstream side of the mismatch nucleotide is a bridged nucleic acid;
(B) one or more nucleotides adjacent to a 3' downstream side of the mismatch nucleotide are a bridged nucleic acid;
(D) the chain length is from 22 to 95 nucleotides; and
(X7) one or more nucleotides placed between the mismatch nucleotide and a nucleotide at a 5' terminus is a bridged nucleic acid.

[30] The non-naturally occurring polynucleotide according to the above [29], wherein the bridged nucleic acid contains at least one or more selected from the group consisting of LNA, AmNA, BNA N-H, BNA N-Me and ENA.

[31] A non-naturally occurring polynucleotide capable of specifically binding to a target nucleotide sequence for altering one or more nucleotides contained in the target nucleotide sequence in an intracellular double-stranded DNA, wherein the non-naturally occurring polynucleotide:

contains one or more mismatch nucleotides against the target nucleotide sequence, and has all features of the following (C), (D), (I) and (X6):

(C) a nucleotide at a 5' terminus is a bridged nucleic acid;
(D) the chain length is from 22 to 95 nucleotides;
(I) a phosphodiester bond moiety between a nucleotide at a 3' terminus and one or more nucleotides adjacent to the nucleotide at a 3' terminus is substituted to a phosphorothioate bond; and
(X6) the mismatch nucleotide is a bridged nucleic acid.

[32] The non-naturally occurring polynucleotide according to the above [31], wherein the non-naturally occurring polynucleotide further has the following feature of (E):
(E) the nucleotide at a 3' terminus is a bridged nucleic acid.
[33] The non-naturally occurring polynucleotide according to the above [31] or [32], wherein the non-naturally occurring polynucleotide further has the following feature of (P):
(P) the nucleotide at a 3' terminus is phosphorylated.
[34] A kit for altering a target nucleotide sequence containing the non-naturally occurring polynucleotide as defined in any one of the above [1] to [33].
[35] A pharmaceutical composition containing the non-naturally occurring polynucleotide as defined in any one of the above [1] to [33].
[36] A method of altering one or more nucleotides contained in a target nucleotide sequence in an intracellular double-stranded DNA, wherein: the method includes introducing a non-naturally occurring polynucleotide containing one or more mismatch nucleotides against the target nucleotide sequence into a cell,

the alteration of the target nucleotide sequence contains at least one or more selected from the group consisting of deletion, insertion and substitution of one or more nucleotides of the target nucleotide sequence,
and
the non-naturally occurring polynucleotide is the non-naturally occurring polynucleotide as defined in any one of the above [1] to [33].

[37] The method according to the above [36], wherein the cell is a prokaryotic cell or a eukaryotic cell.
[38] The method according to the above [37], wherein the eukaryotic cell is at least one selected from the group consisting of a plant cell, an insect cell and an animal cell.

## EFFECTS OF THE INVENTION

According to the present invention, a non-naturally occurring polynucleotide having excellent editing efficiency of a target nucleotide sequence is provided.

## BRIEF DESCRIPTION OF THE DRAWINGS

[FIG. 1] FIG. 1 is a schematic showing a summary of an alteration method of a nucleotide to be edited in a target nucleotide sequence using the non-naturally occurring polynucleotide of the present invention.
[FIG. 2] FIG. 2 is a schematic showing an experimentation system for editing a mutation of an inactivated luciferase gene in a genome of 293-nLD1 cells using the non-naturally occurring polynucleotide of the present invention, and detecting an editing efficiency as a luminescence of luciferase.
[FIG. 3A] FIG. 3A is a view showing non-naturally occurring polynucleotides shown in Example 1-1 of the present invention, i.e., a constitution of 12 kinds of non-naturally occurring polynucleotides of GEO-1 to GEO-9, GEO-242, GEO-11 and GEO-94. GEO-94 is a negative control having a nucleotide sequence without a mismatch nucleotide. In the view, G represents a mismatch nucleotide, G attached with asterisk (i.e., "G*") and L indicate that a pentose in the nucleotide is substituted to LNA.
[FIG. 3B] FIG. 3B is a bar graph showing an editing efficiency (%) of non-naturally occurring polynucleotides shown in Example 1-1 of the present invention, i.e., 12 kinds of non-naturally occurring polynucleotides of GEO-1 to GEO-9, GEO-242, GEO-11 and GEO-94.
[FIG. 4] FIG. 4 is a view showing a constitution of non-naturally occurring polynucleotides shown in Example 1-2 of the present invention. In the view, G represents a mismatch nucleotide, and L indicates that a pentose in the nucleotide is substituted to LNA.

[FIG. 5] FIG. 5 is a view showing a constitution of non-naturally occurring polynucleotides shown in Example 1-3 of the present invention. In the view, G represents a mismatch nucleotide, and L indicates that a pentose in the nucleotide is substituted to LNA.

[FIG. 6] FIG. 6 is a view showing a constitution of a non-naturally occurring polynucleotide shown in Example 1-4 of the present invention. In the view, G represents a mismatch nucleotide, and L indicates that a pentose in the nucleotide is substituted to LNA.

[FIG. 7] FIG. 7 is a view showing a constitution of a non-naturally occurring polynucleotide shown in Example 1-5 of the present invention. In the view, G represents a mismatch nucleotide, and L indicates that a pentose in the nucleotide is substituted to LNA.

[FIG. 8A] FIG. 8A to FIG. 8C are views showing constitutions of a non-naturally occurring polynucleotide shown in Example 1-6 of the present invention. FIG. 8A is a view showing a constitution of a non-naturally occurring polynucleotide in which a phosphodiester bond moiety between LNA at a 5' terminus and 1 to 5 nucleotides adjacent thereto is substituted to a phosphorothioate bond. In the view, G represents a mismatch nucleotide, L indicates that a pentose in the nucleotide is substituted to LNA, and S indicates that a phosphodiester bond moiety is substituted to a phosphorothioate bond.

[FIG. 8B] FIG. 8B is a view showing a constitution of a non-naturally occurring polynucleotide in which a phosphodiester bond moiety between a nucleotide at a 3' terminus and 1 to 7 nucleotides adjacent thereto is substituted to a phosphorothioate bond. In the view, G represents a mismatch nucleotide, L indicates that a pentose in the nucleotide is substituted to LNA, and S indicates that a phosphodiester bond moiety is substituted to a phosphorothioate bond.

[FIG. 8C] FIG. 8C is a view showing a constitution of a non-naturally occurring polynucleotide in which a phosphodiester bond moiety between LNA at a 5' terminus and 4 nucleotides adjacent thereto is substituted to a phosphorothioate bond, and a phosphodiester bond moiety between a nucleotide at a 3' terminus and 4 nucleotides adjacent thereto is substituted to a phosphorothioate bond; a constitution of a non-naturally occurring polynucleotide in which a phosphodiester bond moiety between LNA adjacent to a 5' upstream side of a mismatch nucleotide G and 2 or 4 nucleotides adjacent thereto is substituted to a phosphorothioate bond; as well as a constitution of a non-naturally occurring polynucleotide in which a phosphodiester bond moiety between a nucleotide adjacent to LNA adjacent to a 3' downstream side of a mismatch nucleotide G and 2 or 4 nucleotides adjacent to the adjacent nucleotide is substituted to a phosphorothioate bond, respectively. In the view, G represents a mismatch nucleotide, L indicates that a pentose in the nucleotide is substituted to LNA, and S indicates that a phosphodiester bond moiety is substituted to a phosphorothioate bond.

[FIG. 9] FIG. 9 is a view showing a constitution of a non-naturally occurring polynucleotide shown in Example 1-7 of the present invention. In the view, G represents a mismatch nucleotide, L indicates that a pentose in the nucleotide is substituted to LNA, and S indicates that a phosphodiester bond moiety is substituted to a phosphorothioate bond.

[FIG. 10A] FIG. 10A is a view showing a constitution of a non-naturally occurring polynucleotide shown in Example 1-8 of the present invention. In the view, G represents a mismatch nucleotide, and L indicates that a pentose in the nucleotide is substituted to LNA.

[FIG. 10B] FIG. 10B is a view showing a constitution of a non-naturally occurring polynucleotide shown in Example 1-8 of the present invention. In the view, G represents a mismatch nucleotide, and L indicates that a pentose in the nucleotide is substituted to LNA.

[FIG. 11] FIG. 11 is a view showing a constitution of a non-naturally occurring polynucleotide shown in Example 1-9 of the present invention. In the view, G represents a mismatch nucleotide, L indicates that a pentose in the nucleotide is substituted to LNA, and R indicates that a pentose in the nucleotide is substituted to a ribose.

[FIG. 12] FIG. 12 is a view showing a constitution of a non-naturally occurring polynucleotide shown in Example 1-10 of the present invention. In the view, G represents a mismatch nucleotide, L indicates that a pentose in the nucleotide is substituted to LNA, P indicates that a nucleotide at a 5' terminus and/or a nucleotide at a 3' terminus is phosphorylated, and S indicates that a phosphodiester bond moiety is substituted to a phosphorothioate bond.

[FIG. 13] FIG. 13 is a view showing a constitution of a non-naturally occurring polynucleotide shown in Example 1-11 of the present invention. In the view, G represents a mismatch nucleotide, L indicates that a pentose in the nucleotide is substituted to LNA, H indicates that a pentose in the nucleotide is substituted to BNA-N-H, M indicates that a pentose in the nucleotide is substituted to BNA-N-Me, and E indicates that a pentose in the nucleotide is substituted to ENA.

[FIG. 14] A view showing a summary of a constitution of a non-naturally occurring polynucleotide (GEO-226) shown in Example 1-12 of the present invention. In the view, an arrow shows a position of a mismatch nucleotide, L indicates that a pentose in the nucleotide is substituted to LNA, S indicates that a phosphodiester bond moiety is substituted to a phosphorothioate bond, and P indicates that a 3' terminus is phosphorylated.

[FIG. 15] A view showing a summary of a constitution non-naturally occurring polynucleotide (GEO-172) shown in Example 1-13 of the present invention. In the view, an arrow shows a position of a mismatch nucleotide, L indicates that a pentose in the nucleotide is substituted to LNA, S indicates that a phosphodiester bond moiety is substituted to a phosphorothioate bond, R indicates that the nucleotide is subjected to RNA substitution, and P indicates that a 3'

terminus is phosphorylated.

[FIG. 16A] A view showing a summary of constitutions of non-naturally occurring polynucleotides (GEO-602 to GEO-605) shown in Example 3-3 of the present invention. In the view, G represents a mismatch nucleotide, and L indicates that a pentose in the nucleotide is substituted to LNA. In the non-naturally occurring polynucleotide in the view, nucleotides in L, G and the portions of black band are DNA, and nucleotides in the position of white band are RNA.

[FIG. 16B] A view showing a summary of constitutions of non-naturally occurring polynucleotides (GEO-606 and GEO-693 to GEO-695) shown in Example 3-3 of the present invention. In the view, G represents a mismatch nucleotide, and L indicates that a pentose in the nucleotide is substituted to LNA. In the non-naturally occurring polynucleotide in the view, nucleotides in L, G and the positions of black band are DNA, and nucleotides in the position of white band are RNA.

[FIG. 17A] A view showing a summary of constitutions of non-naturally occurring polynucleotides (GEO-696, GEO-697, GEO-701 and GEO-702) shown in Example 3-3 of the present invention. In the view, G represents a mismatch nucleotide, L indicates that a pentose in the nucleotide is substituted to LNA, and S indicates that a phosphodiester bond moiety is substituted to a phosphorothioate bond. In the non-naturally occurring polynucleotide in the view, nucleotides in L, G and the positions of black band are DNA, and nucleotides in the position of white band are RNA.

[FIG. 17B] A view showing a summary of constitutions of non-naturally occurring polynucleotides (GEO-703, GEO-822 and GEO-823) shown in Example 3-3 of the present invention. In the view, G represents a mismatch nucleotide, L indicates that a pentose in the nucleotide is substituted to LNA, and P indicates that a 3' terminus is phosphorylated. In the non-naturally occurring polynucleotide in the view, nucleotides in L, G and the positions of black band are DNA, and nucleotides in the position of white band are RNA.

## MODES FOR CARRYING OUT THE INVENTION

### < Summary of Invention >

In the conventional technique using a single-stranded polynucleotide, the polynucleotide which was introduced into a cell specifically recognizes and binds to a target nucleotide sequence in a lagging strand of the replicating folk at the time of replication of the double-stranded DNA, and acts as a primer for synthesis of Okazaki fragments by a DNA polymerase. As such, a genome sequence is edited by incorporating a polynucleotide into a newborn DNA strand. On the other hand, since a mismatch repair mechanism which repairs a mismatch occurred at the time of replication of DNA is provided in a cell of an organism, a mismatch by the exogenously introduced polynucleotide is also disadvantageously repaired immediately. A correction of an edition by this mismatch repair mechanism is considered as one of the reasons why an editing efficiency of an alteration of a target nucleotide sequence by a polynucleotide which is exogenously introduced into a cell is lower as compared to an alteration of a target nucleotide sequence using nuclease.

On the other hand, it is assumed that the non-naturally occurring polynucleotide of the present invention has a bridged nucleic acid in the specified position in the non-naturally occurring polynucleotide, and therefore, an edition is accelerated, and the influence by the mismatch repair mechanism is concomitantly reduced, thereby being capable of significantly increasing the editing efficiency of the target nucleotide sequence than that of conventional reports.

In the present invention, the efficiency of alteration of the target nucleotide sequence can be further elevated by properly combining, for example, the number and position of the mismatch nucleotide, the number and position of the bridged nucleic acid, the chain length of the polynucleotide, a sort of a bridged nucleic acid, substitution of a phosphodiester bond moiety to a phosphate part modification bond (for example, phosphorothioate bond) between one or more nucleotides, substitution of a pentose to a ribose in one or more nucleotides (may be abbreviated as "RNA substitution" herein), phosphorylation of a 3' terminus or a 5' terminus, addition of an adapter, and the like in the non-naturally occurring polynucleotide.

### < Nucleotide >

In the present invention, "nucleotide" refers to a generic name of substances in which a phosphate group is bonded to a nucleoside. A nucleoside contains a purine base or a pyrimidine base which is glycoside bonded to a position 1 of a pentose. A linear biopolymer containing a nucleotide as a unit is a polynucleotide (also referred to as "nucleic acid"). The polynucleotide includes two kinds of deoxyribonucleotide (DNA) and ribonucleotide (RNA). DNA is consisted of 4 kinds of nucleotides of adenosine monophosphate (AMP, hereinafter referred to as "A"), guanosine monophosphate (GMP, hereinafter referred to as "G"), cytidine monophosphate (CMP, hereinafter referred to as "C") and thymidine monophosphate (dTMP, hereinafter referred to as "T"). In RNA, inclusion of A, G and T is common to DNA, but uridine monophosphate (UMP, hereinafter referred to as "U") is included instead of T.

< Double-Stranded DNA >

In the present invention, "double-stranded DNA" refers to DNA in which single-stranded DNAs having complementary nucleotide sequence to each other form a hydrogen bond between bases in a direction opposing to each other, thereby forming a double helix. The double-stranded DNA in the present invention includes, but not particularly limited to, genomic DNA, mitochondria DNA, chloroplast DNA and the like.

In addition, in the present invention, the double-stranded DNA containing "target nucleotide sequence" includes preferably a gene associated with genetic disease, and more preferably a gene associated with human genetic disease.

The gene associated with genetic disease includes, for example, a gene associated with adrenal leukodystrophy (ABCD 1), a gene associated with medium-chain acyl-CoA dehydrogenase deficiency (ACADM), a gene associated with Wilson disease (ATP7B), a gene associated with heredity pulmonary arterial hypertension (BMPR2), a gene associated with heredity pulmonary arterial hypertension (BMPR2), a gene associated with X-linked agammaglobulinemia (BTK), a gene associated with cystinuria (CTNS), a gene associated with Duchenne muscular dystrophy (DMD), a gene associated with hemophilia A (F8), a gene associated with hemophilia B (F9), a gene associated with tyrosinemia (FAH), a gene associated with hepatic glycogen storage disease type Ia (G6PC), a gene associated with familial frontotemporal lobar degeneration (GRN), a gene associated with mucopolysaccharidosis type II (IDS), a gene associated with mucopolysaccharidosis type I (IDUA), a gene associated with primary immunodeficiency syndrome (IKBKB), a gene associated with familial hypertrophic cardiomyopathy (MYH7), a gene associated with peroxisome biogenesis disorder (PEX2), a gene associated with hepatic glycogen storage disease type IX (PHKA2), a gene associated with protein C deficiency (PROC), a gene associated with hepatic glycogen storage disease type V (PYGM), a gene associated with familial dilated cardiomyopathy (RBM20), a gene associated with $\alpha$1 antitrypsin deficiency (SERPINA1), a gene associated with citrin deficiency (SLC25A13), a gene associated with cystinuria (SLC7A9), a gene associated with Niemann-Pick disease (SMPD1), a gene associated with amyotrophic lateral sclerosis (SOD1) and the like. Incidentally, all these diseases and genes are human diseases and genes.

< Complementary Nucleotide and Mismatch Nucleotide >

In the double-stranded DNA, G and C, or A and T are bonded by a hydrogen bond such that a combination of a purine base and a pyrimidine base is generally formed. A nucleotide to be paired with the specified nucleotide is referred to as "complementary nucleotide."

In the present invention, "mismatch nucleotide" refers to a nucleotide which cannot form a hydrogen bond between two bases in Watson-Crick model, or a matter that a hydrogen bond cannot be formed between two bases in Watson-Crick model due to absence of corresponding nucleotides. In other words, "a nucleotide which cannot form a hydrogen bond between two bases in Watson-Crick model" is nucleotides other than C against G, nucleotides other than T against A, nucleotides other than A against T, and nucleotides other than G against C. "Matter that a hydrogen bond cannot be formed between two bases in Watson-Crick model due to absence of corresponding nucleotides" refers to absence of corresponding nucleotides to all of A, T, G and C against G, absence of corresponding nucleotides to all of A, T, G and C against A, absence of corresponding nucleotides to all of A, T, G and C against T, and absence of corresponding nucleotides to all of A, T, G and C against G.

For the non-naturally occurring polynucleotide of the present invention, one or more "mismatch nucleotide" for altering a nucleotide to be edited exist, and further one or more "mismatch nucleotide" not relating to an alteration may exist. The latter "mismatch nucleotide" is a mismatch nucleotide for the purpose of improvement of an editing efficiency, and, for example, a mismatch nucleotide inserted between a mismatch nucleotide for the purpose of alteration and a nucleotide at a 3' terminus and a mismatch nucleotide in a nucleotide in the case where an adapter is a nucleotide fall under the mismatch nucleotide.

< Alteration of Target Nucleotide Sequence >

In the present invention, "target nucleotide sequence" is a nucleotide sequence to which a non-naturally occurring polynucleotide specifically binds, as explained below. Here, the term "specifically bind" includes the following Embodiment 1 and Embodiment 2.

Embodiment 1: The case where the non-naturally occurring polynucleotide has only a mismatch with a nucleotide to be edited means a binding to a sequence complementary to the non-naturally occurring polynucleotide except for mismatch with the nucleotide to be edited.

Embodiment 2: The case where the non-naturally occurring polynucleotide has mismatches other than the mismatch with a nucleotide to be edited means a binding to a sequence complementary to the non-naturally occurring polynucleotide except for their mismatch parts.

"Alteration" of a target nucleotide sequence refers to the matter that the specified nucleotide (for example, G) is

substituted to any one of other three nucleotides (A, T or C) for one or more nucleotides of the target nucleotide sequence (hereinafter, referred to as "substitution"), that one or more nucleotides of the target nucleotide sequence are deleted (hereinafter, referred to as "deletion"), or that another nucleotide or nucleotide sequence is inserted between the two specified nucleotides on the target nucleotide sequence (hereinafter, referred to as "insertion"). "Alteration" of the target nucleotide sequence includes occurrence of substitution, deletion and insertion alone, and occurrence of combination thereof.

< Non-naturally occurring Polynucleotide >

In the present invention, "non-naturally occurring polynucleotide" refers to a polynucleotide in which nucleotides other than naturally occurring nucleotides (A, T, G, C or U) (hereinafter, referred to as "non-naturally occurring nucleotide") are included in a nucleotide constituting a polynucleotide. The origin of the non-naturally occurring nucleotide is not particularly limited, and includes ones which are artificially synthesized, ones extracted or purified from a substance containing a non-naturally occurring nucleotide, and the like.

The non-naturally occurring nucleotide includes, but not particularly limited to, a nucleic acid modified at a phosphate part, a nucleic acid modified at a sugar part, a nucleic acid modified at a base part, a nucleic acid in which an adapter is added to a 3' terminus and/or a 5' terminus, and a nucleic acid in which one or more nucleotides are inserted between a mismatch nucleotide and a nucleotide at a 3' terminus. The nucleic acid modified at a phosphate part includes, but not particularly limited to, a nucleic acid in which a phosphodiester bond moiety between one or more nucleotides is substituted to a phosphorothioate bond. A general bond between nucleotides is called as a phosphodiester bond, while a bond sulfated is called as a phosphorothioate bond. In the present invention, a nucleotide having a phosphorothioate bond is referred to as a "phosphorothioated" or "S-addition" nucleotide.

Other examples of the nucleic acid modified at a phosphate part include a nucleic acid having a methyl phosphate bond, a nucleic acid having a boranophosphate bond in which one of the non-bridged oxygen atoms of the phosphodiester bond is substituted to borano group ($BH_3$), a nucleic acid having a mesyl phosphoramidate bond in which one of the non-bridged oxygen atoms of the phosphodiester bond is substituted to - $NSO_2CH_3$, and the like.

The nucleic acid modified at a sugar part includes, but not particularly limited to, a nucleic acid modified at 2' site, a bridged nucleic acid in which 2' site and 4' site are bridged, and the like. The nucleic acid modified at 2' site includes, but not particularly limited to, 2'-F (F-added), 2'-O-Methyl (2'-OMe), 2'-O-Methoxyethyl (2'-MOE), 2'-O-(2-carbamoylethyl), and the like.

The bridged nucleic acid includes, but not particularly limited to, 2'-O,4'-C-methylene-Bridged Nucleic Acid (2',4'-BNA), $BNA^{NC}$, di- or tricyclic bridged nucleic acid, other bridged nucleic acids, and the like. 2',4'-BNA is also called as Locked Nucleic Acid (LNA), and has a structure set forth below.

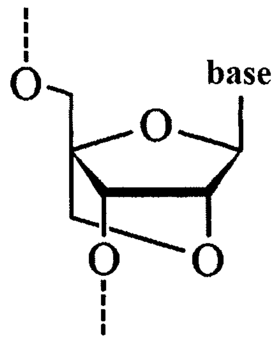

The BNA/LNA analog includes, but not particularly limited to, ethylene-bridged BNA(ENA), amide-bridged BNA(AmNA), 2'-(alkylamino)-LNA, 2'-(acylamino)-LNA, 2'-N-substituted-2'-amino-LNA, $\alpha$-LNA, $\alpha$-L-LNA, $\beta$-D-LNA, 2'-amino-LNA, 2'-thio-LNA, xylo-LNA, 2'-O,4'-C constraint ethyl (cEt)LNA, 2'-O,4'-C constraint methoxyethyl (cMOEt)LNA, carba (cLNA), BNACOC, spirocyclo propylene-bridged nucleic acid (scpBNA), heterocyclic bridged BNA, urea-bridged BNA, sulfonamide-bridged BNA, 5'-methyl substituted BNA, guanidine-bridged nucleic acid (GuNA), 3,4-dihydro-2H-pyran nucleic acid (DpNA), and the like.

$BNA^{NC}$ includes, but not particularly limited to, BNA N-H, BNA N-Me, and BNA N-Bn.

Di- or tricyclic bridged nucleic acid includes, but not particularly limited to, TriNA, $\alpha$-L-TriNA, F-bcDNA, tricyclo DNA (tcDNA), F-tcDNA, dicyclic carbocyclic nucleotide, bicyclo DNA (bcDNA), 2'-C-bridged dicyclic nucleotide (CBBN), and the like.

Other bridged nucleic acids include, but not particularly limited to, oxetane nucleotide, 2'-amino-LNA-derived locked PMO, cyclohexenyl nucleic acid (CeNA), arytriol nucleic acid (ANA), hexitol nucleic acid (HNA), fluoride HNA (F-HNA), pyranosyl-RNA (p-RNA), 3'-deoxy pyranosyl-DNA (p-DNA), and the like.

In the non-naturally occurring polynucleotide of the present invention, when the amount of the bridged nucleic acid is too much, the editing efficiency is disposed to decrease. For example, the proportion of the bridged nucleic acid in the non-naturally occurring polynucleotide of the present invention is preferably from 2 to 30%, more preferably from 2 to 20%, and even more preferably from 2 to 15%, when the number of total nucleotides constituting a non-naturally occurring polynucleotide is defined as 100%.

In the non-naturally occurring polynucleotide of the present invention, it is preferred that higher editing efficiency is exhibited by adding an adapter to a 3' terminus and/or a 5' terminus. The adapter as used herein includes, for example, ones having a function of inhibiting a mismatch repair of a host cell, ones having a function of preserving the non-naturally occurring polynucleotide of the present invention from nuclease digestion, as well as ones having a function of giving higher editing efficiency than the non-naturally occurring polynucleotide defined by SEQ ID NO: 9 (GEO-8) by addition to a 3' terminus and/or a 5' terminus. The adapter in the present invention has one or more functions of these functions, and more specific example of the adapter includes (1) a nucleotide as an adapter, and (2) compounds other than nucleotides modifying termini (as used herein, described as "modification compound").

The assumed mechanism to inhibit mismatch repair of a host cell by adding an adapter to a 3' terminus and/or a 5' terminus of the non-naturally occurring polynucleotide of the present invention is as follows. When one strand of DNA forming a double strand has a 3' terminal structure, the mismatch repair starts from recognition of a strand having 3' terminus by a mismatch repair enzyme in a cell, and then the mismatch nucleotide on the strand having a 3' terminus is removed and repaired. In the state where the non-naturally occurring polynucleotide of the present invention specifically binds to a target sequence, the mismatch nucleotide of the non-naturally occurring polynucleotide is repaired, because the non-naturally occurring polynucleotide has a 3' terminal structure. When a nucleotide is added to a 3' terminus of the non-naturally occurring polynucleotide as an adapter, a possibility that a 3' terminus of the non-naturally occurring polynucleotide avoids the recognition by a mismatch repair enzyme is considered, because the 3' terminus of the non-naturally occurring polynucleotide does not specifically bind to a target sequence and disconnects from the target sequence. Similarly, when the modification compound is added to a 3' terminus of the non-naturally occurring polynucleotide, a possibility that a 3' terminus of the non-naturally occurring polynucleotide avoids the recognition by the mismatch repair enzyme is considered. From the above reasons, it is assumed that an editing efficiency of the non-naturally occurring polynucleotide in which a nucleotide as an adapter or a modification compound is added to a 3' terminus is elevated by inhibiting the mismatch repair of the host cell.

The assumed mechanism to preserve the non-naturally occurring polynucleotide of the present invention from nuclease digestion of a host cell by adding an adapter to a 3' terminus and/or a 5' terminus of the non-naturally occurring polynucleotide of the present invention is as follows. A single-stranded non-naturally occurring polynucleotide introduced into a host cell is a subject of digestion by a nuclease of the host cell. The digestion of the non-naturally occurring polynucleotide by nuclease cannot be completely prevented only by adding a nucleotide as an adapter to a 5' terminus or a 3' terminus of the non-naturally occurring polynucleotide, but it is assumed that the nucleotide as an adapter acts as a buffer, to exhibit an action of inhibiting the progression of digestion by an exonuclease to a mismatch nucleotide placed at center of the non-naturally occurring polynucleotide. On the other hand, the possibility of inhibiting digestion by an exonuclease from a 3' terminus is high by adding a modification compound to a 3' terminus. For example, IdT is utilized to increase a nuclease resistance of an antisense nucleic acid by addition of IdT to a 3' terminus of the antisense nucleic acid.

As described above, a function of inhibiting mismatch repair of a host cell and a function of preserving the non-naturally occurring polynucleotide of the present invention from nuclease digestion are respectively ascribed to different mechanisms, and therefore, the non-naturally occurring polynucleotide of the present invention may have both of these functions.

The structure of the nucleotide as an adapter includes, for example, a linear nucleotide and a nucleotide forming a stem structure which may have a loop. In addition, the chain length of the nucleotide as an adapter is preferably 1 nt (nucleotide) or more, more preferably 3 nt or more, and even more preferably 6 or more, and on the other hand, the chain length is preferably 50 nt or less, more preferably 40 nt or less, and even more preferably 30 or less. Since the non-naturally occurring polynucleotide of the present invention does not contain a nucleotide as an adapter, the chain length of the nucleotide as an adapter does not influence the chain length of the non-naturally occurring polynucleotide of the present invention.

It is preferred that the nucleotide as an adapter is a nucleotide containing a mismatch nucleotide, and it is more preferred that all the nucleotide sequence of the nucleotide as an adapter are mismatched to a sequence of at a 5' upstream side adjacent to a target nucleotide sequence or a sequence at a 3' downstream side adjacent to the target nucleotide sequence, but one or more nucleotides may be complementary to the target nucleotide sequence.

The modification compound as an adapter is preferably a compound which can bind to a 3' terminus and/or a 5' terminus of the non-naturally occurring polynucleotide of the present invention.

The modification compound is preferably a compound having a certain size. For example, the molecular weight of the modification compound is preferably 50 or more, and more preferably 100 or more, and on the other hand, the

molecular weight is preferably 2,000 or less, more preferably 1,500 or less, even more preferably 1,000 or less, even more preferably 800 or less, and even more preferably 500 or less.

The preferred specific example of the modification compound includes at least one selected from the group consisting of fluorescein (FAM), biotin, puromycin, cholesterol, digoxygenin (DIG) and Inverted dT.

The binding of the adapter to the non-naturally occurring polynucleotide of the present invention can be carried out according to known methods.

Since these non-naturally occurring nucleotides having such modification have high affinity to naturally occurring DNA or RNA, an application to nucleic acid medicament and the like has been proceeded.

< Basic Constitution of Non-naturally Occurring Polynucleotide >

The non-naturally occurring polynucleotide of the present invention is a single-stranded polynucleotide, and contains one or more mismatch nucleotides against a target nucleotide sequence, wherein the nucleotides other than the mismatch nucleotide contain a nucleotide complementary to the target nucleotide sequence. Accordingly, the non-naturally occurring polynucleotide of the present invention can specifically bind to the target nucleotide sequence by introducing the non-naturally occurring polynucleotide of the present invention into a cell. Consequently, one or more nucleotides in the target nucleotide sequence can be altered to a desired nucleotide. In the case where the mismatch nucleotide is plural, 2 or more mismatch nucleotides may exist adjacent to each other, and exist at a far position from each other. In the present invention, "plural" regarding the number of the mismatch nucleotide includes 2, 3, 4, 5, 6, 7, 8, 9, 10, 11, 12, 13, 14, 15, 16, 17, 18, 19, 20 or more integers, and includes ranges in which these numbers are defined as an upper limit or a lower limit. For example, the range is 2 to 3, 2 to 4, 2 to 5, ... 2 to 20, 3 to 4, 3 to 5, 3 to 6, ... 3 to 20, 4 to 5, 4 to 6, 4 to 7, ... 4 to 20, 5 to 6, 5 to 7, 5 to 8, ... 5 to 20, ... 18 to 19, 18 to 20 and 19 to 20.

The basic constitution of such the non-naturally occurring polynucleotide of the present invention includes, for example, ones described in the following embodiments (i) to (vi).

(1) Embodiment (i)

One embodiment of the non-naturally occurring polynucleotide of the present invention (Embodiment (i)) is constitutions:

(A) one or more nucleotides adjacent to a 5' upstream side of the mismatch nucleotide are a bridged nucleic acid,
(B) one or more nucleotides adjacent to a 3' downstream side of the mismatch nucleotide are a bridged nucleic acid,
(C) a nucleotide at a 5' terminus is a bridged nucleic acid, and
(D) the chain length is from 22 to 95 nucleotides.

The constitutions can exhibit the effects of the present invention.

The bridged nucleic acid contained in the non-naturally occurring polynucleotide of Embodiment (i) includes, but not particularly limited to, at least one selected from the group consisting of LNA, AmNA, BNA N-H, BNA N-Me and ENA. In the present invention, the bridged nucleic acid of (A) and/or (B) is preferably LNA or BNA N-H, and more preferably LNA. In the present invention, the bridged nucleic acid of (C) is preferably LNA, AmNA, BNA N-H, BNA N-Me and ENA.

Here, the mismatch nucleotide may or may not a bridged nucleic acid, but in Embodiment (i), it is more preferred that the mismatch nucleotide is not a bridged nucleic acid. Among total three nucleotides including a mismatch nucleotide and nucleotides adjacent thereto, the number of the bridged nucleic acid is preferably 2. In addition, "more" in the phrase "more nucleotides are a bridged nucleic acid" defined in the above (A) and (B) is, but not particularly limited, preferably a case where the bridged nucleic acids are continuously two, but not the case of continuously three or more, and a case of one is more preferable.

(2) Embodiment (ii)

Another embodiment of the non-naturally occurring polynucleotide of the present invention (Embodiment (ii)) is constitutions:

(A) one or more nucleotides adjacent to a 5' upstream side of the mismatch nucleotide are a bridged nucleic acid,
(C) a nucleotide at a 5' terminus is a bridged nucleic acid, and
(D) the chain length is from 22 to 95 nucleotides,

provided that the feature of the above (B) is not contained. Such a constitution can also exhibit the effects of the present invention.

The bridged nucleic acid contained in the non-naturally occurring polynucleotide of Embodiment (ii) includes, but not particularly limited to, at least one selected from the group consisting of LNA, AmNA, BNA N-H, BNA N-Me and ENA. In the present invention, the bridged nucleic acid contained in the non-naturally occurring polynucleotide is preferably LNA.

Here, the mismatch nucleotide may or may not be a bridged nucleic acid, but in Embodiment (ii), it is more preferred that the mismatch nucleotide is not a bridged nucleic acid. Among total three nucleotides including a mismatch nucleotide and nucleotides adjacent thereto, the number of the bridged nucleic acid is preferably 2. In addition, "more" in the phrase "more nucleotides are a bridged nucleic acid" defined in the above (A) is, but not particularly limited, preferably a case where the bridged nucleic acids are continuously two, but not the case of continuously three or more, and the number is more preferably one.

(3) Embodiment (iii)

A further embodiment of the non-naturally occurring polynucleotide of the present invention (Embodiment (iii)) is constitutions:

(B) one or more nucleotides adjacent to a 3' downstream side of the mismatch nucleotide are a bridged nucleic acid,
(C) a nucleotide at a 5' terminus is a bridged nucleic acid, and
(D) the chain length is from 22 to 95 nucleotides,

provided that the feature of the above (A) is not contained. Such a constitution can also exhibit the effects of the present invention.

The bridged nucleic acid contained in the non-naturally occurring polynucleotide of Embodiment (iii) includes, but not particularly limited to, at least one selected from the group consisting of LNA, AmNA, BNA N-H, BNA N-Me and ENA. In the present invention, the bridged nucleic acid contained in the non-naturally occurring polynucleotide is preferably LNA.

Here, the mismatch nucleotide may or may not be a bridged nucleic acid, but in Embodiment (iii), it is more preferred that the mismatch nucleotide is not a bridged nucleic acid. Among total three nucleotides including a mismatch nucleotide and nucleotides adjacent thereto, the number of the bridged nucleic acid is preferably 2. In addition, "more" in the phrase "more nucleotides are a bridged nucleic acid" defined in the above (B) is, but not particularly limited, preferably a case where the bridged nucleic acids are continuously two, but not the case of continuously three or more, and the number is more preferably one.

[Chain Length of Non-naturally Occurring Polynucleotide]

As described above, the chain length of the non-naturally occurring polynucleotide of the present invention is from 22 to 95 nt (nucleotide), preferably from 23 to 95 nt, more preferably from 24 to 95 nt, even more preferably from 25 to 95 nt, even more preferably from 27 to 62 nt, and even more preferably from 35 to 53 nt. In the case where the chain length of the non-naturally occurring polynucleotide is within such numeral range, the target polynucleotide sequence can be preferably altered in high editing efficiency.

[Relationship of Mismatch Nucleotide and Bridged Nucleic Acid Adjacent Thereto]

In any of the above Embodiments (i) to (iii), for example if mismatch nucleotide is a bridged nucleic acid, the non-naturally occurring polynucleotide of the present invention can exhibit sufficient editing efficiency to the target nucleotide sequence. In this case, it is more preferred that the bridged nucleic acid is LNA.

In the present invention, at least one of the nucleotides adjacent to the mismatch nucleotide is a bridged nucleic acid. In the relationship of the mismatch nucleotide and the bridged nucleic acid adjacent thereto, the number of the consecutive bridged nucleic acid is not particularly limited, but in the case where plural bridged nucleic acids exist continuously including the case where the mismatch nucleotide is a bridged nucleic acid, the number of the consecutive bridged nucleic acid is preferably 2.

[3' Terminus Is Bridged Nucleic Acid]

In any one of the above Embodiments (i) to (iii), for example, if (E) a nucleotide at a 3' terminus is a bridged nucleic acid, the non-naturally occurring polynucleotide of the present invention can further increase the editing efficiency of the target nucleotide sequence.

[Nucleotide Adjacent to 5' Terminus Is Bridged Nucleic Acid]

In any one of the above Embodiments (i) to (iii), or in addition to the above (E), for example if (F) one or more nucleotides adjacent to a nucleotide at a 5' terminus is a bridged nucleic acid, the non-naturally occurring polynucleotide of the present invention can further increase the editing efficiency of the target nucleotide sequence. In this case, it is preferred that one nucleotide adjacent to the nucleotide at a 5' terminus is a bridged nucleic acid. Further, it is more preferred that the bridged nucleic acid is LNA. In this case, the number of the bridged nucleic acid in the consecutive nucleotide at a 5' terminus is not particularly limited, but is preferably 2, for example when the number from the mismatch nucleotide to the 5' terminus is 12 nt, and is preferably 3, for example when the number of the 5' terminus is 25nt.

[Nucleotide Adjacent to 3' Terminus Is Bridged Nucleic Acid]

In any one of the above Embodiments (i) to (iii), or in addition to the above (E) and/or (F), for example if (G) one or more nucleotides adjacent to a nucleotide at a 3' terminus is a bridged nucleic acid, the non-naturally occurring polynucleotide of the present invention can further increase the editing efficiency of the target nucleotide sequence. In this case, further it is more preferred that the bridged nucleic acid is LNA. In this case, the number of the consecutive bridged nucleic acid in the nucleotide at a 3' terminus is, but not particularly limited, for example, preferably 3 or less, more preferably 2 or less, and even more preferably 1 when the number of nucleotides from the mismatch nucleotide to a 3' terminus is 27 nt. Further, the number of the consecutive bridged nucleic acid in the nucleotide at a 3' terminus is preferably 3 or less, more preferably 2 or less, and even more preferably 1, for example, when the number of nucleotides from the mismatch nucleotide to a 3' terminus is 12 nt.

[Substitution to Phosphate Part Modification Bond]

In any one of the above Embodiments (i) to (iii), or in addition to the above (E), (F) and/or (G), for example if one or more phosphodiester bond moieties between the nucleotides are substituted to a phosphate part modification bond, the non-naturally occurring polynucleotide of the present invention can further increase the editing efficiency of the present invention. The specific constitution includes, but not particularly limited, the following (J).

(J) One or more phosphodiester bond moieties between the nucleotides are substituted to a phosphate part modification bond.

In the present invention, the phosphate part modification bond is a bond in which an atom of a part of the phosphodiester bond is substituted to another atom or a substituent, for example, a sulfur atom, a boron atom, a nitrogen atom, a methyl group or an ester group. The specific example of the phosphate part modification bond includes a phosphorothioate bond, a methyl phosphate bond, a boranophosphate bond and a mesyl phosphoramidate bond.
In the non-naturally occurring polynucleotide of the present invention, the number or the position of the phosphate part modification bond is not particularly limited. Further, the kind of phosphate part modification bond in the non-naturally occurring polynucleotide of the present invention may be one and two or more kinds may be mixed.
The number of the phosphate part modification bond may be one, and the proportion of the phosphate part modification bond in the phosphodiester bond may be 60% or less, but it is preferred that all the phosphodiester bonds are not substituted from the viewpoint of editing efficiency.
One of embodiments of the above (J) includes the following. (X3) A phosphodiester bond moiety between a nucleotide at a 3' terminus and one or more nucleotides adjacent to the nucleotide at a 3' terminus is substituted to a phosphate part modification bond.
In the above (J), the phosphate part modification bond is preferably a phosphorothioate bond, from the viewpoint of an editing efficiency. An embodiment in which the phosphate part modification bond is a phosphorothioate bond includes any one of the following (H1), (I), (K) and (L), or a combination thereof.

(H1) The phosphodiester bond moiety between a nucleotide at a 5' terminus and one or more nucleotides adjacent to the nucleotide at a 5' terminus is substituted to a phosphorothioate bond.
(I) The phosphodiester bond moiety between a nucleotide at a 3' terminus and one or more nucleotides adjacent to the nucleotide at a 3' terminus is substituted to a phosphorothioate bond.
(K) The phosphodiester bond moiety between a bridged nucleic acid adjacent to a 5' upstream side of the mismatch nucleotide and one or more nucleotides further adjacent to the bridged nucleic acid is substituted to a phosphorothioate bond.
(L) The phosphodiester bond moiety between a bridged nucleic acid adjacent to a 3' downstream side of the mismatch nucleotide and one or more nucleotides further adjacent to the bridged nucleic acid is substituted to a phosphorothioate bond.

In the above features (K) and (L), a phosphodiester bond moiety between a bridged nucleic acid and one nucleotide directly adjacent to the bridged nucleic acid may or may not be substituted to a phosphorothioate bond.
The non-naturally occurring polynucleotide of the present invention may have both of the above features (H1) and (I), and in this case, the non-naturally occurring polynucleotide of the present invention preferably has a feature "a phosphodiester bond moiety between a nucleotide at a 5' terminus and one or more consecutive nucleotides adjacent to the nucleotide at a 5' terminus, and a phosphodiester bond moiety between a nucleotide at a 3' terminus and one or more consecutive nucleotides adjacent to the nucleotide at a 3' terminus are being substituted to a phosphorothioate bond."

[Nucleic Acid between Bridged Nucleic Acid Adjacent to 5' Upstream Side of Mismatch Nucleotide and Bridged Nucleic Acid at 5' Terminus is Bridged Nucleic Acid]

In any one of the above Embodiments (i) to (iii), or in addition to the above (E), (F), (G) and/or (J), for example if (M) one or more nucleotides placed between a nucleotide adjacent to a 5' upstream side of the mismatch nucleotide and a nucleotide at a 5' terminus, and placed at a distance of at least one nucleotide from either of a nucleotide adjacent to a 5' upstream side of the mismatch nucleotide and a nucleotide at a 5' terminus are a bridged nucleic acid, the non-naturally occurring polynucleotide of the present invention can further increase the editing efficiency of the target nucleotide sequence.

[Nucleic Acid Between Bridged Nucleic Acid Adjacent to 3' Downstream Side of Mismatch Nucleotide and Bridged Nucleic Acid at 3' Terminus Is Bridged Nucleic Acid]

In any one of the above Embodiments (i) to (iii), or in addition to the above (E), (F), (G), (J) and/or (M), for example if (N) one or more nucleotides placed between a nucleotide adjacent to a 3' downstream side of the mismatch nucleotide and a nucleotide at a 3' terminus, and placed at a distance of at least one nucleotide from either of a nucleotide adjacent to a 3' downstream side of the mismatch nucleotide and a nucleotide at a 3' terminus are a bridged nucleic acid, the non-naturally occurring polynucleotide of the present invention can further increase the editing efficiency of the target nucleotide sequence.

[RNA Substitution]

The non-naturally occurring polynucleotide of the present invention is a single-stranded polynucleotide, but the deoxyribose in one or more nucleotides may be substituted to a ribose.
Specifically, in any one of the above Embodiments (i) to (iii), or in addition to the above (E), (F), (G), (J), (M) and/or (N), for example if (O) a pentose in one or more nucleotides adjacent to the bridged nucleic acid at a 3' terminus is a ribose, and/or (X2) a pentose in one or more nucleotides adjacent to the bridged nucleic acid at 5' terminus is a ribose, the editing efficiency of the target nucleotide sequence can be further increased.

[Phosphorylation of 3' Terminus]

In any one of the above Embodiments (i) to (iii), or in addition to the above (E), (F), (G), (J), (M), (N), (O) and/or (X2), for example if (P) a nucleotide at a 3' terminus is phosphorylated, the non-naturally occurring polynucleotide of the present invention can further increase the editing efficiency of the target nucleotide sequence.

[Modification to Sugar Part of Nucleic Acid]

In any one of the above Embodiments (i) to (iii), or in addition to the above (E), (F), (G), (J), (M), (N), (O), (X2) and/or (P), a modification to a sugar part of a nucleic acid, for example, if (X1) a nucleotide at a 3' terminus and/or one or more nucleotides adjacent to the nucleotide at a 3' terminus is substituted to a nucleic acid modified at 2' site, the non-naturally occurring polynucleotide of the present invention can further increase the editing efficiency of the target nucleotide sequence.
The example of the nucleic acid modified at 2' site is at least one selected from the group consisting of 2'-F, 2'-OMe, 2'-MOE and 2'-O-(2-carbamoylethyl).

[Addition of Adapter to 3' Terminus]

In any one of the above Embodiments (i) to (iii), or in addition to the above (E), (F), (G), (J), (M), (N), (O), (X2), (P)

and/or (X1), for example, if (X4) an adapter is added to a 3' terminus, the non-naturally occurring polynucleotide of the present invention can further increase the editing efficiency of the target nucleotide sequence.

[Insertion of Nucleotide between Mismatch Nucleotide and Nucleotide at 3' Terminus]

In any one of the above Embodiments (i) to (iii), or in addition to the above (E), (F), (G), (J), (M), (N), (O), (X2), (P), (X1) and/or (X4), for example, if (X5) one or more nucleotides are inserted between the mismatch nucleotide and a nucleotide at a 3' terminus, the non-naturally occurring polynucleotide of the present invention can further increase the editing efficiency of the target nucleotide sequence. The "mismatch nucleotide" in (X5) refers to a mismatch nucleotide for the purpose of alteration. Further, the nucleotide to be inserted in (X5) is a mismatch nucleotide, but the mismatch nucleotide used herein is a mismatch nucleotide for the purpose of improving the editing efficiency, and does not fall under a mismatch nucleotide for the purpose of alteration. Further, the nucleotide to be inserted may be a nucleic acid modified at phosphate part mentioned above, a nucleic acid modified at sugar part mentioned above and a nucleic acid modified at base part mentioned above.

The number of the nucleotide to be inserted in (X5) may be one, and when the number is plural, the number is preferably 2 or more, and on the other hand, preferably 6 or less, and more preferably 3 or less.

(4) Embodiment (iv)

A further embodiment of the non-naturally occurring polynucleotide of the present invention (Embodiment (iv)) is a constitution which is one or more kinds selected from the group consisting of:

(A) one or more nucleotides adjacent to a 5' upstream side of the mismatch nucleotide are a bridged nucleic acid, and/or (B) one or more nucleotides adjacent to 3' downstream side of the mismatch nucleotide are a bridged nucleic acid,
(D) the chain length is from 22 to 95 nucleotide, and further (H2) a phosphodiester bond moiety between a nucleotide at a 5' terminus and one or more nucleotides adjacent to the nucleotide at a 5' terminus is substituted to a phosphate part modification bond, (Y1) a nucleotide at a 5' terminus is a mismatch nucleotide and one or more nucleotides adjacent to the nucleotide at a 5' terminus is a mismatch nucleotide, and (Y3) an adapter is added to a 5' terminus.

The constitutions can exhibit the effects of the present invention.
(A), (B) and (D) in Embodiment (iv) are same as those of Embodiment (i).

Here, the mismatch nucleotide may or may not be a bridged nucleic acid, but in Embodiment (iv), it is more preferred that the mismatch nucleotide is not a bridged nucleic acid.

[Substitution to Phosphate Part Modification Bond]

In the Embodiment (iv), in the non-naturally occurring polynucleotide of the present invention, it is preferred that (H2) a phosphodiester bond moiety between a nucleotide at a 5' terminus and one or more nucleotides adjacent to the nucleotide at a 5' terminus is substituted to a phosphate part modification bond from the viewpoint of an editing efficiency.

In the present invention, the phosphate part modification bond is a bond in which an atom of a part of a phosphodiester bond is substituted to another atom or a substituent, for example, a sulfur atom, a boron atom, a nitrogen atom, a methyl group, or an ester group. The specific example of the phosphate part modification bond includes a phosphorothioate bond, a methyl phosphate bond, a boranophosphate bond and a mesyl phosphoramidate bond.

In the non-naturally occurring polynucleotide of the present invention, the number or the position of the phosphate part modification bond is not particularly limited. Further, the kinds of the phosphate part modification bond in the non-naturally occurring polynucleotide of the present invention may be one, or two or more kinds may be mixed.

The number of the phosphate part modification bond may be 1, and the proportion of the phosphate part modification bond in the phosphodiester bond may be 60% or less, but it is preferred that all of the phosphodiester bonds are not substituted, from the viewpoint of an editing efficiency.

[Introduction of Mismatch Nucleotide into 5' Terminal Side]

In the Embodiment (iv), in the non-naturally occurring polynucleotide of the present invention, it is preferred that (Y1) a nucleotide at a 5' terminus is a mismatch nucleotide, and one or more nucleotides adjacent to the nucleotide at a 5' terminus is a mismatch nucleotide, from the viewpoint of an editing efficiency. The mismatch nucleotide in (Y1) is "a mismatch nucleotide for the purpose of improving an editing efficiency," and does not fall under "a mismatch nucleotide for altering the target nucleotide sequence." When the mismatch nucleotide in (Y1) is plural, the mismatch nucleotides may or

may not be adjacent to each other. In addition, the number is preferably 2 or more, and on the other hand, the number is preferably 10 or less, and more preferably 7 or less.

[Addition of Adapter to 5' Terminus]

In the above Embodiment (iv), in the non-naturally occurring polynucleotide of the present invention, it is preferred that (Y3) an adapter is added to a 5' terminus, from the viewpoint of an editing efficiency. The detail of the adapter is as described above.

[RNA Substitution]

The non-naturally occurring polynucleotide of the present invention is a single-stranded polynucleotide, but a deoxyribose in one or more nucleotides may be substituted to a ribose.
Specifically, in Embodiment (iv), further for example, if (Y2) a pentose in the nucleotide at a 5' terminus is a ribose and a pentose in one or more nucleotides adjacent to the nucleotide at a 5' terminus is a ribose, the editing efficiency of the target nucleotide sequence can be further increased.

(5) Embodiment (v)

A further embodiment of the non-naturally occurring polynucleotide of the present invention (Embodiment (v)) is a constitution:

(A) one or more nucleotides adjacent to a 5' upstream side of the mismatch nucleotide are a bridged nucleic acid, and/or (B) one or more nucleotides adjacent to 3' downstream side of the mismatch nucleotide are a bridged nucleic acid,
(D) the chain length is from 22 to 95 nucleotides, and
(X7) one or more nucleotides placed between the mismatch nucleotide and the nucleotide at a 5' terminus is a bridged nucleic acid.

The constitution can exhibit the effects of the present invention.
(A), (B) and (D) in Embodiment (v) are same as those of Embodiment (i).
Here, the mismatch nucleotide may or may not be a bridged nucleic acid, but in Embodiment (v), it is more preferred that the mismatch nucleotide is not a bridged nucleic acid.
In the above Embodiment (v), in the non-naturally occurring polynucleotide of the present invention, (X7) one or more nucleotides placed between the mismatch nucleotide and the nucleotide at a 5' terminus are a bridged nucleic acid, from the viewpoint of an editing efficiency. The bridged nucleic acid defined in (X7) includes, but not particularly limited to, at least one selected from the group consisting of LNA, AmNA, BNA N-H, BNA N-Me and ENA, and LNA is preferred.

(6) Embodiment (vi)

A further embodiment of the non-naturally occurring polynucleotide of the present invention (Embodiment (vi)) is a constitution:

(C) a nucleotide at a 5' terminus is a bridged nucleic acid,
(D) the chain length is from 22 to 95 nucleotides,
(I) a phosphodiester bond moiety between a nucleotide at a 3' terminus and one or more nucleotides adjacent to the nucleotide at a 3' terminus is substituted to a phosphorothioate bond, and
(X6) the mismatch nucleotide is a bridged nucleic acid.

The constitution can exhibit the effects of the present invention.
(C), (D) and (I) in Embodiment (vi) are same as those of Embodiment (i).
In Embodiment (vi), in the non-naturally occurring polynucleotide of the present invention, (X6) the mismatch nucleotide is bridged nucleic acid, from the viewpoint of an editing efficiency. The bridged nucleic acid as defined in the above (X6) includes, but not particularly limited to, at least one selected from the group consisting of LNA, AmNA, BNA N-H, BNA N-Me and ENA, and LNA is preferred.

[3' Terminus Is Bridged Nucleic Acid]

In the above Embodiment (vi), for example, if (E) a nucleotide at a 3' terminus is a bridged nucleic acid, the non-naturally occurring polynucleotide of the present invention can further increase the editing efficiency of the target nucleotide sequence. The bridged nucleic acid defined in (E) includes, but not particularly limited to, at least one selected from the group consisting of LNA, AmNA, BNA N-H, BNA N-Me and ENA, and LNA is preferred.

[Phosphorylation of 3' Terminus]

In the above Embodiment (vi), or further in addition to the above (E), for example, if (P) a nucleotide at a 3' terminus is phosphorylated, the non-naturally occurring polynucleotide of the present invention can further increase the editing efficiency of the target nucleotide sequence.

< Editing Efficiency >

Efficiency of altering the target nucleotide sequence by the non-naturally occurring polynucleotide of the present invention (as used herein, referred to as "editing efficiency") includes, for example, a range of 0.4 or more, preferably 0.5 or more and more preferably 0.6 or more by relatively expressing the editing efficiency as 1.0 in the case where GEO-8 of Examples mentioned below which is a non-naturally occurring polynucleotide shown in SEQ ID NO: 9 is used.

< Kit >

In the present invention, "kit" refers to a genetic engineering tool used for alteration of a target nucleotide sequence containing a non-naturally occurring polynucleotide. The kit of the present invention may contain a buffer, a stabilizer, an antiseptic, other reagents, instructions describing the alteration protocol of the target nucleotide sequence by the non-naturally occurring polynucleotide, and the like within the range not impairing the effects of the present invention.

< Pharmaceutical Composition >

A pharmaceutical composition which can treat the intended diseases can be provided by utilizing a pharmaceutical composition (hereinafter, referred to as "pharmaceutical composition" of the present invention) containing the non-naturally occurring polynucleotide of the present invention, and altering a gene having mutation which does not normally function to a gene which normally functions by an action of the non-naturally occurring polynucleotide.

When the pharmaceutical composition of the present invention is used, the pharmaceutical composition can be administered, for example, orally, intravenously, muscularly, oral mucosally, rectally, vaginally, trans-dermally, intranasally, via inhalation, or the like. Alternatively, an *ex vivo* treatment method can be used by using the pharmaceutical composition of the present invention to cells removed from a patient, culturing the treated cells if necessary, and returning the cells into the body of the patient. The non-naturally occurring polynucleotide of the pharmaceutical composition of the present invention may be used alone or formulated with other ingredients, and the non-naturally occurring polynucleotide can be provided as a formulation by formulating a pharmaceutically acceptable carrier or an additive for a formulation thereto. The pharmaceutically acceptable carrier or an additive includes, but not particularly limited to, a lipid nanoparticle, an excipient, a disintegrant, a disintegration aid, a binder, a lubricant, a coating agent, a pigment, a diluent, a dissolving agent, a dissolution aid, a tonicity agent, a pH adjustment agent, a stabilizer and the like.

The administration of the pharmaceutical composition of the present invention depends upon the severity and reactivity of a pathology to be treated, and is persisted for from days to months, or until a healing is realized or until a regression of the pathology is achieved. One of ordinary skill in the art can determine the optimized dosage, administration method and repeated frequency.

< Disease to Be Treated with Pharmaceutical Composition >

The disease to be treated with the pharmaceutical composition of the present invention includes, for example, a disease caused by substitution of one nucleotide of a genome in a human, for example, adrenal leukodystrophy, medium-chain acyl-CoA dehydrogenase deficiency, Wilson disease, heredity pulmonary arterial hypertension, X-linked agammaglobulinemia, cystinuria, Duchenne muscular dystrophy, hemophilia A, hemophilia B, tyrosinemia, hepatic glycogen storage disease type Ia, familial frontotemporal lobar degeneration, mucopolysaccharidosis type II, mucopolysaccharidosis type I, primary immunodeficiency syndrome, familial hypertrophic cardiomyopathy, peroxisome biogenesis disorder, hepatic glycogen storage disease type IX, protein C deficiency, hepatic glycogen storage disease type V, familial dilated cardiomyopathy, $\alpha$1 antitrypsin deficiency, citrin deficiency, cystinuria, Niemann-Pick disease and amyotrophic

lateral sclerosis.

< Preparation of Non-Naturally Occurring Polynucleotide >

The non-naturally occurring polynucleotide in the present invention can be easily prepared according to a known method in the field of the present invention.

< Introduction of Non-Naturally Occurring Polynucleotide into Cell >

In the present invention, "the non-naturally occurring polynucleotide is introduced into a cell" can be carried out according to a known method depending upon the cell to be introduced. A known method of introducing polynucleotide into a cell (transfection method) is largely separated into two kinds of a viral vector system and a non-viral vector system. The viral vector system is a method of introducing a gene into a cell by utilizing a cell entrance mechanism intrinsically owned by virus, and the method is, but not particularly limited, a method including using adenovirus, retrovirus, lentivirus and the like as a vector. The non-viral vector system includes, but not particularly limited to, lipofection method, electroporation method, microinjection method, particle gun method, and the like. The lipofection method is a method utilizing a phenomenon in which a positively charged cationic liposome binds to around a negatively charged polynucleotide to form a complex and the polynucleotide is incorporated into a cell from a cell surface by an endocytosis phenomenon. The electroporation method is a method including directly applying a high voltage pulse to a cell using a dedicated device to cause uptake of a polynucleotide from a small pore opened on the cell surface. The microinjection method is a method including placing a substance to be introduced in a glass needle with a tip diameter of about 1 μm or so and directly introducing the substance into a cell. The particle gun method is a method including coprecipitating a gold particle and DNA to coat the surface of the gold particle with DNA, and ejecting the gold particle to a target cell by helium compressed gas and the like. The present invention is a method of altering a target nucleotide sequence by introducing only a non-naturally occurring polynucleotide without introducing Cas nuclease into a cell, and the non-viral vector system is more preferable from the viewpoint of safety. The method of the present invention includes, for example, a method which is carried out in the human body or a method including the step which is carried out in the human body.

The non-naturally occurring polynucleotide which is introduced into a cell as such (as used herein, also referred to as "editing nucleic acid") selectively binds to the target nucleotide sequence in a genomic DNA as schematically shown in FIG. 1. The non-naturally occurring polynucleotide of the present invention is mismatched to a nucleotide to be edited in the target nucleotide sequence in an intracellular double-stranded DNA such as genomic DNA, and it is preferred that all the nucleotide sequences other than the nucleotide to be edited are designed to be a nucleotide sequence complementary to the target nucleotide sequence. Justifiably, there may be a mismatch nucleotide in places other than the nucleotide to be edited within the range not violating the purposes of the present invention.

In the case where the nucleotide to be edited in the target nucleotide sequence is, for example, T and this nucleotide is to be altered to C, the mismatch nucleotide in the non-naturally occurring polynucleotide of the present invention is set to G. One of ordinary skill in the art can set the mismatch nucleotide in the non-naturally occurring polynucleotide to alter the nucleotide of the nucleotide to be edited to a desired nucleotide based on the technical common knowledge.

< Method of Altering One or More Nucleotides Contained in Target Nucleotide Sequence in Intracellular Double-Stranded DNA >

"A method of altering one or more nucleotides contained in a target nucleotide sequence in an intracellular double-stranded DNA" of the present invention includes the step of introducing the non-naturally occurring polynucleotide of the present invention mentioned above into a cell. Here, the alteration of the target nucleotide sequence includes at least one or more selected from the group consisting of the deletion, insertion and substitution of one or more nucleotides of the target nucleotide sequence. As the non-naturally occurring polynucleotide used in the alteration method of the present invention, those described in the above Embodiments (i) to (vi) can be preferably used.

< Confirmation Method of Alteration of Target Nucleotide Sequence >

A method of confirming the alteration of the target nucleotide sequence in the present invention includes a method including measuring the activity of the gene containing the target nucleotide sequence, a method including directly measuring the target nucleotide sequence using a next generation sequencer (NGS) and a digital PCR, and the like.

< Cell>

The method of altering the target nucleotide sequence using the non-naturally occurring polynucleotide of the present invention can be applied to all cells having a double-stranded DNA. Further, not only a double-stranded DNA present in a cell, but also a single-stranded DNA derived from a virus of a cell infected with the virus can be altered as a target nucleotide sequence, if a DNA replication mechanism in a cell can be utilized. In other words, "cell" of the present invention includes a prokaryotic cell and a eukaryotic cell.

< Prokaryotic Cell >

In the present invention, "prokaryotic cell" includes, but not particularly limited to, a bacterial cell and an archaeal cell.

< Eukaryotic Cell >

In the present invention, "eukaryotic cell" includes, but not particularly limited to, an animal cell, an insect cell, a plant cell, an algal cell and a fungal cell.

< Animal Cell >

In the present invention, "animal cell" includes, but not particularly limited to, a cell of vertebrate, a cell of invertebrate, a cell derived from animal tissues, a germ cell, a somatic cell and a stem cell. The germ cell includes an oocyte and a sperm cell. The somatic cell includes, but not particularly limited to, a fibroblast, a hematopoietic cell, a neuron, a myocyte, an osteocyte, a hepatocyte, a splenocyte, a brain cell, a kidney cell, and the like. The stem cell includes, but not particularly limited to, an induced pluripotent stem cell (induced pluripotent stem cells, iPS cells), and an embryonic stem cell (embryonic stem cells, ES cells).

< Mammalian Cell >

In the present invention, "mammal" is a group of a vertebrate belonging to a mammalian, and the mammal also includes a human. "Mammalian cell" refers to a cell constituting a mammal or a cell derived from mammal. An example of a mammal other than a human includes, but not particularly limited to, deer, eared seal, hamster, dog, mouse, wolf, whale, zebra, donkey, mustelid, bat, dolphin, anteater, earless seal, cow, wild boar, horse, squirrel, bear, cat, mole, monkey, raccoon, kangaroo, pig, fox, sheep, and the like.

A cell of a human also includes a cell derived from a tissue, a germ cell, a somatic cell and a stem cell, as described above. In the Examples of the present specification, HEK293 cells were used to confirm the alteration of the target nucleotide sequence, but another cell, for example, a HeLa cell can be used to confirm.

< Insect Cell >

In the present invention, "insect" is a generic name of an arthropod classified to Insecta.

As the insect, silkworm, fruit fly, cricket and the like are used, but not particularly limited thereto. In the present invention, "insect cell" includes, but not particularly limited to, a cell constituting a body tissue of an insect, a cell derived from insect tissue, and the like.

< Plant Cell >

In the present invention, "plant" includes, but not particularly limited to, spermatophyte, pteridophyte, bryophyte, alga and the like. The "plant cell" refers to a cell constituting a plant or a cell derived from a plant. The spermatophyte includes angiosperm and gymnosperm. The angiosperm includes dicotyledon and monocotyledon. The dicotyledon includes, but not particularly limited to, morning glory, dandelion, Ericaceae, Rhododendron indicum, eggplant, brassica, pea and the like. The monocotyledon includes, but not particularly limited to, rice, lily, tulip, blackboy, corn, and the like. The gymnosperm includes, but not particularly limited to, pine, Japanese cedar, maidenhair tree, Japanese cypress, and the like. The pteridophyte includes, but not particularly limited to, bracken, flowering fern, Lepisorus thunbergianus, field horsetail, and the like. The bryophyte includes, but not particularly limited to, hepatica, hornwort, Polytrichaceae, bog moss, and the like. The alga includes multicellular alga and monocellular alga. The multicellular alga includes, but not particularly limited to, kelp, wakame, sea lettuce, spirogyra, and the like. The monocellular alga includes, but not particularly limited to, chlorella, euglena, spirulina, Chlamydomonas, Coccomyxa, Botryococcus, Closterium, Bacillariophyceae, and the like.

## EXAMPLES

Hereinafter, the present invention will be particularly described based on specific Examples, without intending to limit the scope of the present invention to the following Examples.

### A. Genome Editing Experimentation Using 293-nLD1 cells (1)

### 1. Preparation of mutant NanoLuc(registered trademark) plasmid

DNA of a region from base number of from 847 to 1,380 was synthesized (Gene Universal Inc.) based on a wild-type nucleotide sequence of NanoLuc gene which is a luciferase gene (GenBank JQ513379), XhoI and ApaI likers were bound thereto, and the nucleotide sequence was incorporated into the corresponding restriction enzyme site of a pcDNA™ 5/FRT/TO plasmid (Thermo Fisher Scientific Inc.) (pcDNA5-nLW1). Here, the wild-type nucleotide sequence of NanoLuc gene is defined as SEQ ID NO: 1.

On the other hand, a mutant nucleotide sequence in which cytosine (C) at the base number 922 of NanoLuc gene was changed to thymine (T) was synthesized, and a mutant which was incorporated into a pcDNA™5/FRT/TO plasmid as mentioned above was produced (pcDNA5-nLD1). The luciferase gene owned by the mutant is an inactivated luciferase gene not exhibiting luciferase activity due to the above point mutation.

### 2. Preparation of stably transformed cell line having mutant NanoLuc gene

Flp-In™-293 cell line which is a human-derived cell line (Thermo Fisher Scientific Inc.) was seeded onto a 6 cm dish to grow up to $1x10^6$ cells, and the cells were cultured in Dulbecco's Modified Eagle Medium (DMEM, Thermo Fisher Scientific Inc.) containing 10% concentration of fetal bovine serum (FBS, Thermo Fisher Scientific Inc.) under an environment of 5% $CO_2$ at 37°C. After 24 hours, a pcDNA5-nLD1 or pcDNA5-nLW1 plasmid (1 μg) and a pOG44 plasmid (3 μg, Thermo Fisher Scientific Inc.) were transfected to the cells according to a usual protocol using Lipofectamine 3000 (Thermo Fisher Scientific Inc.). After 48 hours, TrypLE™ Express Enzyme (Thermo Fisher Scientific Inc.) was added to the cells and incubated for 3 minutes at 37°C, to collect the detached cells, and the cells were suspended to a DMEM + 10% FBS medium containing 50 μg/ml of hygromycin (Thermo Fisher Scientific Inc.), to separate the cells to two 10 cm dishes and culture the cells. Thereafter, the medium was exchanged every three days with the same medium to continue the culture. After about 20 days culture, formation of colony having a sufficient number and size was confirmed, and all cells were stripped from the petri dishes using TrypLE™ Express Enzyme to collect the cells.

The cell in which a mutant NanoLuc was incorporated into a genome was named as 293-nLD1 cell. The mutant NanoLuc gene owned by the 293-nLD1 cell is an inactivated luciferase gene, as mentioned above. This 293-nLD1 cell was used in the later genome editing experimentation.

On the other hand, the cell in which wild-type NanoLuc was incorporated into a genome was named as 293-nLW1, and the cell was used as a positive control of the later genome editing experimentation.

### 3. Introduction of non-naturally occurring polynucleotide into 293-nLD1 cell

293-nLD1 cells were seeded onto a 96 well plate (Nunc(registered trademark) MicroWell™ 96, Nunclon Delta-Treated, Flat-Bottom Microplate, Thermo Fisher Scientific Inc.) to grow up to $1x10^4$ cells, and the cells were cultured in DMEM + 10% FBS medium under an environment of 5% $CO_2$ at 37°C. After 24 hours, 0.05 or 0.1 μg of non-naturally occurring polynucleotides were transfected according to a usual protocol using Lipofectamine 3000.

### 4. Measurement of editing efficiency

• NanoLuc Luciferase Assay

After continuing the culture for 72 hours from the transfection, NanoLuc Luciferase activity was measured. The NanoLuc Luciferase activity was measured according to a usual protocol using Nano-Glo(registered trademark) Luciferase Assay System (Promega Corporation). EnSpire multi-mode plate reader (PerkinElmer Co., Ltd.) was used for the measurement of luminescence of luciferase.

• Count of number of living cells

The number of living cells were measured according to a usual protocol using CellTiter-Blue(registered trademark) Cell Viability Assay (Promega Corporation). EnSpire multi-mode plate reader (PerkinElmer Co., Ltd.) was used for the measurement of fluorescence value of CellTiter-Blue.

The number of living cells of edited cells was between 5 x $10^3$ and 2 x $10^5$, and a calibration curve of the measurement of CellTiter-Blue and the number of cells was generated using two-fold dilution series of the number of 293-nLD1 cells, to calculate the number of living cells based on the calibration curve.

• Calculation of editing efficiency

When the NanoLuc Luciferase activity of 293-nLW1 cells in which wild-type luciferase was incorporated into a genome was measured, the measurement was about 20,000 counts/cell. Accordingly, if the mutation of the inactivated luciferase gene is repaired and wild-type NanoLuc is synthesized, 20,000 counts of luciferase activity per cell will be detected. Based on the value, the editing efficiency (%) was calculated according to the calculation formula:

$$\text{NanoLuc Luciferase activity} / 20{,}000 / \text{Number of Cells} \times 100.$$

5. Measurement of editing efficiency by Next Generation Sequencing (NGS)

A genomic DNA was prepared from 293-nLD1 cell to which the non-naturally occurring polynucleotide was transfected according to a usual protocol using NucleoSpin Tissue (Takara Inc,). The region of about 200 bases from upstream to downstream of the nucleotide to be edited was amplified by PCR against the prepared genomic DNA and a library was generated, to carry out amplicon sequencing to this library with MiSeq/MiSeq Reagent Kit v3 (Illumin) (Bioengineering Lab. Co., Ltd.). From 30,000 to 50,000 amplicon sequences were analyzed per sample, to calculate the editing efficiency from the proportion of counts of the number of edited sequence to counts of the number of wild-type sequence.

6. Synthesis of non-naturally occurring polynucleotide

All the non-naturally occurring polynucleotides containing a modification of a nucleic acid were synthesized in Gene Design Inc. (Japan), which was purified by a simple column or HPLC.

Example 1-1: Modification of Target Nucleotide Sequence by Non-naturally occurring Polynucleotide

A non-naturally occurring polynucleotide was introduced into 293-nLD1 cells, thereby carrying out experimentation measuring the editing efficiency of a target polynucleotide sequence.

As mentioned above, a codon (CAA) encoding position 22 of glutamine (Gln-22) of luciferase gene which was introduced into 293-nLD1 cells was subjected to a point mutation to a stop codon (TAA), and therefore, luciferase activity is not shown per se. If T in the stop codon (TAA) in the inactivated luciferase gene can be replaced to C, the luciferase activity of 293-nLD1 cells is recovered, and an alteration of the target nucleotide sequence can be detected by a luminescence by luciferase (FIG. 2).

In the Example 1-1, each of eleven kinds of non-naturally occurring polynucleotides of GEO-1 to GEO-9, GEO-242 and GEO-11, and a non-naturally occurring polynucleotide of GEO-94 in which an inactivated mutation cannot be repaired due to the absence of a mismatch nucleotide as a negative control was introduced into 293-nLD 1 cells by a transfection method (FIG. 3A).

As to all of GEO-1 to GEO-9, GEO-242 and GEO-11, the nucleotide in a nucleotide at the position corresponding to T of the above stop codon TAA in a target polynucleotide sequence is G, i.e. mismatched, and nucleotides other than the mismatch nucleotide are a nucleotide complementary to the target polynucleotide sequence. GEO-1 to GEO-4 are 21 nt, and GEO-5 to GEO-9, GEO-242, GEO-11 and GEO-94 are 25 nt. In GEO-1 and GEO-5, the mismatch nucleotide G is LNA. In GEO-2 and GEO-6, 1 nucleotide at 5' upstream side and 3' downstream side adjacent to the mismatch nucleotide G is LNA. In GEO-3 and GEO-7, the mismatch nucleotide G and a nucleotide at a 5' terminus are LNA. In GEO-4 and GEO-8, 1 nucleotide at 5' upstream side and 3' downstream side adjacent to the mismatch nucleotide G is LNA, and a nucleotide at a 5' terminus is LNA. In GEO-11, a nucleotide at a 5' terminus is LNA. In GEO-242, the nucleotide at a 3' terminus is LNA. GEO-9 does not have LNA.

In GEO-94, a nucleotide at the position corresponding to a nucleotide to be edited is A, i.e. complementary. Further, in GEO-94, 1 nucleotide at a 5' upstream side and a 3' downstream side adjacent to this nucleotide A is LNA, and a

nucleotide at a 5' terminus is LNA.

The nucleotide sequence, chain length and editing efficiency (%) of the above each editing nucleic acid of GEO-1 to GEO-9, GEO-242, GEO-11 and negative control (GEO-94) are as given in Table 1. In addition, the editing efficiency (%) of each editing nucleic acid was shown also in a bar graph (FIG. 3B).

[Table 1]

Table 1

| Editing Nucleic Acid | SEO ID NO | Sequence | Chain Length (nt) | Editing Efficiency (%) |
|---|---|---|---|---|
| GEO-1 | 2 | t c a a g g a c t t G g t c c a g g t t g | 21 | <0.05 |
| GEO-2 | 3 | t c a a g g a c t T g G t c c a g g t t g | 21 | <0.05 |
| GEO-3 | 4 | T c a a g g a c t t G g t c c a g g t t g | 21 | <0.05 |
| GEO-4 | 5 | T c a a g g a c t T g G t c c a g g t t g | 21 | <0.05 |
| GEO-5 | 6 | g t t c a a g g a c t t G g t c c a g g t t g t a | 25 | <0.05 |
| GEO-6 | 7 | g t t c a a g g a a t T g G t c c a g g t t g t a | 25 | <0.05 |
| GEO-7 | 8 | G t t c a a g g a c t t G g t c c a g g t t g t a | 25 | 0.05 |
| GEO-8 | 9 | G t t c a a g g a c t T g G t c c a g g t t g t a | 25 | 0.25 |
| GEO-11 | 10 | G t t c a a g g a c t t g g t c c a g g t t g t a | 25 | <0.05 |
| GEO-242 | 11 | g t t c a a g g a c t t g g t c c a g g t t g t A | 25 | <0.05 |
| GEO-9 | 12 | g t t c a a g g a c t t g g t c c a g g t t g t a | 25 | <0.05 |
| GEO-94 | 13 | G t t c a a g g a c t T a G t c c a g g t t g t a | 25 | 0 |

In Table 1, a guanosine monophosphate which was a mismatch nucleotide was underlined, and a nucleotide which was substituted to a bridged nucleic acid was shown in capitals. All bridged nucleic acids in Table 1 were LNA.

As shown in Table 1, for absolute value of an editing efficiency, GEO-8 was highest at 0.25%, and GEO-7 was the second highest at 0.05 %. Further, the editing efficiency of GEO-94 which was a negative control was nearly 0%. When the editing efficiency of GEO-8 was defined as 1.0, all of relative editing efficiency of GEO-1 to GEO-6, GEO-9, GEO-242 and GEO-11 were lower than 0.2. Incidentally, GEO-1, GEO-2, GEO-3, GEO-5, GEO-6 and GEO-7 have an identical constitution to one disclosed in Non-Patent Publication 1. Thus, GEO-8 which was the non-naturally occurring polynucleotide of the present invention showed higher editing efficiency by 5 times or more relative to GEO-7 which has already been reported.

In Example 1-2 to Example 1-13 mentioned below, the editing efficiency of GEO-8 was used as a basis, but if the relative editing efficiency is 0.4 or more in the case where the editing efficiency of GEO-8 is defined as 1.0, the editing efficiency shows sufficiently higher value than the editing efficiency previously reported.

## Example 1-2: Chain Length of Non-Naturally Occurring Polynucleotide

In GEO-4 and GEO-8 of Example 1-1, while the arrangement of LNA was same, significant differences were caused in an editing efficiency by the difference of 4 nt.

In Example 1-2 experimentations in the same manner as that of Example 1-1 were carried out to explore the preferred chain length of the non-naturally occurring polynucleotide, using 9 kinds of non-naturally occurring polynucleotides of which the arrangement of LNA was same and the chain length was different relative to GEO-8 (FIG. 4). The 9 kinds of non-naturally occurring polynucleotides used in Example 1-2 are GEO-23 (29 nt), GEO-79 (31 nt), GEO-24 (35 nt), GEO-25 (41 nt), GEO-174 (45 nt), GEO-175 (49 nt), GEO-176 (53 nt), GEO-281 (75 nt) and GEO-282 (95 nt).

The nucleotide sequence, chain length and relative editing efficiency of each editing nucleic acid mentioned above are as given in Table 2.

[Table 2]

Table 2

| Editing Nucleic Acid | SEQ ID NO | Sequence | Chain Length (nt) | Relative Editing Efficiency |
|---|---|---|---|---|
| GEO-8 | 9 | G t t c a a g g a c t T g G t c c a g g t t g t a | 25 | 1.00 |
| GEO-23 | 14 | C t g t t c a a g g a c t T g G t c c a g g t t g t a g c | 29 | 3.16 |
| GEO-79 | 15 | C c t g t t c a a g g a c t T g G t c c a g g t t g t a g c c | 31 | 1.40 |
| GEO-24 | 16 | T c c c t g t t c a a g g a c t T g G t c c a g g t t g t a g c c g g | 35 | 2.92 |
| GE0-25 | 17 | A c c t c c c t g t t c a a g g a c t T g G t c c a g g t t g t a g c c g g c t g | 41 | 3.47 |
| GEO-174 | 18 | A c a c c t c c c t g t t c a a g g a c t T g G t c c a g g t t g t a g c c g g c t g t c | 45 | 3.74 |
| GE0-175 | 19 | A c a c a c c t c c c t g t t c a a g g a c t T g G t c c a g g t t g t a g c c g g c t g t c t g | 49 | 2.94 |
| GE0-176 | 20 | G g a c a c a c c t c c c t g t t c a a g g a c t T g G t c c a g g t t g t a g c c g g c t g t c t g t c | 53 | 2.54 |
| GEO-281 | 21 | T g a a a c a a a c t g g a c a c a c c t c c c t g t t c a a g g a c t T g G t c c a g g t t g t a g c c g g c t g t c t g t c g c c a g t c c c c a | 75 | 0.64 |
| GE0-282 | 22 | C c c g a g a t t c t g a a a c a a a c t g g a c a c a c c t c c c t g t t c a a g g a c t T g G t c c a g g t t g t a g c c g g c t g t c t g t c g c c a g t c c c c a a c g a a a t c t t | 95 | 0.58 |

In Table 2, a guanosine monophosphate which is a mismatch nucleotide was underlined and the nucleotide which was substituted to a bridged nucleic acid were shown in capitals. All the bridged nucleic acids in Table 2 were LNA.

As shown in Table 2, relative editing efficiency of editing nucleic acids having chain length of 25 nt or more and 95 nt or less including GEO-8 which was used as a control showed 0.4 or more, and GEO-174 having 45 nt showed highest relative editing efficiency of 3.74. In GEO-281 and GEO-282 having longer chain length, a tendency in which relative editing efficiency decreased was found, but it was found that the polynucleotides showed high value of 0.58 or more.

Example 1-3: LNA Modification of 3' Terminus of Non-Naturally Occurring Polynucleotide

The Example 1-3 explored the editing efficiency of a constitution in which a pentose in a nucleotide at a 3' terminus was replaced to LNA.

Specifically, the editing efficiency was measured in the same manner as that of Example 1-1 using each of GEO-16 in which a nucleotide at a 3' terminus is LNA in GEO-6; and GEO-17 in which a further nucleotide at a 3' terminus is LNA in GEO-8 (FIG. 5).

The nucleotide sequence, chain length and relative editing efficiency of each editing nucleic acid mentioned above are as given in Table 3.

[Table 3]

Table 3

| Editing Nucleic Acid | SEO ID NO | Sequence | Chain Length (nt) | Relative Editing Efficiency |
|---|---|---|---|---|
| GEO-8 | 9 | G t t c a a g g a c t T g G t c c a g g t t g t a | 25 | 1.00 |
| GEO-6 | 7 | g t t c a a g g a a t T g G t c c a g g t t g t a | 25 | 0.05 |
| GEO-16 | 23 | g t t c a a g g a c t T g G t c c a g g t t g t A | 25 | 0.16 |
| GEO-17 | 24 | G t t c a a g g a c t T g G t c c a g g t t g t A | 25 | 2.00 |

In Table 3, a guanosine monophosphate which was a mismatch nucleotide was underlined and the nucleotide which was substituted to a bridged nucleic acid was shown in capitals. All the bridged nucleic acids in Table 3 were LNA.

As shown in Table 3, the relative editing efficiency of GEO-16 was 0.16, and that of GEO-17 was 2.00. From the results, it was found that the relative editing efficiency was low in a constitution in which 1 nucleotide at a 5' upstream side and a 3' downstream side adjacent to the mismatch nucleotide was LNA and the 3' terminus was LNA (GEO-16). In the constitution in which 1 nucleotide at a 5' upstream side and a 3' downstream side adjacent to a mismatch nucleotide was LNA and the 5' terminus was LNA (hereinafter, referred to as "constitution of GEO-8"), it was shown that the relative editing efficiency was improved when the 3' terminus was further LNA. On the other hand, in the case of a constitution in which the 3' terminus was LNA and the 5' terminus was non-modified (GEO-16), it was found that the editing efficiency was equivalent to that of GEO-6, and sufficient editing efficiency was not shown. From the above, it was shown to be important that the nucleotide at a 5' terminus was a bridged nucleic acid such as LNA for exhibiting sufficient editing efficiency.

Example 1-4: LNA Modification of One or More Nucleotides Adjacent to LNA at 5' Terminus of Non-Naturally Occurring Polynucleotide

Example 1-4 explored the editing efficiency of a constitution in which one or more nucleotides adjacent to the 5' terminus were further replaced to LNA relative to the constitution of GEO-8 of Example 1-1 and the constitution of GEO-17 of Example 1-3.

Specifically, the editing efficiency was measured in the same manner as Example 1-1 using each of GEO-18 in which 1 nucleotide adjacent to the 5' terminus was further LNA in GEO-8; GEO-19 in which consecutive 2 nucleotides adjacent to the 5' terminus were further LNA in GEO-8; and GEO-68 in which 1 nucleotide adjacent to the 5' terminus was further LNA in GEO-17 (FIG. 6).

The nucleotide sequence, chain length and relative editing efficiency of each editing nucleic acid mentioned above are as given in Table 4.

[Table 4]

Table 4

| Editing Nucleic Acid | SEQ ID NO | Sequence | Chain Length (nt) | Relative Editing Efficiency |
|---|---|---|---|---|
| GE0-8 | 9 | G t t c a a g g a c t T g G t c c a g g t t g t a | 25 | 1. 00 |
| GEO-18 | 25 | G T t c a a g g a c t T g G t c c a g g t t g t a | 25 | 2.05 |
| GE0-19 | 26 | G T T c a a g g a c t T g G t c c a g g t t g t a | 25 | 0.66 |
| GEO-17 | 24 | G t t c a a g g a c t T g G t c c a g g t t g t A | 25 | 2.00 |
| GE0-68 | 27 | G T t c a a g g a c t T g G t c c a g g t t g t A | 25 | 1. 64 |

In Table 4, a guanosine monophosphate which was a mismatch nucleotide was underlined and the nucleotide which was substituted to a bridged nucleic acid was shown in capitals. All the bridged nucleic acids in Table 4 were LNA.

As shown in Table 4, the relative editing efficiency of GEO-18 was 2.05, and that of GEO-19 was 0.66. From the results, it was found that in the constitution of GEO-8, a constitution in which one or more nucleotides adjacent to the 5' terminus were further replaced to LNA even exhibited sufficient editing efficiency. Further, relative editing efficiency of GEO-17 was 2.00, and that of GEO-68 was 1.64. From the results, it was found that that a constitution in which 1 nucleotide

adjacent to the 5' terminus was further replaced to LNA even exhibited sufficient editing efficiency, in a constitution in which 1 nucleotide at the 5' upstream side and the 3' downstream side adjacent to a mismatch nucleotide was LNA and the 5' terminus was LNA, and further the 3' terminus was LNA (hereinafter, referred to as "constitution of GEO-17").

In the constitution of GEO-8, the relative editing efficiency of GEO-19 in which 2 nucleotides adjacent to the 5' terminus were further replaced to LNA was 0.66, and the relative editing efficiency was slightly decreased as compared to the constitution of GEO-8. On the other hand, in Example 1-8 mentioned below, when the 5' upstream side of the mismatch nucleotide was extended, the relative editing efficiency of GEO-313 in which 1 nucleotide adjacent to the 5' terminus was further replaced to LNA was 5.23 in the constitution of GEO-8, and the relative editing efficiency of GEO-315 in which 2 nucleotides adjacent to the 5' terminus were further replaced to LNA was 6.59 in the constitution of GEO-8. It was suggested that if the constitution was changed such as that the chain length of the non-naturally occurring polynucleotide was changed, the editing efficiency may increase even in the case where the plural nucleotides adjacent to the 5' terminus were further replaced to LNA in the constitution of GEO-8.

Example 1-5: LNA Modification of One or More Nucleotides Adjacent to LNA at 3' Terminus of Non-Naturally Occurring Polynucleotide

Example 1-5 explored the editing efficiency of a constitution in which one or more nucleotides adjacent to a nucleotide at a 3' terminus were further replaced to LNA relative to the constitution of GEO-17 of Example 1-3.

Specifically, the editing efficiency was measured in the same manner as Example 1-1 using each of GEO-69 in which 1 nucleotide adjacent to a nucleotide at a 3' terminus was further LNA in GEO-17; and GEO-71 in which consecutive 2 nucleotides adjacent to the nucleotide at a 3' terminus were further LNA in GEO-17 (FIG.7).

The nucleotide sequence, chain length and relative editing efficiency of each editing nucleic acid mentioned above are as given in Table 5.

[Table 5]

Table 5

| Editing Nucleic Acid | SEO ID NO | Sequence | Chain Length (nt) | Relative Editing Efficiency |
|---|---|---|---|---|
| GE0-8 | 9 | G t t c a a g g a c t T g G t c c a g g t t g t a | 25 | 1.00 |
| GEO-17 | 24 | G t t c a a g g a c t T g G t c c a g g t t g t A | 25 | 2.00 |
| GEO-69 | 28 | G t t c a a g g a c t T g G t c c a g g t t g T A | 25 | 1.01 |
| GEO-71 | 29 | G t t c a a g g a c t T g G t c c a g g t t G T A | 25 | 0.51 |

In Table 5, a guanosine monophosphate which was a mismatch nucleotide was underlined, and the nucleotide substituted to a bridged nucleic acid was shown in capitals. All bridged nucleic acids in Table 5 were LNA.

As shown in Table 5, the relative editing efficiency of GEO-69 was 1.01, and that of GEO-71 was 0.51. From the results, in the constitution of GEO-17, a constitution in which 1 nucleotide adjacent to a nucleotide at a 3' terminus was further replaced to LNA exhibited an editing efficiency equivalent to that of GEO-8, and a constitution in which 2 nucleotides adjacent to the nucleotide at a 3' terminus were replaced to LNA exhibited an editing efficiency which was a half or so of that of GEO-8.

On the other hand, in Example 1-8 mentioned below, when the 3' downstream side of the mismatch nucleotide was extended, the relative editing efficiency of GEO-319 in which 1 nucleotide adjacent to the 3' terminus was further replaced to LNA was 2.18 in the constitution of GEO-17, and the relative editing efficiency of GEO-321 in which 2 nucleotides adjacent to the 3' terminus were further replaced to LNA was 1.40 in the constitution of GEO-17. In the constitution of GEO-17, even when the plural nucleotides adjacent to the 3' terminus were further replaced to LNA, it was suggested that the editing efficiency increases if the other constitution is changed such as that chain length of the non-naturally occurring polynucleotide is changed.

Example 1-6: Substitution of Phosphodiester Bond Moiety Between One or More Nucleotides Contained in Non-Naturally Occurring Polynucleotide to Phosphorothioate Bond (1)

Example 1-6 explored the editing efficiency of a constitution in which a phosphodiester bond moiety between one

or more nucleotides was substituted to a phosphorothioate bond relative to the constitution of GEO-8 of Example 1-1.

Specifically, the editing efficiency was measured in the same manner as Example 1-1 using each of GEO-158, GEO-159, GEO-160, GEO-29 and GEO-161 in which a phosphodiester bond moiety between a nucleotide at a 5' terminus and consecutive 1 to 5 nucleotides adjacent to the nucleotide at a 5' terminus was further substituted to a phosphorothioate bond in GEO-8 (FIG. 8A); GEO-162, GEO-163, GEO-164, GEO-30, GEO-165, GEO-272 and GEO-196 in which a phosphodiester bond moiety between a nucleotide at a 3' terminus and consecutive 1 to 7 nucleotides adjacent to the nucleotide at a 3' terminus was further substituted to a phosphorothioate bond in GEO-8 (FIG. 8B); GEO-31 in which a phosphodiester bond moiety between a nucleotide at a 5' terminus and consecutive 4 nucleotides adjacent to the nucleotide at a 5' terminus was further substituted to a phosphorothioate bond, and a phosphodiester bond moiety between a nucleotide at a 3' terminus and consecutive 4 nucleotides adjacent to the nucleotide at a 3' terminus was substituted to a phosphorothioate bond in GEO-8 (FIG. 8C); GEO-126, GEO-127 in which a phosphodiester bond moiety between LNA at a 5' upstream side adjacent to the mismatch nucleotide G and consecutive 2 or 4 nucleotides adjacent to the LNA was further substituted to a phosphorothioate bond in GEO-8 (FIG. 8C); as well as GEO-128, GEO-129 in which a phosphodiester bond moiety between a nucleotide adjacent to LNA at the 3' downstream side adjacent to the mismatch nucleotide G and the consecutive 2 or 4 nucleotides adjacent to the adjacent nucleotide was further substituted to a phosphorothioate bond in GEO-8 (FIG. 8C).

The nucleotide sequence, chain length and relative editing efficiency of each editing nucleic acid mentioned above are as given in Table 6.

[Table 6]

Table6

| Editing Nucleic Acid | SEO ID NO | Sequence | Chain Length (nt) | Relative Editing Efficiency |
|---|---|---|---|---|
| GE0-8 | 9 | G t t c a a g g a c t T g G t c c a g g t t g t a | 25 | 1.00 |
| GEO-158 | 30 | G*t t c a a g g a c t T g G t c c a g g t t g t a | 25 | 2.40 |
| GEO-159 | 31 | G*t*t c a a g g a c t T g G t c c a g g t t g t a | 25 | 3.19 |
| GEO-160 | 32 | G*t*t*c a a g g a c t T g G t c c a g g t t g t a | 25 | 3.02 |
| GEO-29 | 33 | G*t*t*c*a a g g a c t T g G t c c a g g t t g t a | 25 | 2.86 |
| GEO-161 | 34 | G*t*t*c*a*a g g a c t T g G t c c a g g t t g t a | 25 | 2.27 |
| GEO-162 | 35 | G t t c a a g g a c t T g G t c c a g g t t g t*a | 25 | 3.64 |
| GEO-163 | 36 | G t t c a a g g a c t T g G t c c a g g t t g*t*a | 25 | 2.07 |
| GEO-164 | 37 | G t t c a a g g a c t T g G t c c a g g t t*g*t*a | 25 | 3.08 |
| GEO-30 | 38 | G t t c a a g g a c t T g G t c c a g g t*t*g*t*a | 25 | 4.17 |
| GEO-165 | 39 | G t t c a a g g a c t T g G t c c a g g*t*t*g*t*a | 25 | 4.24 |
| GEO-272 | 40 | G t t c a a g g a c t T g G t c c a g*g*t*t*g*t*a | 25 | 3.40 |
| GEO-196 | 41 | G t t c a a g g a c t T g G t c c a*g*g*t*t*g*t* a | 25 | 3.98 |
| GEO-31 | 42 | G*t*t*c*a a g g a c t T g G t c c a g g t*t*g*t* a | 25 | 5.68 |
| GEO-126 | 43 | G t t c a a g g a c*t*T g G t c c a g g t t g t a | 25 | 0.99 |
| GEO-127 | 44 | G t t c a a g g*a*c*t*T g G t c c a g g t t g t a | 25 | 0.70 |
| GEO-128 | 45 | G t t c a a g g a c t T g G t*c*c a g g t t g t a | 25 | 0.55 |
| GEO-129 | 46 | G t t c a a g g a c t T g G t*c*c*a*g g t t g t a | 25 | 2.15 |

In Table 6, a guanosine monophosphate which was a mismatch nucleotide was underlined, and the nucleotide

substituted to a bridged nucleic acid was shown in capitals. All bridged nucleic acid in Table 6 were LNA. An asterisk (*) was inserted between the corresponding two or more nucleotides at the position in which the phosphodiester bond moiety between two or more nucleotides was substituted to a phosphorothioate bond.

As shown in Table 6, the relative editing efficiency of GEO-158 was 2.40, that of GEO-159 was 3.19, that of GEO-160 was 3.02, that of GEO-29 was 2.86, and that of GEO-161 was 2.27. From these results, it was shown that editing efficiency was improved when the phosphodiester bond moiety between a nucleotide at a 5' terminus and 1 to 5 nucleotides adjacent to the nucleotide at a 5' terminus was substituted to a phosphorothioate bond in the constitution of GEO-8.

Further, the relative editing efficiency of GEO-162 was 3.64, that of GEO-163 was 2.04, that of GEO-164 was 3.08, that of GEO-30 was 4.17, that of GEO-165 was 4.24, that of GEO-272 was 3.40, and that of GEO-196 was 3.97. From these results, it was shown that the editing efficiency was improved when the phosphodiester bond moiety between a nucleotide at a 3' terminus and 1 to 7 nucleotides adjacent to the nucleotide at a 3' terminus was substituted to a phosphorothioate bond in the constitution of GEO-8.

Further, the relative editing efficiency of GEO-31 was 5.68. From the result, it was shown that the editing efficiency was improved when the phosphodiester bond moiety between a nucleotide at a 5' terminus and consecutive 4 nucleotides adjacent to the nucleotide at a 5' terminus, and a nucleotide at a 3' terminus and consecutive 4 nucleotides adjacent to the nucleotide at a 3' terminus was substituted to a phosphorothioate bond in the constitution of GEO-8.

Further from Table 6, the relative editing efficiency of GEO-126 was 0.99, and that of GEO-127 was 0.70. From these results, it was found that the editing efficiency was equivalent to that of GEO-8 when the phosphodiester bond moiety between LNA adjacent to the 5' upstream side of the mismatch nucleotide and consecutive 2 or 4 nucleotides further adjacent to the LNA was substituted to a phosphorothioate bond in the constitution of GEO-8.

Further from Table 6, the relative editing efficiency of GEO-128 was 0.55, and that of GEO-129 was 2.15. From these results, it was shown that an editing efficiency which was a half of that of GEO-8 was exhibited when the phosphodiester bond moiety between consecutive 2 nucleotides adjacent to further 3' downstream side of LNA adjacent to a 3' downstream side of the mismatch nucleotide was substituted to a phosphorothioate bond, an editing efficiency was improved when the phosphodiester bond moiety between consecutive 4 nucleotides adjacent to further 3' downstream side of LNA adjacent to 3' downstream side of the mismatch nucleotide was substituted to a phosphorothioate bond in the constitution of GEO-8. Here, as shown in Table 6 and FIG. 8C, in GEO-128 and GEO-129, the phosphodiester bond moiety between LNA adjacent to a 3' downstream side of the mismatch nucleotide and a nucleotide directly adjacent to the LNA is not substituted to a phosphorothioate bond.

<u>Example 1-7: Substitution of Phosphodiester Bond Moiety Between One or More Nucleotides Contained in Non-Naturally Occurring Polynucleotide to Phosphorothioate Bond (2)</u>

Example 1-7 explored the editing efficiency of the constitution in which the phosphodiester bond moiety between one or more nucleotides was further substituted to a phosphorothioate bond relative to the constitution of GEO-17 of Example 1-3.

Specifically, the editing efficiency was measured in the same manner as Example 1-1 using GEO-155, GEO-167, GEO-168 and GEO-169 in which the phosphodiester bond moiety between a nucleotide at a 3' terminus and consecutive 1 to 4 nucleotides adjacent to the nucleotide at a 3' terminus was substituted to a phosphorothioate bond in GEO-17 (FIG. 9).

The nucleotide sequence, chain length and relative editing efficiency of each editing nucleic acid mentioned above are as given in Tabel 7.

[Table 7]

Table 7

| Editing Nucleic Acid | SEQ ID NO | Sequence | Chain Length (nt) | Relative Editing Efficiency |
|---|---|---|---|---|
| GEO-8 | 9 | G t t c a a g g a c t T <u>g</u> G t c c a g g t t g t a | 25 | 1.00 |
| GEO-17 | 24 | G t t c a a g g a c t T <u>g</u> G t c c a g g t t g t A | 25 | 2.00 |
| GEO-155 | 47 | G t t c a a g g a c t T <u>g</u> G t c c a g g t t g t*A | 25 | 3.63 |
| GEO-167 | 48 | G t t c a a g g a c t T <u>g</u> G t c c a g g t t g*t*A | 25 | 6.05 |
| GEO-168 | 49 | G t t c a a g g a c t T <u>g</u> G t c c a g g t t*g*t*A | 25 | 5.81 |

(continued)

| Editing Nucleic Acid | SEQ ID NO | Sequence | Chain Length (nt) | Relative Editing Efficiency |
|---|---|---|---|---|
| GEO-169 | 50 | G t t c a a g g a c t T g G t c c a g g t*t*g*t*A | 25 | 2.90 |

In Table 7, a guanosine monophosphate which was a mismatch nucleotide was underlined, and the nucleotide substituted to a bridged nucleic acid was shown in capitals. All the bridged nucleic acids in Table 7 were LNA. An asterisk (*) was inserted between the corresponding two or more nucleotides at the position in which the phosphodiester bond moiety between two or more nucleotides was substituted to a phosphorothioate bond.

As shown in Table 7, the relative editing efficiency of GEO-155 was 3.63, that of GEO-167 was 6.05, that of GEO-168 was 5.81, and that of GEO-169 was 2.90. From these results, it was shown that the editing efficiency was improved when the phosphodiester bond moiety between a nucleotide at a 3' terminus and 1 to 4 nucleotides adjacent to the nucleotide at a 3' terminus was substituted to a phosphorothioate bond in the constitution of GEO-17.

Example 1-8: Chain Lengths of 5' Upstream Side and 3' Downstream Side of Mismatch Nucleotide, and Position of LNA

Example 1-8 explored the influence on the editing efficiency when the chain length at a 5' upstream region and the chain length at a 3' downstream region of a mismatch nucleotide were changed, in the case where the mismatch nucleotide contained in a non-naturally occurring polynucleotide was one. Further, the editing efficiency was explored when the number and the position of LNA near the 5' terminus, and the number and the position of LNA near the 3' terminus were properly changed.

Specifically, the editing efficiency was measured in the same manner as Example 1-1 using each of GEO-25 in which the chain length was 41 nt, the 5' upstream region of the mismatch nucleotide G was 20 nt and 3' downstream region thereof was 20 nt, 1 nucleotide at the 5' upstream side and 3' downstream side adjacent to the mismatch nucleotide G was LNA, and a nucleotide at a 5' terminus was LNA; GEO-311 in which the chain length was 38 nt, the 5' upstream region of the mismatch nucleotide G was 25 nt and 3' downstream region thereof was 12 nt, 1 nucleotide at the 5' upstream side and 3' downstream side adjacent to the mismatch nucleotide G was LNA, and a nucleotide at a 5' terminus was LNA; GEO-312 in which the nucleotide in 12th position toward the 5' upstream side calculated from the nucleotide at the 5' upstream side adjacent to the mismatch nucleotide G was LNA in GEO-311; GEO-313 in which 1 nucleotide adjacent to LNA at the 5' terminus was LNA in GEO-311; GEO-314 in which 1 nucleotide adjacent to LNA at the 5' terminus was LNA, and the nucleotide in 12th position toward the 5' upstream side calculated from the nucleotide at the 5' upstream side adjacent to the mismatch nucleotide G was LNA in GEO-311; GEO-315 in which the consecutive 2 nucleotides adjacent to LNA at the 5' terminus was LNA in GEO-311; as well as GEO-316 in which the consecutive 2 nucleotides adjacent to LNA at the 5' terminus was LNA, and the nucleotide in 12th position toward the 5' upstream side calculated from the nucleotide at the 5' upstream side adjacent to the mismatch nucleotide G was LNA in GEO-311 (FIG. 10A).

Further specifically, the editing efficiency was measured in the same manner as Example 1-1 using each of GEO-317 in which the chain length was 40 nt, the 5' upstream region of the mismatch nucleotide G was 12 nt and the 3' downstream region thereof was 27 nt, 1 nucleotide at the 5' upstream side and 3' downstream side adjacent to the mismatch nucleotide G was LNA, and nucleotides at a 5' terminus and a 3' terminus were LNA; GEO-318 in which the nucleotide in 12th position toward the 3' downstream side calculated from the nucleotide at the 3' downstream side adjacent to the mismatch nucleotide G was LNA in GEO-317; GEO-319 in which 1 nucleotide adjacent to LNA at the 3' terminus was LNA in GEO-317; GEO-320 in which 1 nucleotide adjacent to LNA at the 3' terminus was LNA, and the nucleotide in 12th position toward the 3' downstream side calculated from the nucleotide at the 3' downstream side adjacent to the mismatch nucleotide G was LNA in GEO-317; GEO-321 in which the consecutive 2 nucleotides adjacent to LNA at the 3' terminus was LNA in GEO-317; as well as GEO-322 in which the consecutive 2 nucleotides adjacent to LNA at the 3' terminus was LNA, and the nucleotide in 12th position toward the 3' downstream side calculated from the nucleotide of the 3' downstream side adjacent to the mismatch nucleotide G was LNA in GEO-317 (FIG. 10B).

The nucleotide sequence, chain length and relative editing efficiency of each editing nucleic acid mentioned above are as given in Table 8.

[Table 8]

Table 8

| Editing Nucleic Acid | SEQ ID NO | Sequence | Chain Length (nt) | Relative Editing Efficiency |
|---|---|---|---|---|
| GE0-8 | 9 | G t t c a a g g a c t T g G t c c a g g t t g t a | 25 | 1.00 |
| GEO-25 | 17 | A c c t c c c t g t t c a a g g a c t T g G t c c a g g t t g t a g c c g g c t g | 41 | 3.47 |
| GEO-311 | 51 | G a c a c a c c t c c c t g t t c a a g g a c t T g G t c c a g g t t g t a | 38 | 3.31 |
| GEO-312 | 52 | G a c a c a c c t c c c t G t t c a a g g a c t T g G t c c a g g t t g t a | 38 | 4.16 |
| GEO-313 | 53 | G A c a c a c c t c c c t g t t c a a g g a c t T g G t c c a g g t t g t a | 38 | 5.23 |
| GEO-314 | 54 | G A c a c a c c t c c c t G t t c a a g g a c t T g G t c c a g g t t g t a | 38 | 6.57 |
| GEO-315 | 55 | G A C a c a c c t c c c t g t t c a a g g a c t T g G t c c a g g t t g t a | 38 | 6.59 |
| GEO-316 | 56 | G A C a c a c c t c c c t G t t c a a g g a c t T g G t c c a g g t t g t a | 38 | 7.02 |
| GE0-317 | 57 | G t t c a a g g a c t T g G t c c a g g t t g t a g c c g g c t g t c t g t c G | 40 | 2.55 |
| GEO-318 | 58 | G t t c a a g g a c t T g G t c c a g g t t g t A g c c g g c t g t c t g t c G | 40 | 2.66 |
| GEO-319 | 59 | G t t c a a g g a c t T g G t c c a g g t t g t a g c c g g c t g t c t g t C G | 40 | 2.18 |
| GEO-320 | 60 | G t t c a a g g a c t T g G t c c a g g t t g t A g c c g g c t g t c t g t C G | 40 | 2.51 |
| GEO-321 | 61 | G t t c a a g g a c t T g G t c c a g g t t g t a g c c g g c t g t c t g T C G | 40 | 1.40 |
| GEO-322 | 62 | G t t c a a g g a c t T g G t c c a g g t t g t A g c c g g c t g t c t g T C G | 40 | 0.77 |

In Table 8, a guanosine monophosphate which was a mismatch nucleotide was underlined, and the nucleotide substituted to a bridged nucleic acid was shown in capitals. All bridged nucleic acids in Table 8 were LNA.

As shown in Table 8, the relative editing efficiency of GEO-25 was 3.47, that of GEO-311 was 3.31, that of GEO-312 was 4.16, that of GEO-313 was 5.23, that of GEO-314 was 6.57, that of GEO-315 was 6.59, that of GEO-316 was 7.02, that of GEO-317 was 2.55, that of GEO-318 was 2.66, that of GEO-319 was 2.18, that of GEO-320 was 2.51, that of GEO-321 was 1.40, and that of GEO-322 was 0.77.

By the comparison of GEO-8 to GEO-311, it was shown that when the chain length at the 5' upstream region of the mismatch nucleotide was extended, the editing efficiency was increased. By comparing GEO-313 to GEO-18 of Example 1-4 and GEO-315 to GEO-19 of Example 1-4, it was shown that the editing efficiency was increased by extending the chain length at the 5' upstream region of the mismatch nucleotide, even when plural nucleotides adjacent to a nucleotide at a 5' terminus were LNA.

By comparing GEO-311 to GEO-312, GEO-313 to GEO-314, and GEO-315 to GEO-316, respectively, it was shown that the editing efficiency was further increased when the nucleotide in $12^{th}$ position toward the 5' upstream side calculated from the nucleotide at the 5' upstream side adjacent to the mismatch nucleotide was LNA.

Since GEO-317, GEO-318, GEO-319, GEO-320, GEO-321 and GEO-322 in which the chain length at the 3' downstream region of the mismatch nucleotide was extended had relatively low editing efficiency as compared to GEO-311, GEO-312, GEO-313, GEO-314, GEO-315 and GEO-316 in which the chain length at the 5' upstream region of the mismatch nucleotide was extended, it was suggested that extension of the chain length at the 5' upstream region of the mismatch nucleotide was effective.

As a result of comparison of GEO-317 to GEO-318 and GEO-319 to GEO-320 respectively, since a significant difference was not found in the editing efficiency, it was suggested that the influence of replacement of the nucleotide in 12th position toward the 3' downstream side calculated from the nucleotide at the 3' downstream side adjacent to the mismatch nucleotide G to LNA on the editing efficiency was small in the case where the chain length of the 3' downstream region of the mismatch nucleotide was extended.

Example 1-9: RNA Substitution of Nucleotide Near 3' Terminus

Example 1-9 explored the editing efficiency of a constitution in which one or more nucleotide adjacent to a nucleotide at a 3' terminus was further subjected to RNA substitution relative to the constitution of GEO-17 of Example 1-3.

Specifically, the editing efficiency was measured in the same manner as Example 1-1 using each of GEO-150 in which 1 nucleotide adjacent to a nucleotide at a 3' terminus was subjected to RNA substitution in GEO-17; GEO-73 in which consecutive 2 nucleotides adjacent to the nucleotide at a 3' terminus was subjected to RNA substitution; GEO-151 in which the consecutive 3 nucleotides adjacent to the nucleotide at a 3' terminus was subjected to RNA substitution; and GEO-74 in which the consecutive 4 nucleotides adjacent to the nucleotide at a 3' terminus was subjected to RNA substitution (FIG. 11).

The nucleotide sequence, chain length and relative editing efficiency of each editing nucleic acid mentioned above are as given in Table 9.

[Table 9]

Table 9

| Editing Nucleic Acid | SEQ ID NO | Sequence | Chain Length (nt) | Relative Editing Efficiency |
|---|---|---|---|---|
| GE0-8 | 9 | G t t c a a g g a c t T g G t c c a g g t t g t a | 25 | 1.00 |
| GEO-17 | 24 | G t t c a a g g a c t T g G t c c a g g t t g t A | 25 | 2.00 |
| GEO-150 | 63 | G t t c a a g g a c t T g G t c c a g g t t g u A<br>↑ | 25 | 1.65 |
| GEO-73 | 64 | G t t c a a g g a c t T g G t c c a g g t t g u A<br>↑ ↑ | 25 | 4.20 |
| GEO-151 | 65 | G t t c a a g g a c t T g G t c c a g g t u g u A<br>↑ ↑ ↑ | 25 | 0.78 |
| GEO-74 | 66 | G t t c a a g g a c t T g G t c c a g g u u g u A<br>↑ ↑ ↑ ↑ | 25 | 1.12 |

In Table 9, a guanosine monophosphate which was a mismatch nucleotide was underlined, and the nucleotide substituted to a bridged nucleic acid was shown in capitals. All bridged nucleic acids in Table 9 were LNA. As to the nucleotide subjected to RNA substitution, arrow was attached to the base symbol of the corresponding nucleotide.

As shown in Table 9, the relative editing efficiency of GEO-150 was 1.65, that of GEO-73 was 4.20, that of GEO-151 was 0.78, and that of GEO-74 was 1.12. From these results, it was shown that the editing efficiency was improved compared to that of GEO-8 when one or two nucleotides adjacent to the nucleotide at a 3' terminus were subjected to RNA substitution in the constitution of GEO-17, and that the editing efficiency was equivalent to that of GEO-8 when three or four nucleotides were subjected to RNA substitution.

Example 1-10: Phosphorylation of Nucleotide at 3' Terminus and/or 5' Terminus

Example 1-10 explored the editing efficiency of the constitution in which a nucleotide at a 3' terminus and/or a 5' terminus was further phosphorylated relative to the constitution of GEO-8 of Example 1-1 or GEO-17 of Example 1-3.

Specifically, the editing efficiency was measured in the same manner as Example 1-1 using each of GEO-26 in which nucleotide at a 5' terminus was phosphorylated in GEO-8; GEO-27 in which the nucleotide at a 3' terminus was phosphorylated and GEO-28 in which nucleotides at both the 5' terminus and 3' terminus were phosphorylated; GEO-223

in which the nucleotide at a 3' terminus was phosphorylated in GEO-17; and GEO-224 in which the phosphodiester bond moiety between a nucleotide at a 3' terminus and a nucleotide adjacent thereto was substituted to a phosphorothioate bond, and a nucleotide at a 3' terminus was phosphorylated in GEO-17 (FIG. 12).

The nucleotide sequence, chain length and relative editing efficiency of each editing nucleic acid mentioned above are as given in Table 10.

[Table 10]

Table 1 0

| Editing Nucleic Acid | SEQ ID NO | Sequence | Chain Length (nt) | Relative Editing Efficiency |
|---|---|---|---|---|
| GEO-8 | 9 | G t t c a a g g a c t T g G t c c a g g t t g t a | 25 | 1.00 |
| GEO-26 | 67 | p G t t c a a g g a c t T g G t c c a g g t t g t a | 25 | 1.14 |
| GEO-27 | 68 | G t t c a a g g a c t T g G t c c a g g t t g t a p | 25 | 2.23 |
| GEO-28 | 69 | p G t t c a a g g a c t T g G t c c a g g t t g t a p | 25 | 2.91 |
| GEO-17 | 24 | G t t c a a g g a c t T g G t c c a g g t t g t A | 25 | 2.00 |
| GEO-223 | 70 | G t t c a a g g a c t T g G t c c a g g t t g t A p | 25 | 2.70 |
| GEO-224 | 71 | G t t c a a g g a c t T g G t c c a g g t t g t*Ap | 25 | 2.29 |

In Table 10, a guanosine monophosphate which was a mismatch nucleotide was underlined, and the nucleotide substituted to a bridged nucleic acid was shown in capitals. All the bridged nucleic acids in Table 10 were LNA. An asterisk (*) was inserted between the corresponding two or more nucleotides at the position in which the phosphodiester bond moiety between two or more nucleotides was substituted to a phosphorothioate bond. Further, "p" was attached to a 5' terminus and/or a 3' terminus for indicating that the 5' terminus and/or the 3' terminus was phosphorylated.

As shown in Table 10, the relative editing efficiency of GEO-26 was 1.14, that of GEO-27 was 2.23, that of GEO-27 was 2.23, that of GEO-28 was 2.91, and the relative editing efficiency of GEO-223 was 2.70 and that of GEO-224 was 2.29. From these results, it was shown that the editing efficiency was not influenced by phosphorylating only 5' terminus in the constitution of GEO-8, while editing efficiency was increased by phosphorylating 3' terminus. In addition, higher editing efficiency than the case where only 3' terminus was phosphorylated was exhibited when both the 5' terminus and the 3' terminus were phosphorylated. Similarly, it was shown also in the constitution of GEO-17 that the editing efficiency was increased when the 3' terminus was phosphorylated. In this case, the increase of the editing efficiency was not acknowledged even when the phosphodiester bond moiety between a nucleotide at a 3' terminus and a nucleotide adjacent thereto was substituted to a phosphorothioate bond.

Example 1-11: Kinds of Bridged Nucleic Acid

Example 1-11 explored the editing efficiency of the constitution in which LNA at the 5' terminus was replaced to other bridged nucleic acids in the constitution of GEO-8 of Example 1-1.

Specifically, the editing efficiency was measured in the same manner as Example 1-1 using each of GEO-41 in which LNA at the 5' terminus was substituted to BNA-N-H; GEO-43 in which LNA at the 5' terminus was substituted to BNA-N-Me; and GEO-57 in which LNA at the 5' terminus was substituted to ENA in GEO-8 (FIG. 13).

The nucleotide sequence, chain length and relative editing efficiency of each editing nucleic acid mentioned above are as given in Table 11.

[Table 11]

Table 1 1

| Editing Nucleic Acid | SEO ID NO | Sequence | Chain Length (nt) | Relative Editing Efficiency |
|---|---|---|---|---|
| GEO-8 | 9 | G t t c a a g g a c t T g G t c c a g g t t g t a | 25 | 1. 00 |
| GEO-41 | 72 | G t t c a a g g a c t T g G t c c a g g t t g t a | 25 | 1.38 |
| GEO-43 | 73 | G t t c a a g g a c t T g G t c c a g g t t g t a | 25 | 1.51 |
| GEO-57 | 74 | G t t c a a g g a c t T g G t c c a g g t t g t a | 25 | 1.57 |

In Table 11, a guanosine monophosphate which was a mismatch nucleotide was underlined, and the nucleotide substituted to a bridged nucleic acid was shown in capitals. The kinds of bridged nucleic acid in each editing nucleic acid in Table 11 is as mentioned above.

As shown in Table 11, the relative editing efficiency of GEO-41 was 1.38, that of GEO-43 was 1.51, and that of GEO-57 was 1.57. From these results, it was shown that the 5' terminus may be substituted to not only LNA, but also other bridged nucleic acids such as BNA-N-H, BNA-N-Me and ENA, and the substitution may rather increase the editing efficiency in the constitution of GEO-8.

Example 1-12: Combination of Nucleic Acid Modifications (1)

Example 1-12 explored the editing efficiency of the constitution in which the substitution of the phosphodiester bond moiety to a phosphorothioate bond and the phosphorylation of 3' terminus were further combined in the constitution of GEO-8.

Specifically, the editing efficiency was measured in the same manner as Example 1-1 using GEO-226 in which the phosphodiester bond moiety between the consecutive 5 nucleotides adjacent to a nucleotide at a 3' terminus was substituted to a phosphorothioate bond, and the 3' terminus was phosphorylated in GEO-8 (FIG. 14).

The nucleotide sequence, chain length and relative editing efficiency of each editing nucleic acid mentioned above are as given in Table 12.

[Table 12]

Table 1 2

| Editing Nucleic Acid | SEQ ID NO | Sequence | Chain Length (nt) | Relative Editing Efficiency |
|---|---|---|---|---|
| GEO-8 | 9 | G t t c a a g g a c t T g G t c c a g g t t g t a | 25 | 1.00 |
| GEO-226 | 75 | G t t c a a g g a c t T g G t c c a g g*t*t*g*t*a p | 25 | 8.19 |

In Table 12, a guanosine monophosphate which was a mismatch nucleotide was underlined, and the nucleotide substituted to a bridged nucleic acid was shown in capitals. All bridged nucleic acids in Table 12 were LNA. An asterisk (*) was inserted between the corresponding two or more nucleotides at the position in which the phosphodiester bond moiety between two or more nucleotides was substituted to a phosphorothioate bond. Further, "p" was attached to a 3' terminus for indicating that the 3' terminus was phosphorylated.

As shown in Table 12, the relative editing efficiency of GEO-226 was 8.19. From the result, it was shown that the editing efficiency was extremely increased by further combining the substitution of the phosphodiester bond moiety to a phosphorothioate bond and phosphorylation of 3' terminus in the constitution of GEO-8.

Example 1-13: Combination of Nucleic Acid Modifications (2)

Example 1-13 explored the editing efficiency of the constitution in which the substitution of the phosphodiester bond moiety to a phosphorothioate bond, RNA substitution and phosphorylation of 3' terminus were further combined in the constitution of GEO-23 of Example 1-3.

Specifically, the editing efficiency was measured in the same manner as Example 1-1 using GEO-172 in which the phosphodiester bond moiety between the consecutive 4 nucleotides adjacent to a nucleotide at a 5' terminus was

substituted to a phosphorothioate bond, the consecutive 2 nucleotides adjacent to a nucleotide at a 3' terminus were subjected to RNA substitution, and the 3' terminus was phosphorylated in GEO-23 (FIG. 15).

The nucleotide sequence, chain length and relative editing efficiency of each editing nucleic acid mentioned above are as given in Table 13.

[Table 13]

Table 13

| Editing Nucleic Acid | SEQ ID NO | Sequence | Chain Length (nt) | Relative Editing Efficiency |
|---|---|---|---|---|
| GEO-23 | 14 | C t g t t c a a g g a c t T <u>g</u> G t c c a g g t t g t a g c | 29 | 3. 16 |
| GE0-172 | 76 | C*t*g*t*t c a a g g a c t T <u>g</u> G t c c a g g t t g t a g Cp<br>↑ ↑ | 29 | 8.48 |

In Table 13, a guanosine monophosphate which was a mismatch nucleotide was underlined, and the nucleotide substituted to a bridged nucleic acid was shown in capitals. All bridged nucleic acids in Table 13 were LNA. An asterisk (*) was inserted between the corresponding two or more nucleotides at the position in which the phosphodiester bond moiety between two or more nucleotides was substituted to a phosphorothioate bond. As to the nucleotide subjected to the RNA substitution, arrows were attached to the base symbol of the corresponding nucleotide. Further, "p" was attached to a 3' terminus for indicating that the 3' terminus was phosphorylated.

As shown in Table 13, the relative editing efficiency of GEO-172 was 8.48. From the result, it was shown that the editing efficiency was significantly increased by further combining the substitution of the phosphodiester bond moiety to a phosphorothioate bond, the RNA substitution and the phosphorylation of the 3' terminus in the constitution of GEO-17.

<u>Discussion</u>

The above Example 1-1 to Example 1-13 of the present invention and disclosures in Patent Publications and Non-Patent Publications were compared.

(1) From Example 1-1, it was found that GEO-8 which is the non-naturally occurring polynucleotide of the present invention exhibited higher editing efficiency by 5 folds or more against GEO-7 corresponding to the polynucleotide having highest editing efficiency among the constitutions disclosed in Non-Patent Publication 1 (i.e., chain length, the position of mismatch nucleotide and the position of LNA are identical to those of GEO-8). When comparing GEO-8 and GEO-7, GEO-8 of the present invention is different in the point that the mismatch nucleotide itself is not LNA, and both the nucleotide at 5' side and the nucleotide at 3' side adjacent to the mismatch nucleotide are substituted to LNA.

With respect to the point, Patent Publication 4 describes that an oligonucleotide containing LNA adjacent to a mismatch nucleotide did not exhibit improvement, but an oligonucleotide having LNA placed at a distance of 1 nucleotide from mismatch exhibited 5 folds of average increase, and it can be said that Patent Publication 4 aggressively excludes the constitution containing LNA adjacent to the mismatch nucleotide.

Non-Patent Publication 1 focuses on an avoidance from the mismatch repair, and further Non-Patent Publications 1 and 2 focus on an elucidation of a mechanism of how a synthetic single-stranded DNA in which a mismatch nucleotide is LNA carries out genome alteration of mammalian cell. Among total 60 or more kinds of synthetic single-stranded DNAs which was tested in Non-Patent Publication 1, a nucleotide sequence in which both the nucleotide at a 5' side and the nucleotide at a 3' side adjacent to a mismatch nucleotide are substituted to LNA is only one kind, and all of the total 50 or more kinds of single-stranded DNAs tested in Non-Patent Publication 2 are a nucleotide sequence in which mismatch nucleotide itself is LNA, and a nucleotide sequence in which both the nucleotide at a 5' side and the nucleotide at a 3' side adjacent to a mismatch nucleotide are substituted to LNA was not tested. In addition, the discussion of Non-Patent Publication 1 emphasizes that the substitution of mismatch nucleotide itself to LNA is important for avoidance of the mismatch repair, and the publication does not refer to a nucleotide sequence in which both the nucleotide at a 5' side and the nucleotide at a 3' side adjacent to a mismatch nucleotide are substituted to LNA.

Accordingly, it can be said that the descriptions of Patent Publication 4, and Non-Patent Publications 1 and 2 are an inhibitory factor for a motivation for adopting features that both the nucleotide at a 5' side and the nucleotide at a 3' side adjacent to a mismatch nucleotide are substituted to LNA for improving the editing efficiency, i.e. features (A) and (B) of the present invention. Further, the experimentation results shown in Example 1-1 of the specification are directly opposite to the suggestion of Patent Publication 4, and it can be also said that the effects of the present invention cannot be expected from Patent Publication 4 and Non-Patent Publications 1 and 2 from the point of the effects of the invention.

Among total 110 or more kinds of synthetic single-stranded DNAs tested in Non-Patent Publications 1 and 2, a synthetic single-stranded DNA in which a nucleotide at a 5' terminus was LNA and a 3' terminus was not LNA was only one kind in each publication. A synthetic single-stranded DNA in which two of a nucleotide at a 5' terminus and a mismatch nucleotide were LNA exhibited about 2 folds of editing efficiency as compared to a synthetic single-stranded DNA in which only a mismatch nucleotide was LNA, nevertheless the synthetic single-stranded DNA in which the 5' terminus was LNA was not studied any more.

Non-Patent Publication 2 concludes that a region more 5' side than a mismatch nucleotide was degraded by an endonuclease but not an exonuclease, and therefore, suggesting that the effect of modifying a 5' terminus with LNA to protect from the exonuclease is restrictive. In fact, since the LNA modification of 5' terminus increased the editing efficiency by only two folds, it is led that the LNA modification of 5' terminus is not so important. Therefore, the combination of a nucleotide sequence in which both the nucleotide at a 5' side and the nucleotide at a 3' side adjacent to a mismatch nucleotide are substituted to LNA and the LNA modification of 5' terminus is not tested.

Accordingly, it can be said that the descriptions of Non-Patent Publications 1 and 2 are an inhibitory factor for an attempt to substitute the 5' terminus of the non-naturally occurring polynucleotide to LNA to improve an editing efficiency.

(2) From Example 1-2, it was found that a certain level or more of editing efficiency was acknowledged if the chain length of the non-naturally occurring polynucleotide was 25 nt or more and 95 nt or less.

Regarding chain length, conventionally there have been many publications describing that nucleotide having 25 bases or so is preferred, and the longer the chain length is, the lower the effects are, whereas the present invention is different from the existing studies in the point that the effects were high in the present invention even if the chain length was long. Specifically, Non-Patent Publication 1 studies the optical length of the editing nucleotide in a polynucleotide in which only a mismatch nucleotide was LNA, and as a result, it is shown that the 25 nucleotides in length are optical, and the editing efficiency is decreased in a nucleotide having a length longer than 25 nucleotides. In addition, Patent Publication 4 uses a polynucleotide having 24 nucleotides in length.

On the other hand, it has been discovered that the editing efficiency is further enhanced in a nucleotide having a length longer than 25 nucleotides in the non-naturally occurring polynucleotide of the present invention.

(3) It was shown from Example 1-3 that the feature (C) a nucleotide at a 5' terminus is a bridged nucleic acid is important for the purpose that a nucleotide sequence in which both the nucleotide at a 5' side and the nucleotide at a 3' side adjacent to a mismatch nucleotide are substituted to LNA exhibits an effect. Further, it was found from the comparison of GEO-8, GEO-16 and GEO-17 that when both nucleotides at a 5' terminus and a 3' terminus are a bridged nucleic acid, the editing efficiency was elevated by two folds. In addition, it was found that the effect of a nucleotide at a 3' terminus being LNA was strongly exhibited when a nucleotide at a 5' terminus was LNA.

(4) Example 1-4 and Example 1-5 showed that a sufficient editing efficiency was exhibited even if one or more nucleotides adjacent to a 5' terminus or a 3' terminus were substituted to LNA, and there was a case where an editing efficiency may be improved depending on the position and the number of the nucleotide substituted to LNA. Such effects were neither described nor suggested in any of Patent Publications and Non-Patent Publications mentioned above.

(5) It was shown from Example 1-6 and Example 1-7 that in a nucleotide sequence in which both the nucleotide at a 5' side and the nucleotide at a 3' side adjacent to a mismatch nucleotide are substituted to LNA, an editing efficiency was improved when the phosphodiester bond moiety between a nucleotide at a 5' terminus or a 3' terminus and 1 to 7 nucleotides adjacent thereto was substituted to a phosphorothioate bond. Since the editing efficiency is not decreased even if the number of a phosphorothioate bond is increased by one in both of a 5' terminal side and a 3' terminal side, it is lead that a sufficient editing efficiency is probably exhibited even if the number of the phosphodiester bond is 8 or more. Such effects were neither described nor suggested in any of Patent Publications and Non-Patent Publications mentioned above. In addition, the effect that S-addition from both ends especially strongly improves the editing effect when a 5' terminus or a 3' terminus is LNA is a completely new discovery which the present inventors disclosed in the specification.

(6) It was shown from Example 1-8 that the editing efficiency was increased when the chain length of a 5' upstream region and a 3' downstream region of the mismatch nucleotide was extended, and further it was shown that the editing efficiency was increased by substituting some nucleotides in the extended region to LNA. Such effects were neither described nor suggested in any of Patent Publications and Non-Patent Publications mentioned above.

(7) From Example 1-9, Example 1-10, Example 1-12 and Example 1-13, the effects that the editing efficiency was significantly increased by combining the modifications such as the substitution of the phosphodiester bond moiety to a phosphorothioate bond, the RNA substitution, and the phosphorylation of a 3' terminus or the phosphorylation of a 5' terminus, in addition to the substitution to a bridged nucleic acid were exhibited. Such effects were neither described nor

suggested in any of Patent Publications and Non-Patent Publications mentioned above.

(8) It was shown from Example 1-11 that regarding the substitution of the 5' terminus to a bridged nucleic acid, the improving effect of the editing efficiency was exhibited by substituting the terminus to not only LNA, but also other bridged nucleic acids such as BNA-N-H, BNA-N-Me and ENA. Such effects were neither described nor suggested in any of Patent Publications and Non-Patent Publications mentioned above.

B. Genome Editing Experiments Using 293-nLD2 Cell, 293-nLD3 Cell, 293-nLD4-1 Cell, 293-nLD4-2 Cell, 293-nLD4-3 Cell, 293-nLD5 Cell, 293-nLD6 Cell, 293-nLD7 Cell, 293-nLD8 Cell or 293-nLD9 Cell 1. Preparation of Various Mutant NanoLuc(registered trademark) Plasmids

(1) nDL2

A mutant nucleotide sequence in which a guanine (G) at the nucleotide number 894 was changed to an adenine (A) and a cytosine (C) at the nucleotide number 895 was changed to a thymine (T) in a NanoLuc gene was synthesized, to prepare a mutant in which the mutant nucleotide was incorporated into a pcDNA™5/FRT/TO plasmid in the same manner as the above (pcDNA5-nLD2). The luciferase gene owned by the mutant is an inactivated luciferase gene not exhibiting a luciferase activity due to the above mutation.

(2) nDL3

A mutant nucleotide sequence in which a thymine (T) at the nucleotide number 956 was changed to an adenine (A), a thymine (T) at the nucleotide number 957 was changed to a guanine (G), and a cytosine (C) at the nucleotide number 958 was changed to a thymine (T) in a NanoLuc gene was synthesized, to prepare a mutant in which the mutant nucleotide was incorporated into a pcDNA™5/FRT/TO plasmid in the same manner as the above (pcDNA5-nLD3). The luciferase gene owned by the mutant is an inactivated luciferase gene not exhibiting a luciferase activity due to the above mutation.

(3) nDL4-1

A mutant nucleotide sequence in which a cytosine (C) at the nucleotide number 922 was changed to a thymine (T), and a guanine (G) at the nucleotide number 931 was changed to a thymine (T) in NanoLuc gene was synthesized, to prepare a mutant in which the mutant nucleotide was incorporated into a pcDNA™5/FRT/TO plasmid in the same manner as the above (pcDNA5-nLD4-1). The luciferase gene owned by the mutant is an inactivated luciferase gene not exhibiting a luciferase activity due to the above mutation.

(4) nDL4-2

A mutant nucleotide sequence in which a cytosine (C) at the nucleotide number 922 was changed to a thymine (T), and a guanine (G) at the nucleotide number 937 was changed to a thymine (T) in NanoLuc gene was synthesized, to prepare a mutant in which the mutant nucleotide was incorporated into a pcDNA™5/FRT/TO plasmid in the same manner as the above (pcDNA5-nLD4-2). The luciferase gene owned by the mutant is an inactivated luciferase gene not exhibiting a luciferase activity due to the above point mutation.

(5) nDL4-3

A mutant nucleotide sequence in which a guanine (G) at the nucleotide number 937 was changed to a thymine (T), and a cytosine (C) at the nucleotide number 958 was changed to a thymine (T) in NanoLuc gene was synthesized, to prepare a mutant in which the mutant nucleotide was incorporated into a pcDNA™5/FRT/TO plasmid in the same manner as the above (pcDNA5-nLD4-3). The luciferase gene owned by the mutant is an inactivated luciferase gene not exhibiting a luciferase activity due to the above point mutation.

(6) nDL5

A mutant nucleotide sequence in which a guanine (G) at the nucleotide number 976 was deleted in NanoLuc gene was synthesized, to prepare a mutant in which the mutant nucleotide was incorporated into a pcDNA™5/FRT/TO plasmid in the same manner as the above (pcDNA5-nLD5). The luciferase gene owned by the mutant is an inactivated luciferase gene not exhibiting a luciferase activity due to the above point mutation.

(7) nDL6

A mutant nucleotide sequence in which an adenine (A) at the nucleotide number 911 and a cytosine (C) at the nucleotide number 912 were deleted in NanoLuc gene was synthesized, to prepare a mutant in which the mutant nucleotide was incorporated into a pcDNA™5/FRT/TO plasmid in the same manner as the above (pcDNA5-nLD6). The luciferase gene owned by the mutant is an inactivated luciferase gene not exhibiting a luciferase activity due to the above point mutation.

(8) nDL7

A mutant nucleotide sequence in which a thymine (T) was inserted between a thymine (T) at the nucleotide number 930 and a guanine (G) at the nucleotide number 931 in NanoLuc gene was synthesized, to prepare a mutant in which the mutant nucleotide was incorporated into a pcDNA™5/FRT/TO plasmid in the same manner as the above (pcDNA5-nLD7). The luciferase gene owned by the mutant is an inactivated luciferase gene not exhibiting a luciferase activity due to the above point mutation.

(9) nDL8

A mutant nucleotide sequence in which a thymine (T) and an adenine (A) were inserted in this order between an adenine (A) at the nucleotide number 978 and an adenine (A) at the nucleotide number 979 in NanoLuc gene was synthesized, to prepare a mutant in which the mutant nucleotide was incorporated into a pcDNA™5/FRT/TO plasmid in the same manner as the above (pcDNA5-nLD8). The luciferase gene owned by the mutant is an inactivated luciferase gene not exhibiting a luciferase activity due to the above point mutation.

(10) nDL9

A mutant nucleotide sequence in which a thymine (T) at the nucleotide number 981, a cytosine (C) at the nucleotide number 982, a cytosine (C) at the nucleotide number 983 and a guanine (G) at the nucleotide number 984 were deleted in NanoLuc gene was synthesized, to prepare a mutant in which the mutant nucleotide was incorporated into a pcDNA™5/FRT/TO plasmid in the same manner as the above (pcDNA5-nLD9). The luciferase gene owned by the mutant is an inactivated luciferase gene not exhibiting a luciferase activity due to the above point mutation.

2. Preparation of Stable Transformed Cell Line Having Various Mutant NanoLuc Genes

Flp-In™-293 cell line which was a cell line derived from a human (Thermo Fisher Scientific Inc.) was seeded to a 6 cm dish to grow up to $1x10^6$ cells, and the cells were cultured in Dulbecco's Modified Eagle Medium (DMEM, Thermo Fisher Scientific Inc.) containing 10% concentration of fetal bovine serum (FBS, Thermo Fisher Scientific Inc.) under an environment of 5% $CO_2$ at 37°C. After 24 hours, pcDNA5-nLD2, pcDNA5-nLD3, pcDNA5-nLD4-1, pcDNA5-nLD4-2, pcDNA5-nLD4-3, pcDNA5-nLD5, pcDNA5-nLD6, pcDNA5-nLD7, pcDNA5-nLD8 or pcDNA5-nLD9 plasmid (1 μg) and pOG44 plasmid (3 μg, Thermo Fisher Scientific Inc.) was transfected according to a usual protocol using Lipofectamine 3000 (Thermo Fisher Scientific Inc.). After 48 hours, TrypLE™ Express Enzyme (Thermo Fisher Scientific Inc.) was added, the mixture was incubated for 3 minutes at 37°C, the detached cells were collected, the cells were suspended into DMEM + 10% FBS medium containing hygromycin (Thermo Fisher Scientific Inc.) at a concentration of 50 μg/ml, and the suspended cells were separately cultured in two 10 cm dishes. Thereafter, the culture continued while exchanging the medium with the same medium every 3 days. After the culture for about 20 days, the formation of colonies with sufficient number and size was confirmed, and all cells were detached from a petri dish using TrypLE™ Express Enzyme and collected.

Each cell line obtained using the above various plasmids was named as follows.

Cells in which pcDNA5-nLD2 was incorporated into a genome were named as 293-nLD2 cell. Cells in which pcDNA5-nLD3 was incorporated into a genome were named as 293-nLD3 cell. Cells in which pcDNA5-nLD4-1 was incorporated into a genome were named as 293-nLD4-1 cell. Cells in which pcDNA5-nLD4-2 was incorporated into a genome were named as 293-nLD4-2 cell. Cells in which pcDNA5-nLD4-3 was incorporated into a genome were named as 293-nLD4-3 cell. Cells in which pcDNA5-nLD5 was incorporated into a genome were named as 293-nLD5 cell. Cells in which pcDNA5-nLD6 was incorporated into a genome were named as 293-nLD6 cell. Cells in which pcDNA5-nLD7 was incorporated into a genome were named as 293-nLD7 cell. Cells in which pcDNA5-nLD8 was incorporated into a genome were named as 293-nLD8 cell. Cells in which pcDNA5-nLD9 was incorporated into a genome were named as 293-nLD9 cell.

The mutant NanoLuc gene owned by the above each cell is an inactivated luciferase gene as mentioned above.

The above each cell was used in the following genome editing experiments.

## 3. Introduction of Non-Naturally Occurring Polynucleotide into 293-nLD2 to 9 Cells

293-nLD2 cell was seeded onto a 96 well plate (Nunc(registered trademark) MicroWell™ 96, Nunclon Delta-Treated, Flat-Bottom Microplate, Thermo Fisher Scientific Inc.) to grow up to 1x$10^4$ cells, and the cells were cultured in DMEM + 10% FBS medium under an environment of 5% $CO_2$ at 37°C. After 24 hours, non-naturally occurring polynucleotide in each concentration of 0.0125 μg, 0.025 μg, 0.050 μg, 0.100 μg and 0.200 μg was transfected according to a usual protocol with Lipofectamine 3000.

293-nLD3 cell, 293-nLD4-1 cell, 293-nLD4-2 cell, 293-nLD4-3 cell, 293-nLD5 cell, 293-nLD6 cell, 293-nLD7 cell, 293-nLD8 cell and 293-nLD9 cell were also transfected in the same manner as that of 293-nLD2 cell.

## 4. Measurement of Editing Efficiency

• NanoLuc Luciferase Assay

After continuing the culture for 72 hours since the transfection, NanoLuc Luciferase activity was measured. NanoLuc Luciferase activity was measured according to a usual protocol using Nano-Glo(registered trademark) Luciferase Assay System (Promega Corporation). EnSpire multimode plate reader (PerkinElmer Co., Ltd.) was used for the measurement of luminescence of luciferase.

• Count of Living Cells

The number of living cells were measured according to a usual protocol using CellTiter-Blue(registered trademark) Cell Viability Assay (Promega Corporation). EnSpire multimode plate reader (PerkinElmer Co., Ltd.) was used for the measurement of fluorescent value of CellTiter-Blue.

For the number of living cells of edited cells, 2-folds dilution series of 293-nLD2 cell number between 5 x $10^3$ and 2 x $10^5$ was used to create a calibration curve of measured value of CellTiter-Blue and the number of cells, to calculate the numbers of living cells based on the calibration curve.

For also 293-nLD3 cell, 293-nLD4-1 cell, 293-nLD4-2 cell, 293-nLD4-3 cell, 293-nLD5 cell, 293-nLD6 cell, 293-nLD7 cell, 293-nLD8 cell and 293-nLD9 cell, the number of living cells were calculated in the same manner as that of 293-nLD2 cell.

• Calculation of Editing Efficiency

NanoLuc Luciferase activity of 293-nLW1 cell in which wild-type luciferase was incorporated into a genome was measured, and as a result, the measurement was about 20,000 counts/cell. Accordingly, if the mutation of the inactivated luciferase gene is repaired and wild-type NanoLuc is synthesized, 20,000 counts of luciferase activity per cell will be detected. Based on the value, the editing efficiency (%) was calculated according to the formula: NanoLuc Luciferase Activity / 20,000 / Number of Cells x 100.

## 5. Evaluation of Editing Efficiency of Non-Naturally Occurring Polynucleotide by AUC Log

The evaluation of the editing efficiency of each non-naturally occurring polynucleotide was carried out as follows.

NanoLuc Luciferase activity was measured at 5 points of concentration (0.0125 μg, 0.025 μg, 0.050 μg, 0.100 μg, and 0.200 μg) of non-naturally occurring polynucleotide, and Dose-response curve (Dose-response curve) in which an axis showing the concentration of non-naturally occurring polynucleotide (X axis) was defined as logarithm was created. Value in which X axis of Area under the curve (Area under the curve, hereinafter referred to as "AUC") was defined as logarithm (hereinafter, referred to as "AUC log") was obtained from the created Dose-response curve. Prism (GraphPad) was used for the calculation of AUC log.

## 6. Synthesis of Non-Naturally Occurring Polynucleotide

All non-naturally occurring polynucleotide including modification of nucleic acid were synthesized in Gene Design Inc. (Japan), which was purified by a simple column or HPLC.

Example 2-1: Study of Two Nucleotide Substitution (1)

An experiment measuring the editing efficiency of the target polynucleotide sequence was carried out by introducing the non-naturally occurring polynucleotide into 293-nLD2 cell.

In the Example, each of 5 kinds of the non-naturally occurring polynucleotides of D2-1 to D2-6 was introduced into 293-nLD2 cell by a transfection method.

The nucleotide sequence, chain length and relative editing efficiency of each editing nucleic acid mentioned above are as given in Table 14.

[Table 14]

Table 1 4

| Editing Nucleic Acid | SEQ ID NO | Sequence | Chain Length (nt) | Relative Editing Efficiency |
|---|---|---|---|---|
| D2-1 | 77 | t a g c c g g c t g t c t g t c g c c a g t c c c c a a c g a a a t c | 35 | 0.13 |
| D2-2 | 78 | t a g c c g g c t g t c t g t C G C C a g t c c c c a a c g a a a t c | 35 | 1.00 |
| D2-3 | 79 | T a g c c g g c t g t c t g t C G C C a g t c c c c a a c g a a a t c | 35 | 4.94 |
| D2-4 | 80 | T c c a g g t t g t a g c c g g c t g t c t g t C G C C a g t c c c c a a c g | 39 | 3.13 |
| D2-5 | 81 | T C C a g g t t g t a g c c g g c t g t c t g t C G C c a g t c c c*c*a*a*c*g | 39 | 1.71 |
| D2-6 | 82 | T C C a g g t t g t a g c c g g c t g t c t g t c G C c a g t c c c*c*a*a*c*g | 39 | 1.64 |

In Table 14, the mismatch nucleotide was underlined, and the nucleotide substituted to a bridged nucleic acid was shown in capitals. All bridged nucleic acids in Table 14 were LNA. An asterisk (*) was inserted between the corresponding two or more nucleotides at the position in which the phosphodiester bond moiety between two or more nucleotides was substituted to a phosphorothioate bond.

The structure of D2-2 is similar to the structure reported in Non-Patent Publication 1. The relative editing efficiency in which a value of AUC of D2-2 was defined as a basis (1.00) was calculated, and shown in Table 14. Here, AUC in the case where Tris-EDTA (Tris-HCl 10 mM, EDTA 1 mM, pH7.4, hereinafter referred to as "TE") was used as a negative control was 123, and AUC of D2-2 was 64,642.

As shown in Table 14, the relative editing efficiency of D2-1 was 0.13, that of D2-3 was 4.94, that of D2-4 was 3.13, that of D2-5 was 1.71, and that of D2-6 was 1.64. From these results, it was found that two nucleotides contained in the target nucleotide sequence could be altered by the non-naturally occurring polynucleotide of the present invention.

Example 2-2: Study of Tree Nucleotides Substitution

An experiment measuring the editing efficiency of the target polynucleotide sequence was carried out by introducing the non-naturally occurring polynucleotide into 293-nLD3 cell.

In the Example, each of 4 kinds of non-naturally occurring polynucleotides of D3-1 to D3-5 was introduced into 293-nLD3 cell by a transfection method.

The nucleotide sequence, chain length and relative editing efficiency of each editing nucleic acid mentioned above are as given in Table 15.

[Table 15]

Table 1 5

| Editing Nucleic Acid | SEQ ID NO | Sequence | Chain Length (nt) | Relative Editing Efficiency |
|---|---|---|---|---|
| D3-1 | 83 | gacaccccgagattctgaaacaaa ctggacacacct | 36 | 0.23 |
| D3-2 | 84 | gacaccccgagattcTGAAAcaaa ctggacacacct | 36 | 1.00 |
| D3-3 | 85 | GacaccccgagattcTGAAAcaaa ctggacacacct | 36 | 3.44 |
| D3-4 | 86 | Ggagttacggacaccccgagattc TGAAAcaaactggaca | 40 | 2.47 |
| D3-5 | 87 | GGAgttacggacaccccgagattc TGAAAcaaact*g*g*a*c*a | 40 | 7.89 |

In Table 15, the mismatch nucleotide was underlined, and the nucleotide substituted to a bridged nucleic acid was shown in capitals. All bridged nucleic acid in Table 15 were LNA. An asterisk (*) was inserted between the corresponding two or more nucleotides at the position in which the phosphodiester bond moiety between two or more nucleotides was substituted to a phosphorothioate bond.

All structures shown in Table 15 are three nucleotides substitution, and such example has not been previously reported.

The relative editing efficiency in which a value of AUC of D3-2 was defined as a basis (1.00) was calculated and shown in Table 15. Here, AUC in the case where TE was used as a negative control was 119, and AUC of D3-2 was 14,436. As shown in Table 15, the relative editing efficiency of D3-1 was 0.23, that of D3-3 was 3.44, that of D3-4 was 2.47, and that of D3-5 was 7.89. From these results, it was found that three nucleotides contained in the target nucleotide sequence can be altered by the non-naturally occurring polynucleotide of the present invention.

Example 2-3: Study of Two Nucleotides Substitution (2)

An experiment measuring the editing efficiency of the target polynucleotide sequence was carried out by introducing the non-naturally occurring polynucleotides into 293-nLD4-1 cell, 293-nLD4-2 cell or 293-nLD4-3.

In the experiment using 293-nLD4-1 cell, each of 3 kinds of non-naturally occurring polynucleotides of D4-1-1 to D4-1-4 was introduced into 293-nLD4-1 cell by a transfection method.

In the Experiment using 293-nLD4-2 cell, each of 3 kinds of non-naturally occurring polynucleotides of D4-2-1 to D4-2-4 was introduced into 293-nLD4-2 cell by a transfection method.

In the experiment using 293-nLD4-3 cell, each of 3 kinds of non-naturally occurring polynucleotides of D4-3-1 to D4-3-4 was introduced into 293-nLD4-3 cell by a transfection method.

The nucleotide sequence, chain length and relative editing efficiency of each editing nucleic acid mentioned above are as given in Table 16.

[Table 16]

Table 16

| Editing Nucleic Acid | SEQ ID NO | Sequence | Chain Length (nt) | Relative Editing Efficiency |
|---|---|---|---|---|
| D4-1-1 | 88 | aacaaactggacacacctcactgttcaagg acttggtccaggttgta | 47 | 0.67 |
| D4-1-2 | 89 | aacaaactggacacacctcactgtTcAagg actTgGtccaggttgta | 47 | 1.00 |

(continued)

| Editing Nucleic Acid | SEQ ID NO | Sequence | Chain Length (nt) | Relative Editing Efficiency |
|---|---|---|---|---|
| D4-1-3 | 90 | AacaaactggacacacctcactgtTcAagg<br>actTgGtccaggttgta | 47 | 3.19 |
| D4-1-4 | 91 | AACaaactggacacacctcactgtTcAagg<br>actTgGtccagg*t*t*g*t*a | 47 | 9.45 |
| D4-2-1 | 92 | ttctaaaacaaactggacacacctccctgt<br>taaaggacttggtccaggttgta | 53 | 0.04 |
| D4-2-2 | 93 | ttctaaaacaaactggacacacctCcCtgt<br>taaaggactTgGtccaggttgta | 53 | 1.00 |
| D4-2-3 | 94 | TtctaaaacaaactggacacacctCcCtgt<br>taaaggactTgGtccaggttgta | 53 | 2.83 |
| D4-2-4 | 95 | TTCtaaaacaaactggacacacctCcCtgt<br>taaaggactTgGtccagg*t*t*g*t*a | 53 | 3.87 |
| D4-3-1 | 96 | ggagttacggacaccccgagattctgaaac<br>aaactggacacacctccctgttaaaggac | 59 | 0.00 |
| D4-3-2 | 97 | ggagttacggacaccccgagattcTgAaac<br>aaactggacacacctCcCtgttaaaggac | 59 | 1.00 |
| D4-3-3 | 98 | GgagttacggacaccccgagattcTgAaac<br>aaactggacacacctCcCtgttaaaggac | 59 | 1.76 |
| D4-3-4 | 99 | GGAgttacggacaccccgagattcTgAaac<br>aaactggacacacctCcCtgttaa*a*g*g*a*<br>c | 59 | 4.70 |

In Table 16, the mismatch nucleotide was underlined, and the nucleotide substituted to a bridged nucleic acid was shown in capitals. All bridged nucleic acids in Table 16 were LNA. An asterisk (*) was inserted between the corresponding two or more nucleotides at the position in which the phosphodiester bond moiety between two or more nucleotides was substituted to a phosphorothioate bond.

All structures shown in Table 16 are two nucleotides substitution which is present in far position, and such example has not been previously reported.

In D4-1-1 to D4-1-4, there are eight nucleotides distance between two mismatch nucleotides against the target nucleotide sequence. The relative editing efficiency in which the value of AUC of D4-1-2 was defined as a basis (1.00) was calculated and shown in Table 16. Here, AUC in the case where TE was used as a negative control was 47.16, and AUC of D4-1-2 was 14904. As shown in Table 16, the relative editing efficiency of D4-1-1 was 0.67, that of D4-1-3 was 3.19, and that of D4-1-4 was 9.45.

In D4-2-1 to D4-2-4, there are 14 nucleotides distance between two mismatch nucleotides against the target nucleotide sequence. The relative editing efficiency in which the value of AUC of D4-2-2 was defined as a basis (1.00) was calculated and shown in Table 16. Here, AUC in the case where TE was used as a negative control was 47.16, and AUC of D4-2-2 was 63770. As shown in Table 16, the relative editing efficiency of D4-2-1 was 0.04, that of D4-2-3 was 2.83, and that of D4-2-4 was 3.87.

In D4-3-1 to D4-3-4, there are 20 nucleotides distance between two mismatch nucleotides against the target nucleotide sequence. The relative editing efficiency in which the value of AUC of D4-3-2 was defined as (1.00) was calculated and shown in Table 16. Here, AUC in the case where TE was used as a negative control was 47.16, and AUC of D4-3-2 was 104690. As shown in Table 16, the relative editing efficiency of D4-3-1 was 0.00, that of D4-3-3 was 1.76, and that of D4-3-4 was 4.70.

From these results, it was found that alteration sites do not need to be adjacent to each other when the modification of the nucleotide contained in the target nucleotide sequence are plural, and plural nucleotide contained in the target nucleotide sequence can be currently modified, even if the target nucleotides are far from each other by at least 20 nucleotides.

Example 2-4: Study of One Nucleotide Insertion

An experiment measuring the editing efficiency of target polynucleotide sequence by introducing the non-naturally occurring polynucleotide into 293-nLD5 cell.

In the Example, each of 4 kinds of non-naturally occurring polynucleotides of D5-2, D5-4, D5-5 and D5-6, and a non-naturally occurring polynucleotide D5-1 as a control was introduced into 293-nLD5 cell by a transfection method.

The nucleotide sequence, chain length and relative editing efficiency of each editing nucleic acid mentioned above are as given in Table 17.

[Table 17]

Table 17

| Editing Nucleic Acid | SEQ ID NO | Sequence | Chain Length (nt) | Relative Editing Efficiency |
|---|---|---|---|---|
| D5-1 | 100 | t c c t t t g g a t c g g a g t t a [c] g g a c a c c c c g a g a t t c | 35 | 0.33 |
| D5-2 | 101 | t c c t t t g g a t c g g a g t t A [c] G g a c a c c c c g a g a t t c | 35 | 1.00 |
| D5-4 | 102 | A g g a c a a t c c t t t g g a t c g g a g t t A [c] G g a c a c c c c g a g | 38 | 10. 44 |
| D5-5 | 103 | A G G a c a a t c c t t t g g a t c g g a g t t A [c] G g a c a c c*c*c*g*a*g | 38 | 16. 19 |
| D5-6 | 104 | A G G a c a a t c c t t t g g a t c g g a g t t A [C] G g a c a c c*c*c*g*a*g | 38 | 2.11 |

In Table 17, the mismatch nucleotide was underlined, and the nucleotide substituted to a bridged nucleic acid was shown in capitals. All bridged nucleic acids in Table 17 were LNA. An asterisk (*) was inserted between the corresponding two or more nucleotides at the position in which the phosphodiester bond moiety between two or more nucleotides was substituted to a phosphorothioate bond. Further, the nucleotide in [ ] shows a nucleotide for inserting a nucleotide complementary to the nucleotide in [ ] into the target nucleotide sequence.

The structure of D5-2 is similar to the structure reported in Non-Patent Publication 1. The relative editing efficiency when the value of AUC of D5-2 was defined as a basis (1.00) was calculated and shown in Table 17. Here, AUC in the case where Tris-EDTA (Tris-HCl 10 mM, EDTA 1 mM, pH7.4, hereinafter referred to as "TE") was used as a negative control was 672.3, and AUC of D5-2 was 75293.

As shown in Table 17, the relative editing efficiency of D5-1 as a control was 0.33, that of D5-4 was 10.44, that of D5-5 was 16.19, and that of D5-6 was 2.11. From these results, it was found that one desired nucleotide can be inserted into a deletion site contained in the target nucleotide sequence by the non-naturally occurring polynucleotide of the present invention.

Example 2-5: Study of Two Nucleotides Insertion

An experiment measuring the editing efficiency of the target polynucleotide sequence was carried out by introducing the non-naturally occurring polynucleotide into 293-nLD6 cell.

In the Example, each of 9 kinds of non-naturally occurring polynucleotides of D6-1 to D6-10 was introduced into 293-nLD6 cell by a transfection method.

The nucleotide sequence, chain length and relative editing efficiency of each editing nucleic acid mentioned above are as given in Table 18.

[Table 18]

Table 1 8

| Editing Nucleic Acid | SEQ ID NO | Sequence | Chain Length (nt) | Relative Editing Efficiency |
|---|---|---|---|---|
| D6-1 | 105 | cctgttcaaggacttggtccaggt<br>t[gt]agccggctgtct | 39 | 0.60 |
| D6-2 | 106 | cctgttcaaggacttggtccaggt<br>T[gt]Agccggctgtct | 39 | 1.00 |
| D6-3 | 107 | Cctgttcaaggacttggtccaggt<br>T[gt]Agccggctgtct | 39 | 12.62 |
| D6-4 | 108 | CCTgttcaaggacttggtccaggt<br>T[gt]Agccggc*t*g*t*c*t | 39 | 17.70 |
| D6-5 | 109 | CCTgttcaaggacttggtccaggt<br>T[GT]Agccggc*t*g*t*c*t | 39 | 2.29 |
| D6-6 | 110 | CCTgttcaaggacttggtccaggt<br>T[GT]agccggc*t*g*t*c*t | 39 | 17.52 |
| D6-7 | 111 | CCTgttcaaggacttggtccaggt<br>T[Gt]Agccggc*t*g*t*c*t | 39 | 40. 12 |
| D6-8 | 112 | CCTgttcaaggacttggtccaggt<br>T[gT]Agccggc*t*g*t*c*t | 39 | 24. 11 |
| D6-9 | 113 | CCTgttcaaggacttggtccaggt<br>t[GT]Agccggc*t*g*t*c*t | 39 | 7.66 |
| D6-10 | 114 | CCTgttcaaggacttggtccaggt<br>t[GT]agccggc*t*g*t*c*t | 39 | 17.37 |

In Table 18, the mismatch nucleotide was underlined, and the nucleotide substituted to a bridged nucleic acid was shown in capitals. All bridged nucleic acids in Table 18 were LNA. An asterisk (*) was inserted between the corresponding two or more nucleotides at the position in which the phosphodiester bond moiety between two or more nucleotides was substituted to a phosphorothioate bond. Further, the nucleotide in [ ] shows a nucleotide for inserting a nucleotide complementary to the nucleotide in [ ] into the target nucleotide sequence.

All the structure shown in Table 18 are two nucleotides insertion, and such example has not previously reported.

The relative editing efficiency in which the value of AUC of D6-2 was defined as a basis (1.00) was calculated and shown in Table 18. Here, AUC in the case where TE was used as a negative control was 158, and AUC of D3-2 was 4924. As shown in Table 18, the relative editing efficiency of D6-1 was 0.60, that of D6-3 was 12.62, that of D6-4 was 17.70, that of D6-5 was 2.29, that of D6-6 was 7.52, that of D6-7 was 40.12, that of D6-8 was 24.11, that of D6-9 was 7.66, and that of D6-10 was 17.37. From these results, it was found that a desired nucleotide can be inserted by the non-naturally occurring polynucleotide of the present invention even if deletion sites contained in the target nucleotide sequence were two.

Example 2-6: Study of One Nucleotide Deletion

An experiment measuring the editing efficiency of the target polynucleotide sequence was carried out by introducing the non-naturally occurring polynucleotide into 293-nLD7 cell.

In the Example, each of 3 kinds of non-naturally occurring polynucleotides of D7-1 to D7-4 was introduced into 293-nLD7 cell by a transfection method.

The nucleotide sequence, chain length and relative editing efficiency of each editing nucleic acid mentioned above are as given in Table 19.

[Table 19]

Table 19

| Editing Nucleic Acid | SEQ ID NO | Sequence | Chain Length (nt) | Relative Editing Efficiency |
|---|---|---|---|---|
| D7-1 | 115 | acaaactggacacacctccctgttc<br>xaaggacttggtc | 37 | 0.72 |
| D7-2 | 116 | acaaactggacacacctccctgttC<br>xAaggacttggtc | 37 | 1.00 |
| D7-3 | 117 | AcaaactggacacacctccctgttC<br>xAaggacttggtc | 37 | 2.10 |
| D7-4 | 118 | ACAaactggacacacctccctgttC<br>xAaggact*t*g*g*t*c | 37 | 11.31 |

In Table 19, the mismatch nucleotide was underlined, and the nucleotide substituted to a bridged nucleic acid was shown in capitals. The mismatch nucleotide x in Table 19 means that a nucleotide complementary to a nucleotide present in the target nucleotide sequence was deleted in the editing nucleic acid. All bridged nucleic acids in Table 19 were LNA. An asterisk (*) was inserted between the corresponding two or more nucleotides at the position in which the phosphodiester bond moiety between two or more nucleotides was substituted to a phosphorothioate bond.

All structures shown in Table 19 are one nucleotide deletion, and such example has not been previously reported. The relative editing efficiency in which the value of AUC of D7-2 was defined as a basis (1.00) was calculated and shown in Table 19. Here, AUC in the case where TE was used as a negative control was 9964, and AUC of D7-2 was 12715. As shown in Table 19, the relative editing efficiency of D7-1 was 0.72, that of D7-3 was 2.10, and that of D7-4 was 11.31. From these results, it was found that a desired nucleotide can be removed from the target nucleotide sequence by the non-naturally occurring polynucleotide of the present invention.

Example 2-7: Study of Two Nucleotides Deletion

An experiment measuring the editing efficiency of the target polynucleotide sequence was carried out by introduced the non-naturally occurring polynucleotide into 293-nLD8 cell.

In the Example, each of 3 kinds of non-naturally occurring polynucleotides of D8-1 to D8-4 was introduced into 293-nLD8 cell by a transfection method.

The nucleotide sequence, chain length and relative editing efficiency of each editing nucleic acid mentioned above are as given in Table 20.

[Table 20]

Table 20

| Editing Nucleic Acid | SEQ ID NO | Sequence | Chain Length (nt) | Relative Editing Efficiency |
|---|---|---|---|---|
| D8-1 | 119 | tcaggacaatcctttggatcggagt<br>xxtacggacacccc | 37 | 1.03 |
| D8-2 | 120 | tcaggacaatcctttggatcggagT<br>xxTacggacacccc | 37 | 1.00 |
| D8-3 | 121 | TcaggacaatcctttggatcggagT<br>xxTacggacacccc | 37 | 4.84 |
| D8-4 | 122 | TCAggacaatcctttggatcggagT<br>xxTacggac*a*c*c*c*c | 37 | 22.95 |

In Table 20, the mismatch nucleotide was underlined, and the nucleotide substituted to a bridged nucleic acid was

shown in capitals. The mismatch nucleotide xx in Table 20 means that two nucleotides complementary to nucleotides present in the target nucleotide sequence were deleted in the editing nucleic acid. All bridged nucleic acids in Table 20 were LNA. An asterisk (*) was inserted between the corresponding two or more nucleotides at the position in which the phosphodiester bond moiety between two or more nucleotides was substituted to a phosphorothioate bond.

All structures shown in Table 20 are two nucleotides deletion, and such example has not been previously reported. The relative editing efficiency in which the value of AUC of D7-2 was defined as a basis (1.00) was calculated and shown in Table 19. Here, AUC in the case where TE was used as a negative control was 1243, and AUC of D8-2 was 1303. As shown in Table 20, the relative editing efficiency of D8-1 was 1.03, that of D8-3 was 4.84, and that of D8-4 was 22.95. From these results, it was found that the desired plural nucleotides can be removed from the target nucleotide sequence by the non-naturally occurring polynucleotide of the present invention.

Example 2-8: Study of Four Nucleotides Insertion

An experiment measuring the editing efficiency of the target polynucleotide sequence was carried out by introducing the non-naturally occurring polynucleotide into 293-nLD9 cell.

In the Example, each of 4 kinds of the non-naturally occurring polynucleotides of D9-1, D9-2, D9-3, D9-5 and D9-6 was introduced into 293-nLD9 cell by a transfection method.

The nucleotide sequence, chain length and relative editing efficiency of each editing nucleic acid mentioned above are as given in Table 21.

[Table 21]

Table 2 1

| Editing Nucleic Acid | SEQ ID NO | Sequence | Chain Length (nt) | Relative Editing Efficiency |
|---|---|---|---|---|
| D9-1 | 123 | tcctttggatcggagtta[cgga]ca ccccgagattctgaa | 39 | 0.37 |
| D9-2 | 124 | tcctttggatcggagttA[cgga]Ca ccccgagattctgaa | 39 | 1.00 |
| D9-3 | 125 | TcctttggatcggagttA[cgga]Ca ccccgagattctgaa | 39 | 5.12 |
| D9-5 | 126 | AggacaatcctttggatcggagttA [cgga]Caccccgagatt | 41 | 7.02 |
| D9-6 | 127 | AGGacaatcctttggatcggagttA [cgga]Caccccg*a*g*a*t*t | 41 | 9.14 |

In Table 21, the mismatch nucleotide was underlined, and the nucleotide substituted to a bridged nucleic acid was shown in capitals. All bridged nucleic acids in Table 21 were LNA. An asterisk (*) was inserted between the corresponding two or more nucleotides at the position in which the phosphodiester bond moiety between two or more nucleotides was substituted to a phosphorothioate bond. Further, the nucleotide in [ ] shows a nucleotide for inserting a nucleotide complementary to the nucleotide in [ ] into the target nucleotide sequence.

The structure of D9-2 is similar to the structure reported in Non-Patent Publication 1. The relative editing efficiency in which the value of AUC of D9-2 was defined as a basis (1.00) was calculated and shown in Table 21. Here, AUC in the case where Tris-EDTA (Tris-HCl 10 mM, EDTA 1 mM, pH7.4, hereinafter referred to as "TE") was used as a negative control was 383.8, and AUC of D9-2 was 18543. As shown in Table 21, the relative editing efficiency of D9-1 was 0.37, that of D9-3 was 5.12, that of D9-5 was 7.02, and that of D9-6 was 3.14. From these results, it was found that a desired nucleotide can be inserted by the non-naturally occurring polynucleotide of the present invention, even if the deletion sites contained in the target nucleotide sequence were four.

It was found from the above Example 2-1 to Example 2-8 that two or more nucleotides on the target polynucleotide can be substituted, that one or more nucleotides can be inserted into the target polynucleotide, and that one or more nucleotides can be removed from the target polynucleotide by using the non-naturally occurring polynucleotide of the present invention.

C. Genome Editing Experiment Using 293-nLD1 Cell (2)

Using 293-nLD1 cell produced in the above mentioned "A. genome editing experimentation using 293-nLD1 cells (1)," the following genome editing experiments were carried out.

1. Introduction of Non-Naturally Occurring Polynucleotide into 293-nLD 1 Cell

293-nLD1 cells were seeded onto a 96 well plate (Nunc(registered trademark) MicroWell™ 96, Nunclon Delta-Treated, Flat-Bottom Microplate, Thermo Fisher Scientific Inc.) to grow up to $1x10^4$ cells, the cells were cultured in DMEM + 10% FBS medium under an environment of 5% $CO_2$ at 37°C. After 24 hours, 0.05 or 0.1 μg of non-naturally occurring polynucleotide was transfected according to a usual protocol using Lipofectamine 3000.

2. Measurement of Editing Efficiency and Evaluation of Editing Efficiency of Non-Naturally Occurring Polynucleotide by AUC Log

The measurement of the editing efficiency and evaluation of the editing efficiency were carried out according to the method described in the above "B. Genome Editing Experiment Using 293-nLD2 Cell, 293-nLD3 Cell, 293-nLD4-1 Cell, 293-nLD4-2 Cell, 293-nLD4-3 Cell, 293-nLD5 Cell, 293-nLD6 Cell, 293-nLD7 Cell, 293-nLD8 Cell or 293-nLD9 Cell."

3. Synthesis of Non-Naturally Occurring Polynucleotide

All the non-naturally occurring polynucleotides including a modification of a nucleic acid were synthesized in Gene Design Inc. (Japan), which were purified with a simple column or HPLC.

Example 3-1: Alteration of Target Nucleotide Sequence by Non-Naturally Occurring Polynucleotide Having Various Features

An experiment measuring the editing efficiency of the target polynucleotide sequence was carried out by introducing the non-naturally occurring polynucleotide into 293-nLD1 cell.

In the Example, each of non-naturally occurring polynucleotides shown in Table 31 was introduced into 293-nLD1 cell by a transfection method.

The nucleotide sequence, chain length and relative editing efficiency of each editing nucleic acid mentioned above are as given in Table 31.

[Table 31-1]

Table 31 - 1

| Editing Nucleic Acid | SEQ ID NO | Sequence | Chain Length (nt) | Relative Editing Efficiency |
|---|---|---|---|---|
| GEO-8 | 9 | G t t c a a g g a c t Tg G t c c a g g t t g t a | 25 | 1.00 |
| GEO-226 | 128 | G t t c a a g g a c t Tg G t c c a g g * t * t * g * t * ap | 25 | 4.00 |
| GEO-198 | 129 | G t t c a a g g a c t T g G* t * c * c * a * g * g * t * t * g * t * a | 25 | 4.08 |
| GEO-200 | 130 | G t t c a a g g a c t T g G t c c a g g t t g u * A<br>↑ | 25 | 4.02 |
| GEO-204 | 131 | G t t c a a g g a c t T g G t c c a g g t t * G (F) * T (F) * A | 25 | 4.14 |
| GEO-213 | 132 | G t t c a a g g a c t T g G t c c a g g t t g * t * A (MT) | 25 | 4.57 |
| GEO-390 | 133 | G * t * t c a a g g a c t Tg G t c c a g g t t g t * A | 25 | 4.85 |
| GEO-391 | 134 | G * t * t c a a g g a c t Tg G t c c a g g t t * g * t * A | 25 | 4.65 |
| GEO-392 | 135 | G * t * t c a a g g a c t Tg G t c c a g g t t g t * a | 25 | 5.29 |
| GEO-393 | 136 | G * t * t c a a g g a c t Tg G t c c a g g t t g * t * A | 25 | 4.49 |

(continued)

| Editing Nucleic Acid | SEQ ID NO | Sequence | Chain Length (nt) | Relative Editing Efficiency |
|---|---|---|---|---|
| GEO-394 | 137 | G * t * t c a a g g a c t Tg G t c c a g g * t * t * g * t * a | 25 | 4.00 |

[Table 31-2]

Table 31 - 2

| Editing Nucleic Acid | SEQ ID NO | Sequence | Chain Length (nt) | Relative Editing Efficiency |
|---|---|---|---|---|
| GEO-399 | 138 | G * u * u * c * a a g g a c t T g G t c c a g g t t * g * t * A<br>↑ ↑ | 25 | 4.19 |
| GEO-400 | 139 | G * u * u * c * a a g g a c t T g G t c c a g g t t g t * a<br>↑ ↑ | 25 | 4.56 |
| GEO-401 | 140 | G * u * u * c * a a g g a c t T g G t c c a g g t t g * t * A<br>↑ ↑ | 25 | 4.12 |
| GEO-427 | 141 | G * t * t c a a g g a c t T g G t c c a g g t t g t * a p | 25 | 4.45 |
| GEO-474 | 142 | G A C a c a c c t c c c t g t t c a a g g a c t T g G t c c a g g * t * t * g * t * a | 38 | 6.05 |
| GEO-597 | 143 | G A C a c a c c t c c c t g t t c a a g g a c t T g G t c c a g g * t * t * g * t * a p | 38 | 4.80 |
| GEO-651 | 144 | G t t c a a g g a c t T g G t c c a g g t t g t * (MP) a | 25 | 4.21 |

In Table 31, the mismatch nucleotide was underlined, and the nucleotide substituted to a bridged nucleic acid was shown in capitals. All bridged nucleic acids in Table 31 were LNA. As to the nucleotide subjected to RNA substitution, arrow was attached to the base symbol of the corresponding nucleotide. An asterisk (*) was inserted between the corresponding nucleotides at the position in which the phosphodiester bond moiety between nucleotides was substituted to a phosphorothioate bond. In the non-naturally occurring polynucleotide in which a 5' terminus and/or a 3' terminus was phosphorylated, "p" was attached to the 5' terminus and/or the 3' terminus.

Further, G(F) and T(F) in GEO-204 indicate that the both are subjected to 2'-Fluoro modification, A(MT) in GEO-213 indicates that it is subjected to 2'-O-Methyl modification, and t*(MP)a in GEO-651 indicates that a bond between t and a is a methyl phosphate bond.

As shown in Table 31, it was found that the non-naturally occurring polynucleotide of the present invention had 4 times or more higher editing efficiency than GEO-8 which was used as a basis.

Example 3-2: Alteration of Target Nucleotide Sequence by Non-Naturally Occurring Polynucleotide Accompanying Mismatch Nucleotide at 3' Terminus

An experiment measuring the editing efficiency of the target polynucleotide sequence was carried out by introducing the non-naturally occurring polynucleotide into 293-nLDI cell.

In the Example, each of non-naturally occurring polynucleotides shown in Table 32 was introduced into 293-nLD1 cell by a transfection method.

The nucleotide sequence, chain length and relative editing efficiency of each editing nucleic acid mentioned above are as given in Table 32. Here, the chain length of an editing nucleic acid body and the chain length of an adapter were separately described in Table 32, whereas each sequence defined as SEQ ID NO is a combination of the editing nucleic acid body and the adapter.

[Table 31-1-1]

Table 32 - 1 - 1

| Editing Nucleic Acid | SEQ ID NO | Sequence | Chain Length of Edited Nucleic Acid (nt) | Chain Length of Adapter (nt) | Relative Editing Efficiency |
|---|---|---|---|---|---|
| GE0-8 | 9 | G t t c a a g g a c t T g G t c c a g g t t g t a | 25 | 0 | 1.00 |
| GEO-496 | 145 | G t t c a a g g a c t T g G t c c a g g t t g t a t a t | 25 | 3 | 1.48 |
| GE0-497 | 146 | G t t c a a g g a c t T g G t c c a g g t t g t a t a T | 25 | 3 | 1.85 |
| GE0-500 | 147 | G t t c a a g g a c t T g G t c c a g g t t g * t * a * t * a * t | 25 | 3 | 2.92 |
| GEO-508 | 148 | G t t c a a g g a c t T g G t c c a g g t t g * t * a * u * a * u<br>↑ ↑ ↑ | 25 | 3 | 2.40 |
| GE0-501 | 149 | G t t c a a g g a c t T g G t c c a g g t t g * t * a * t * a * T | 25 | 3 | 2.94 |

[Table 32-1-2]

Table 32-1 -2

| Editing Nucleic Acid | SEQ ID NO | Sequence | Chain Length of Edited Nucleic Acid (nt) | **Chain Length** of Adapter (nt) | Relative Editing Efficiency |
|---|---|---|---|---|---|
| GE0-498 | 150 | G t t c a a g g a c t T g G t c c a g g t t g t a<br>t a t a t a | 25 | 6 | 1.80 |
| GE0-499 | 151 | G t t c a a g g a c t T g G t c c a g g t t g t a<br>t a t a t A | 25 | 6 | 2.46 |
| GE0-502 | 152 | G t t c a a g g a c t T g G t c c a g g t t g t a<br>t * a * t * a * t * a | 25 | 6 | 4.36 |
| GE0-509 | 153 | G t t c a a g g a c t T g G t c c a g g t t g t a<br>u * a * u * a * u * a<br>↑ ↑ ↑ ↑ | 25 | 6 | 3.44 |
| GEO-503 | 154 | G t t c a a g g a c t T g G t c c a g g t t g t a<br>t * a * t * a * t * A | 25 | 6 | 5.49 |
| GE0-506 | 155 | G t t c a a g g a c t T g G t c c a g g t t g t a<br>t a t a u a<br>↑ ↑ ↑ | 25 | 6 | 2.33 |
| GE0-507 | 156 | G t t c a a g g a c t T g G t c c a g g t t g t a<br>t a t a u A<br>↑ ↑ ↑ | 25 | 6 | 2.80 |

[Table 32-2-1]

Table 3 2-2- 1

| Editing Nucleic Acid | SEQ ID NO | Sequence | Chain Length (nt) | Chain Length of Adapter (nt) | Relative Editing Efficiency |
|---|---|---|---|---|---|
| GEO-8 | 9 | G t t c a a g g a c t Tg G t c c a g g t t g t a | 25 | 0 | 1.00 |
| GEO-496 | 157 | G t t c a a g g a c t T g G t c c a g g t t g t a<br>t a t | 25 | 3 | 1.48 |
| GEO-562 | 158 | G t t c a a g g a c t T g G t c c a g g t t g t a<br>t a a t t a | 25 | 6 | 3.04 |
| GEO-554 | 159 | G t t c a a g g a c t T g G t c c a g g t t g t a<br>t a a t t a g t g | 25 | 9 | 3.69 |
| GE0-555 | 160 | G t t c a a g g a c t T g G t c c a g g t t g t a<br>t a a t t a g t g a g t | 25 | 12 | 4.31 |
| GE0-556 | 161 | G t t c a a g g a c t T g G t c c a g g t t g t a<br>t a a t t a g t g a g t g a t | 25 | 15 | 1.96 |

[Table 32-2-2]

Table 32-2-2

| Editing Nucleic Acid | SEQ ID NO | Sequence | Chain Length (nt) | Chain Length of Adapter (nt) | Relative Editing Efficiency |
|---|---|---|---|---|---|
| GE0-557 | 162 | GttcaaggactTgGtccaggttgta taattagtgagtgataac | 25 | 18 | 1. 55 |
| GE0-501 | 163 | GttcaaggactTgGtccaggttg*t*a*t*a*T | 25 | 3 | 2.94 |
| GE0-563 | 164 | GttcaaggactTgGtccaggttgta t*a*a*t*t*a | 25 | 6 | 4.49 |
| GEO-558 | 165 | GttcaaggactTgGtccaggttgta taat*t*a*g*t*g | 25 | 9 | 2.99 |
| GEO-559 | 166 | GttcaaggactTgGtccaggttgta taattag*t*g*a*g*t | 25 | 12 | 3.02 |
| GEO-560 | 167 | GttcaaggactTgGtccaggttgta taattagtga*g*t*g*a*t | 25 | 15 | 1.89 |
| GEO-561 | 168 | GttcaaggactTgGtccaggttgta taattagtgagtg*a*t*a*a*c | 25 | 18 | 2.36 |

[Table 32-3]

Table 32 - 3

| Editing Nucleic Acid | SEQ ID NO | Sequence | **Chain Length** (nt) | Chain Length of Adapter (nt) | Relative Editing Efficiency |
|---|---|---|---|---|---|
| GEO-8 | 9 | G t t c a a g g a c t T g G t c c a g g t t g t a | 25 | 0 | 1.00 |
| GE0-630 | 169 | G t t c a a g g a c t T g G t c c a g g * t * t * g * t * a t c c g g c | 25 | 6 | 2. 12 |
| GE0-631 | 170 | G t t c a a g g a c t T g G t c c a g g * t * t * g * t * a t a c g g c | 25 | 6 | 2.45 |
| GEO-632 | 171 | G t t c a a g g a c t T g G t c c a g g * t * t * g * t * a t a a g g c | 25 | 6 | 2.86 |
| GE0-680 | 172 | G t t c a a g g a c t T g G t c c a g g * t * t * g * t * a t a a t g c t g | 25 | 8 | 2.56 |
| GE0-681 | 173 | G t t c a a g g a c t T g G t c c a g g * t * t * g * t * a t a a t t c t g | 25 | 8 | 3.82 |

[Table 32-4]

Table 32 - 4

| Editing Nucleic Acid | SEQ ID NO | Sequence | Chain **Length** (nt) | Chain Length of Adapter (nt) | Relative Editing Efficiency |
|---|---|---|---|---|---|
| GE0-8 | 9 | G t t c a a g g a c t T g G t c c a g g t t g t a | 25 | 0 | 1.00 |
| GE0-632 | 174 | G t t c a a g g a c t T g G t c c a g g * t * t * g * t * a t a a g g c | 25 | 6 | 2.86 |
| GE0-707 | 175 | G t t c a a g g a c t T g G t c c a g g t t * g * t * a t a a g g c | 25 | 6 | 2.15 |
| GEO-708 | 176 | G t t c a a g g a c t T g G t c c a g g t t g * t * a t a a g g c | 25 | 6 | 1.62 |
| GE0-709 | 177 | G t t c a a g g a c t T g G t c c a g g t t g t * a t a a g g c | 25 | 6 | 1.72 |

[Table 32-5]

Table 32-5

| Editing Nucleic Acid | SEQ ID NO | Sequence | **Chain Length** (nt) | Relative Editing Efficiency |
|---|---|---|---|---|
| GEO-8 | 9 | G t t c a a g g a c t T g G t c c a g g t t g t a | 25 | 1.00 |
| GEO-687 | 178 | G t t c a a g g a c t T g G t c c a g g * t * t * g * t * a [t] g<br>c c g g c | 32 | 2. 14 |
| GE0-688 | 179 | G t t c a a g g a c t T g G t c c a g g * t * t * g * t * a [t t]<br>g c c g g c | 33 | 2. 50 |
| GE0-689 | 180 | G t t c a a g g a c t T g G t c c a g g * t * t * g * t * a [t t<br>t] g c c g g c | 34 | 3.36 |
| GE0-690 | 181 | G t t c a a g g a c t T g G t c c a g g * t * t * g * t * a [a] g<br>c c g g c | 32 | 2.89 |
| GE0-691 | 182 | G t t c a a g g a c t T g G t c c a g g * t * t * g * t * a [a a]<br>g c c g g c | 33 | 2.85 |
| GE0-692 | 183 | G t t c a a g g a c t T g G t c c a g g * t * t * g * t * a [a a<br>a] g c c g g c | 34 | 2. 18 |

[Table 32-6]

Table 32 - 6

| Editing Nucleic Acid | SEQ ID NO | Sequence | Chain Length (nt) | Chain Length of Adapter (nt) | Relative Editing Efficiency |
|---|---|---|---|---|---|
| GEO-8 | 9 | G t t c a a g g a c t T g G t c c a g g t t g t a | 25 | 0 | 1.00 |
| GE-O-737 | 184 | G t t c a a g g a c t T g G t c c a g g t t g t a<br>c g c g c g p<br>↑ ↑ ↑ ↑ ↑ ↑ | 25 | 6 | 2. 84 |
| GE-O-738 | 185 | G t t c a a g g a c t T g G t c c a g g t t g t a<br>c g c g c G<br>↑ ↑ ↑ ↑ ↑ ↑ | 25 | 6 | 2.52 |
| GE-O-747 | 186 | G t t c a a g g a c t T g G t c c a g g * t * t * g * t * a<br>c g c g c g<br>↑ ↑ ↑ ↑ ↑ ↑ | 25 | 6 | 2.34 |

**In** Table 32, the mismatch nucleotide was underlined, and the nucleotide substituted to a bridged nucleic acid was shown in capitals. All bridged nucleic acids in Table 32 were LNA. As to the nucleotide subjected to RNA substitution, arrow was attached to the base symbol of the corresponding nucleotide. An asterisk (*) was inserted between the corresponding nucleotides at the position in which the phosphodiester bond moiety between nucleotides was substituted to a phosphorothioate bond. In the non-naturally occurring polynucleotide in which a 5' terminus and/or a 3' terminus was phosphorylated, "p" was attached to the 5' terminus and/or the 3' terminus. Further, [t], [tt] and [ttt] respectively indicate that one, two and tree thymidine monophosphates were inserted, and [a], [aa] and [aaa] respectively indicate that one, two and tree adenosine monophosphates were inserted.

As shown in Table 32, it was found that the non-naturally occurring polynucleotide of the present invention had higher editing efficiency than that of GEO-8 which was used as a basis.

Example 3-3: Alteration of Target Nucleotide Sequence by Non-Naturally Occurring Polynucleotide Accompanying Adapter at 3' Terminus

An experiment measuring the editing efficiency of the target polynucleotide sequence was carried out by introducing the non-naturally occurring polynucleotide into 293-nLD1 cell.

In the Example, each of non-naturally occurring polynucleotides shown in Table 33 was introduced into 293-nLD1 cell by a transfection method.

The nucleotide sequence, chain length and relative editing efficiency of each editing nucleic acid mentioned above are as given in Table 33. Here, the chain length of an editing nucleic acid body and the chain length of an adapter were separately described in Table 33, whereas each sequence defined as SEQ ID NO is a combination of the editing nucleic acid body and the adapter.

[Table 33-1]

Table 33-1

| Editing Nucleic Acid | SEQ ID NO | Sequence | Chain Length (nt) | Chain Length of Adapter (nt) | Relative Editing Efficiency |
|---|---|---|---|---|---|
| GEO-8 | 9 | GttcaaggactTgGtccaggttgta | 25 | 0 | 1.00 |
| GE-O-602 | 187 | GttcaaggactTgGtccaggttgta<br>taattagtgagactucacuaauua<br>↑↑↑↑↑↑↑↑↑↑ | 25 | 24 | 3.23 |
| GE-O-603 | 188 | GttcaaggactTgGtccaggttgta<br>taattagtgagacuucacuaauua<br>↑↑↑↑↑↑↑↑↑↑↑↑↑↑ | 25 | 24 | 5.18 |
| GE-O-604 | 189 | GttcaaggactTgGtccaggttgta<br>uaauuagugagacttcactaatta<br>↑↑↑↑↑↑↑↑↑↑ | 25 | 24 | 1.00 |
| GE-O-605 | 190 | GttcaaggactTgGtccaggttgta<br>uaauuagugagacutcactaatta<br>↑↑↑↑↑↑↑↑↑↑↑↑↑↑ | 25 | 24 | 1.09 |
| GE-O-606 | 191 | GttcaaggactTgGtccaggttgta<br>uaauuagugagacuucacuaauua<br>↑↑↑↑↑↑↑↑↑↑↑↑↑↑↑↑↑↑↑↑↑↑↑↑ | 25 | 24 | 1.05 |
| GE-O-693 | 192 | GttcaaggactTgGtccaggttgta<br>taattagtgagtgcucucacucacuaauua<br>↑↑↑↑↑↑↑↑↑↑↑↑↑↑↑↑↑ | 25 | 30 | 2.10 |
| GE-O-694 | 193 | GttcaaggactTgGtccaggttgta<br>taattagugaccuaauua<br>↑↑↑↑↑↑↑↑↑↑↑ | 25 | 18 | 2.51 |

[Table 33-2]

Table 3 3 - 2

| Editing Nucleic Acid | SEQ ID NO | Sequence | Chain Length (nt) | Chain Length of Adapter (nt) | Relative Editing Efficiency |
|---|---|---|---|---|---|
| GEO-695 | 194 | GttcaaggactTgGtccaggttgta<br>taatuagcauua<br>↑↑↑↑↑↑↑↑ | 25 | 12 | 2.60 |
| GEO-696 | 195 | GttcaaggactTgGtccaggttgta<br>taattagtgagacacuucacuaauua<br>↑↑↑↑↑↑↑↑↑↑↑↑↑↑↑↑ | 25 | 26 | 4.75 |
| GEO-697 | 196 | GttcaaggactTgGtccaggttgta<br>taatt*a*g*t*g*agacuucacuaauua<br>↑↑↑↑↑↑↑↑↑↑↑↑↑↑ | 25 | 24 | 3.29 |
| GEO-701 | 197 | GttcaaggactTgGtccaggttgta<br>taattagtgagacac*u*u*c*a*cuaauua<br>↑↑↑↑↑ ↑ ↑ ↑ ↑ ↑↑↑↑↑↑↑ | 25 | 26 | 2.85 |
| GEO-702 | 198 | GttcaaggactTgGtccaggttgta<br>taattagtgagacacuucac*u*a*a*u*ua<br>↑↑↑↑↑↑↑↑↑↑ ↑ ↑ ↑ ↑ ↑↑ | 25 | 26 | 2.42 |
| GEO-703 | 199 | GttcaaggactTgGtccaggttgta<br>taattagtgagacacuucacuaauuap<br>↑↑↑↑↑↑↑↑↑↑↑↑↑↑↑↑ | 25 | 26 | 2.57 |
| GEO-822 | 200 | GttcaaggactTgGtccaggttgta<br>taattagtgagaucucacuaauua<br>↑↑↑↑↑↑↑↑↑↑↑↑↑↑ | 25 | 24 | 5.40 |
| GEO-823 | 201 | GttcaaggactTgGtccaggttgta<br>taattagtgagaccucacuaauua<br>↑↑↑↑↑↑↑↑↑↑↑↑↑↑ | 25 | 24 | 5.29 |

**In** Table 33, the mismatch nucleotide was underlined, and the nucleotide substituted to a bridged nucleic acid was shown in capitals. All bridged nucleic acids in Table 33 were LNA. As to the nucleotide subjected to RNA substitution, arrow was attached to the base symbol of the corresponding nucleotide. An asterisk (*) was inserted between the corresponding nucleotides at the position in which the phosphodiester bond moiety between nucleotides was substituted to a phosphorothioate bond. In the non-naturally occurring polynucleotide in which a 5' terminus and/or a 3' terminus was phosphorylated, "p" was attached to the 5' terminus and/or the 3' terminus.

Further, in each non-naturally occurring polynucleotide shown in Table 33, the nucleotides in the second row are an adapter, and each adapter forms a stem structure which may have a loop. FIG. 16A, FIG. 16B, FIG. 17A and FIG. 17B showed schematics of the stem structure of each non-naturally occurring polynucleotide.

As shown in Table 33, it was found that the non-naturally occurring polynucleotide of the present invention had an editing efficiency equal to or higher than that of GEO-8 which was used as a basis.

Example 3-4: Alteration of Target Nucleotide Sequence by Non-Naturally Occurring Polynucleotide of Embodiment (vi)

An experiment measuring the editing efficiency of the target polynucleotide sequence was carried out by introducing the non-naturally occurring polynucleotide into 293-nLDl cell.

In the Example, each of non-naturally occurring polynucleotides shown in Table 34 was introduced into 293-nLD1 cell by a transfection method.

The nucleotide sequence, chain length and relative editing efficiency of each editing nucleic acid mentioned above are as given in Table 34.

[Table 34]

Table3 4

| Editing Nucleic Acid | SEQ ID NO | Sequence | **Chain Length** (nt) | Relative Editing Efficiency |
|---|---|---|---|---|
| GEO-8 | 9 | G t t c a a g g a c t T<u>g</u> G t c c a g g t t g t a | 25 | 1.00 |
| GEO-232 | 202 | G t t c a a g g a c t t <u>G</u> g t c c a g g t t g t *A | 25 | 1.94 |
| GEO-237 | 203 | G t t c a a g g a c t t <u>G</u> g t c c a g g * t * t * g * t * a | 25 | 3.85 |
| GEO-671 | 204 | G t t c a a g g a c t t <u>G</u> g t c c a g g * t * t * g * t * a p | 25 | 2.13 |
| GEO-672 | 205 | G t t c a a g g a c t t <u>G</u> g t c c a * g * g * t * t * g * t * a p | 25 | 2.79 |

In Table 34, the mismatch nucleotide was underlined, and the nucleotide substituted to a bridged nucleic acid was shown in capitals. All bridged nucleic acids in Table 34 were LNA. An asterisk (*) was inserted between the corresponding nucleotides at the position in which the phosphodiester bond moiety between nucleotides was substituted to a phosphorothioate bond. In the non-naturally occurring polynucleotide in which a 5' terminus and/or a 3' terminus was phosphorylated, "p" was attached to the 5' terminus and/or the 3' terminus.

As shown in Table 34, it was found that the non-naturally occurring polynucleotide of the present invention had higher editing efficiency than that of GEO-8 which was used as a basis.

Example 3-5: Alteration of Target Nucleotide Sequence by Non-Naturally Occurring Polynucleotide in Which Nucleotide at 5' Terminus Is not LNA

An experiment measuring the editing efficiency of the target polynucleotide sequence was carried out by introducing the non-naturally occurring polynucleotide into 293-nLD1 cell.

In the Example, each of the non-naturally occurring polynucleotides shown in Table 41 was introduced into 293-nLD1 cell by a transfection method.

The nucleotide sequence, chain length and relative editing efficiency of each editing nucleic acid mentioned above are as given in Table 41.

[Table 41-1]

Table 41 - 1

| Editing Nucleic Acid | SEQ ID NO | Sequence | **Chain Length** (nt) | Relative Editing Efficiency |
|---|---|---|---|---|
| GEO-8 | 9 | G t t c a a g g a c t T g G t c c a g g t t g t a | 25 | 1.00 |
| GEO-323 | 206 | g * a * c * a * c * a c c t c c c t g t t c a a g g a c t T g G t c c a g g t t g t a | 38 | 1.38 |
| GEO-324 | 207 | g * a * c * a * c * a c c t c c c t G t t c a a g g a c t T g G t c c a g g t t g t a | 38 | 1.47 |
| GEO-649 | 208 | c g c g c g G t t c a a g g a c t T g G t c c a g g t t g t a<br>↑ ↑ ↑ ↑ ↑ ↑ | 31 | 1.55 |
| GEO-650 | 209 | c c c c c c G t t c a a g g a c t T g G t c c a g g t t g t a | 31 | 1.25 |
| GEO-653 | 210 | c g c g c g c g G t t c a a g g a c t T g G t c c a g g t t g t a<br>↑ ↑ ↑ ↑ ↑ ↑ ↑ ↑ | 33 | 1.29 |
| GEO-654 | 211 | c g c g c g c g c g G t t c a a g g a c t T g G t c c a g g t t g t a<br>↑ ↑ ↑ ↑ ↑ ↑ ↑ ↑ ↑ ↑ | 35 | 1.78 |
| GEO-656 | 212 | g c g c g c G t t c a a g g a c t T g G t c c a g g t t g t a<br>↑ ↑ ↑ ↑ ↑ ↑ | 31 | 1.34 |
| GEO-657 | 213 | c c c g g g G t t c a a g g a c t T g G t c c a g g t t g t a<br>↑ ↑ ↑ ↑ ↑ ↑ | 31 | 2.72 |

[Table 41-2]

Table 41 - 2

| Editing Nucleic Acid | SEQ ID NO | Sequence | Chain Length (nt) | Relative Editing Efficiency |
|---|---|---|---|---|
| GEO-8 | 9 | G t t c a a g g a c t Tg G t c c a g g t t g t a | 25 | 1.00 |
| GEO-827 | 214 | C t g t t c a a g g a c t Tg G t c c a g g t t g t a | 27 | 2.86 |
| GEO-828 | 215 | c T g t t c a a g g a c t Tg G t c c a g g t t g t a | 27 | 1.86 |
| GEO-831 | 216 | C c t g t t c a a g g a c t Tg G t c c a g g t t g t a | 28 | 1.28 |
| GEO-832 | 217 | c C t g t t c a a g g a c t Tg G t c c a g g t t g t a | 28 | 2.44 |
| GEO-833 | 218 | c c T g t t c a a g g a c t Tg G t c c a g g t t g t a | 28 | 1.03 |

In Table 41, the mismatch nucleotide was underlined, and the nucleotide substituted to a bridged nucleic acid was shown in capitals. All bridged nucleic acids used herein were LNA. As to the nucleotide subjected to RNA substitution, arrow was attached to the base symbol of the corresponding nucleotide. An asterisk (*) was inserted between the corresponding nucleotides at the position in which the phosphodiester bond moiety between nucleotides was substituted to a phosphorothioate bond.

All bridged nucleic acids in GEO-827 and GEO-831 were LNA. Further, mismatch nucleotides at 5' termini in or nearby GEO-649, GEO-650, GEO-653, GEO-654, GEO-656 and GEO-657 are a mismatch nucleotide for the purpose of improving editing efficiency, but not a mismatch nucleotide for the purpose of alteration.

As shown in Table 41, it was found that the non-naturally occurring polynucleotide of the present invention had higher editing efficiency than that of GEO-8 which was used as a basis.

Example 3-6: Alteration of Target Nucleotide Sequence by Non-Naturally Occurring Polynucleotide Accompanying Modification Compound at 5' Terminus or 3' Terminus

An experiment measuring the editing efficiency of the target polynucleotide sequence was carried out by introducing the non-naturally occurring polynucleotide into 293-nLD1 cell.

In the Example, each of non-naturally occurring polynucleotides shown in Table 42 and 43 was introduced into 293-nLD1 cell by a transfection method.

The nucleotide sequence, chain length and relative editing efficiency of each editing nucleic acid mentioned above are as given in Tables 42 and 43.

[Table 42]

Table4 2

| Editing Nucleic Acid | SEQ ID NO | Sequence | Chain Length (nt) | Relative Editing Efficiency |
|---|---|---|---|---|
| GEO-8 | 9 | G t t c a a g g a c t Tg G t c c a g g t t g t a | 25 | 1.00 |
| GEO-880 | 219 | "Biotin"G t t c a a g g a c t Tg G t c c a g g t t g t a | 25 | 1.38 |
| GEO-881 | 220 | "F A M" G t t c a a g g a c t Tg G t c c a g g t t g t a | 25 | 1.47 |
| GEO-789 | 221 | "F A M" * G t t c a a g g a c t Tg G t c c a g g t t g t a | 25 | 1.55 |

[Table 43]

Table 43

| Editing Nucleic Acid | SEQ ID NO | Sequence | Chain Length (nt) | Relative Editing Efficiency |
|---|---|---|---|---|
| GEO-8 | 9 | G t t c a a g g a c t Tg G t c c a g g t t g t a | 25 | 1.00 |
| GEO-882 | 222 | G t t c a a g g a c t T g G t c c a g g t t g t a" Biotin" | 25 | 2.38 |
| GEO-883 | 223 | G t t c a a g g a c t T g G t c c a g g t t g t a "F A M" | 25 | 3.77 |
| GEO-790 | 224 | G t t c a a g g a c t T g G t c c a g g t t g t a *"F A M" | 25 | 8.35 |
| GEO-791 | 225 | G t t c a a g g a c t Tg G t c c a g g * t * t * g * t * a " F A M " | 25 | 11.46 |
| GEO-817 | 226 | G t t c a a g g a c t T g G t c c a g g t t g t a"Pur" | 25 | 3.43 |
| GEO-818 | 227 | G t t c a a g g a c t Tg G t c c a g g t t g t a "C h o l " | 25 | 1.50 |
| GEO-819 | 228 | G t t c a a g g a c t Tg G t c c a g g t t g t a " D I G " | 25 | 1.50 |
| GEO-821 | 229 | G t t c a a g g a c t T g G t c c a g gt t g t a " I d T" | 25 | 4.39 |

In Tables 42 to 43, the mismatch nucleotide was underlined, and the nucleotide substituted to a bridged nucleic acid was shown in capitals. All bridged nucleic acids in Tables 42 to 43 were LNA. An asterisk (*) was inserted between the corresponding nucleotides at the position in which the phosphodiester bond moiety between nucleotides was substituted to a phosphorothioate bond.

Here, in Tables 42 to 43, Biotin is a biotin as a modification compound, FAM is a fluorescein (FAM) as a modification compound, Pur is a puromycin as a modification compound, Chol is a cholesterol as a modification compound, DIG is a digoxygenin as a modification compound, and IdT is an Inverted dT as a modification compound. Here, the molecular weight of the biotin (Biotin) is 244.31, the molecular weight of FAM is 376.32, the molecular weight of the puromycin (Pur) is 471.51, the molecular weight of the cholesterol (Chol) is 386.65, the molecular weight of the digoxygenin (DIG) is 414.31, and the molecular weight of the Inverted dT (IdT) is 304.2. Further, mismatch nucleotides at 5' termini in or nearby GEO-649, GEO-650, GEO-653, GEO-654, GEO-656 and GEO-657 are a mismatch nucleotide for the purpose of improving editing efficiency, but not a mismatch nucleotide for the purpose of alteration.

As shown in Tables 42 to 43, it was found that the non-naturally occurring polynucleotide of the present invention had higher editing efficiency than that of GEO-8 which was used as a basis.

Example 3-7: Study of Introduction of Phosphate Part Modification Bond to 5' Side or 3' Side

An experiment measuring the editing efficiency of the target polynucleotide sequence was carried out by introducing the non-naturally occurring polynucleotide accompanying a phosphate part modification bond at a 5' side or a 3' side from the mismatch nucleotide for the purpose of alteration into 293-nLD1 cell.

In the Example, each of the non-naturally occurring polynucleotides shown in Tables 44 to 45 was introduced into 293-nLD1 cell by a transfection method.

The nucleotide sequence, chain length and relative editing efficiency of each editing nucleic acid mentioned above are as given in Tables 44 to 45.

[Table 44]

Table 44

| Editing Nucleic Acid | SEQ ID NO | Sequence | Chain Length (nt) | Relative Editing Efficiency |
|---|---|---|---|---|
| GEO-8 | 9 | G t t c a a g g a c t Tg G t c c a g g t t g t a | 25 | 1.00 |
| GEO-376 | 230 | G * t t * c a a g g a c t Tg G t c c a g g t t g t a | 25 | 1.51 |
| GEO-377 | 231 | G * t t * c a * a g g a c t Tg t c c a g g t t g t a | 25 | 1.47 |
| GEO-835 | 232 | G t t c a a g g * a * c t Tg G t c c a g g t t g t a | 25 | 1.59 |
| GEO-836 | 233 | G t t c a a * g * g a c t Tg G t c c a g g t t g t a | 25 | 1.74 |

(continued)

| Editing Nucleic Acid | SEQ ID NO | Sequence | Chain Length (nt) | Relative Editing Efficiency |
|---|---|---|---|---|
| GEO-837 | 234 | G t t c * a * a g g a c t Tg G t c c a g g t t g t a | 25 | 2.65 |

[Table 45]

Table4 5

| Editing Nucleic Acid | SEQ ID NO | Sequence | Chain Length (nt) | Relative Editing Efficiency |
|---|---|---|---|---|
| GEO-8 | 9 | G t t c a a g g a c t T g G t c c a g g t t g t a | 25 | 1.00 |
| GEO-129 | 235 | G t t c a a g g a c t Tg G t * c * c * a * g g t t g t a | 25 | 2.50 |
| GEO-371 | 236 | G t t c a a g g a c t Tg G t c c a g g t t * g t * a | 25 | 4.12 |
| GEO-372 | 237 | G t t c a a g g a c t Tg G t c c a g g * t t * g t * a | 25 | 2.82 |
| GEO-838 | 238 | G t t c a a g g a c t Tg G t * c * c a g g t t g t a | 25 | 1.51 |
| GEO-839 | 239 | G t t c a a g g a c t Tg G t c c * a * g g t t g t a | 25 | 8.78 |
| GEO-840 | 240 | G t t c a a g g a c t Tg G t c c a g* g* t t g t a | 25 | 5.42 |

In Tables 44 to 45, the mismatch nucleotide was underlined, and the nucleotide substituted to a bridged nucleic acid was shown in capitals. All bridged nucleic acids in Tables 44 to 45 were LNA. An asterisk (*) was inserted between the corresponding nucleotides at the position in which the phosphodiester bond moiety between nucleotides was substituted to a phosphorothioate bond.

As shown in Tables 44 to 45, it was found that the non-naturally occurring polynucleotide of the present invention had a higher editing efficiency than that of GEO-8 which was used as a basis.

INDUSTRIAL APPLICABILITY

The method using a non-naturally occurring polynucleotide of the present invention allows an alteration of a target nucleotide sequence only by an introduction of a polynucleotide without introducing a gene encoding a foreign nuclease or an exogenous nuclease into a cell, and the method can be applied to a genome-editing technology having extremely high safety, so that the method has an industrial applicability.

## Claims

1. A non-naturally occurring polynucleotide capable of specifically binding to a target nucleotide sequence for altering one or more nucleotides comprised in the target nucleotide sequence in an intracellular double-stranded DNA, wherein the non-naturally occurring polynucleotide:

   comprises one or more mismatch nucleotides against the target nucleotide sequence,
   has a bridged nucleic acid shown in at least any of the following (A) and (B), and further has features of (C) and (D):

      (A) one or more nucleotides adjacent to a 5' upstream side of the mismatch nucleotide are a bridged nucleic acid;
      (B) one or more nucleotides adjacent to a 3' downstream side of the mismatch nucleotide are a bridged nucleic acid;
      (C) a nucleotide at a 5' terminus is a bridged nucleic acid; and
      (D) the chain length is from 22 to 95 nucleotides.

2. The non-naturally occurring polynucleotide according to claim 1, wherein the non-naturally occurring polynucleotide further has the following feature of (E):

(E) a nucleotide at a 3' terminus is a bridged nucleic acid.

**3.** The non-naturally occurring polynucleotide according to claim 1 or 2, wherein the non-naturally occurring polynucleotide further has the following feature of (F):
(F) one or more nucleotides adjacent to a nucleotide at a 5' terminus are a bridged nucleic acid.

**4.** The non-naturally occurring polynucleotide according to any one of claims 1 to 3, wherein the non-naturally occurring polynucleotide further has the following feature of (G):
(G) one or more nucleotides adjacent to a nucleotide at a 3' terminus are a bridged nucleic acid.

**5.** The non-naturally occurring polynucleotide according to any one of claims 1 to 4, wherein the non-naturally occurring polynucleotide further has the following feature of (J):
(J) one or more phosphodiester bond moieties between the nucleotides are substituted to a phosphate part modification bond.

**6.** The non-naturally occurring polynucleotide according to claim 5, wherein the phosphate part modification bond comprises at least one selected from the group consisting of a phosphorothioate bond, a methyl phosphate bond, a boranophosphate bond and a mesyl phosphoramidate bond.

**7.** The non-naturally occurring polynucleotide according to any one of claims 1 to 6, wherein the non-naturally occurring polynucleotide further has the following feature of (M):
(M) one or more nucleotides placed between a nucleotide adjacent to a 5' upstream side of the mismatch nucleotide and a nucleotide at a 5' terminus, and placed at a distance of at least 1 nucleotide from both of the nucleotide adjacent to a 5' upstream side of the mismatch nucleotide and the nucleotide at a 5' terminus are a bridged nucleic acid.

**8.** The non-naturally occurring polynucleotide according to any one of claims 1 to 7, wherein the non-naturally occurring polynucleotide further has the following feature of (N):
(N) one or more nucleotides placed between a nucleotide adjacent to a 3' downstream side of the mismatch nucleotide and a nucleotide at a 3' terminus, and placed at a distance of at least 1 nucleotide from both of the nucleotide adjacent to a 3' downstream side of the mismatch nucleotide and the nucleotide at a 3' terminus are a bridged nucleic acid.

**9.** The non-naturally occurring polynucleotide according to any one of claims 1 to 8, wherein the non-naturally occurring polynucleotide further has the following features of (O) and/or (X2):

(O) a pentose in one or more nucleotides adjacent to the bridged nucleic acid at a 3' terminus is a ribose; and/or
(X2) a pentose in one or more nucleotides adjacent to the bridged nucleic acid at a 5' terminus is a ribose.

**10.** The non-naturally occurring polynucleotide according to any one of claims 1 to 9, wherein the non-naturally occurring polynucleotide further has the following feature of (P):
(P) the nucleotide at a 3' terminus is phosphorylated.

**11.** The non-naturally occurring polynucleotide according to any one of claims 1 to 10, wherein the bridged nucleic acid comprises at least one or more selected from the group consisting of LNA, AmNA, BNA N-H, BNA N-Me and ENA.

**12.** The non-naturally occurring polynucleotide according to any one of claims 1 to 11, wherein the non-naturally occurring polynucleotide further has the following feature of (X1):
(X1) a nucleotide at a 3' terminus and/or one or more nucleotides adjacent to the nucleotide at a 3' terminus is a nucleic acid modified at a 2' site.

**13.** The non-naturally occurring polynucleotide according to claim 12, wherein the nucleic acid modified at a 2' site comprises at least one selected from the group consisting of 2'-F, 2'-OMe, 2'-MOE and 2'-O-(2-carbamoylethyl).

**14.** The non-naturally occurring polynucleotide according to any one of claims 1 to 13, wherein the non-naturally occurring polynucleotide further has the following feature of (X4):
(X4) an adapter is added to a 3' terminus.

**15.** The non-naturally occurring polynucleotide according to claim 14, wherein the adapter has a function of inhibiting a mismatch repair of a host cell.

**16.** The non-naturally occurring polynucleotide according to claim 14 or 15, wherein the adapter has a function of preserving the non-naturally occurring polynucleotide from nuclease digestion.

**17.** The non-naturally occurring polynucleotide according to any one of claims 14 to 16, wherein the adapter has a function of giving higher editing efficiency than a non-naturally occurring polynucleotide defined by SEQ ID NO: 9 (described as "GEO-8").

**18.** The non-naturally occurring polynucleotide according to any one of claims 14 to 17, wherein the adapter is a nucleotide comprising a mismatch nucleotide.

**19.** The non-naturally occurring polynucleotide according to any one of claims 14 to 18, wherein the adapter is a nucleotide forming a stem structure which may have a loop.

**20.** The non-naturally occurring polynucleotide according to any one of claims 14 to 17, wherein the adapter is a compound other than a nucleotide modifying a terminus (described as "modification compound").

**21.** The non-naturally occurring polynucleotide according to claim 20, wherein the molecular weight of the modification compound is 2,000 or less.

**22.** The non-naturally occurring polynucleotide according to any one of claims 1 to 21, wherein the non-naturally occurring polynucleotide further has the following feature of (X5):
(X5) one or more nucleotides are inserted between the mismatch nucleotide and the nucleotide at a 3' terminus.

**23.** A non-naturally occurring polynucleotide capable of specifically binding to a target nucleotide sequence for altering one or more nucleotides comprised in the target nucleotide sequence in an intracellular double-stranded DNA, wherein the non-naturally occurring polynucleotide:

comprises one or more mismatch nucleotides against the target nucleotide sequence; and
has a bridged nucleic acid shown in at least any of the following (A) and (B), has a feature of (D), and further has one or more features selected from the group consisting of (H2), (Y1) and (Y3):

(A) one or more nucleotides adjacent to a 5' upstream side of the mismatch nucleotide are a bridged nucleic acid;
(B) one or more nucleotides adjacent to a 3' downstream side of the mismatch nucleotide are a bridged nucleic acid;
(D) the chain length is from 22 to 95 nucleotides;
(H2) a phosphodiester bond moiety between a nucleotide at a 5' terminus and one or more nucleotides adjacent to the nucleotide at a 5' terminus is substituted to a phosphate part modification bond;
(Y1) the nucleotide at a 5' terminus is a mismatch nucleotide, and one or more nucleotides adjacent to the nucleotide at a 5' terminus are a mismatch nucleotide; and
(Y3) an adapter is added to a 5' terminus.

**24.** The non-naturally occurring polynucleotide according to claim 23, wherein the adapter has a function of inhibiting a mismatch repair of a host cell.

**25.** The non-naturally occurring polynucleotide according to claim 23 or 24, wherein the adapter has a function of preserving the non-naturally occurring polynucleotide from nuclease digestion.

**26.** The non-naturally occurring polynucleotide according to any one of claims 23 to 25, wherein the adapter has a function of giving a higher editing efficiency than a non-naturally occurring polynucleotide defined by SEQ ID NO: 9 (GEO-8).

**27.** The non-naturally occurring polynucleotide according to any one of claims 23 to 26, wherein the adapter is a modification compound, and the molecular weight of the modification compound is 2,000 or less.

**28.** The non-naturally occurring polynucleotide according to any one of claims 23 to 27, wherein the non-naturally occurring polynucleotide further has the following feature of (Y2):
(Y2) a pentose in the nucleotide at a 5' terminus is a ribose, and a pentose in one or more nucleotides adjacent to the nucleotide at a 5' terminus is a ribose.

**29.** A non-naturally occurring polynucleotide capable of specifically binding to a target nucleotide sequence for altering one or more nucleotides comprised in the target nucleotide sequence in an intracellular double-stranded DNA, wherein the non-naturally occurring polynucleotide:

comprises one or more mismatch nucleotides against the target nucleotide sequence, and
has a bridged nucleic acid shown in at least any of the following (A) and (B), and further has features of (D) and (X7):

(A) one or more nucleotides adjacent to a 5' upstream side of the mismatch nucleotide is a bridged nucleic acid;
(B) one or more nucleotides adjacent to a 3' downstream side of the mismatch nucleotide are a bridged nucleic acid;
(D) the chain length is from 22 to 95 nucleotides; and
(X7) one or more nucleotides placed between the mismatch nucleotide and a nucleotide at a 5' terminus is a bridged nucleic acid.

**30.** The non-naturally occurring polynucleotide according to claim 29, wherein the bridged nucleic acid comprises at least one or more selected from the group consisting of LNA, AmNA, BNA N-H, BNA N-Me and ENA.

**31.** A non-naturally occurring polynucleotide capable of specifically binding to a target nucleotide sequence for altering one or more nucleotides comprised in the target nucleotide sequence in an intracellular double-stranded DNA, wherein the non-naturally occurring polynucleotide:

comprises one or more mismatch nucleotides against the target nucleotide sequence, and
has all features of the following (C), (D), (I) and (X6):

(C) a nucleotide at a 5' terminus is a bridged nucleic acid;
(D) the chain length is from 22 to 95 nucleotides;
(I) a phosphodiester bond moiety between a nucleotide at a 3' terminus and one or more nucleotides adjacent to the nucleotide at a 3' terminus is substituted to a phosphorothioate bond; and
(X6) the mismatch nucleotide is a bridged nucleic acid.

**32.** The non-naturally occurring polynucleotide according to claim 31, wherein the non-naturally occurring polynucleotide further has the following feature of (E):
(E) the nucleotide at a 3' terminus is a bridged nucleic acid.

**33.** The non-naturally occurring polynucleotide according to claim 31 or 32, wherein the non-naturally occurring polynucleotide further has the following feature of (P):
(P) the nucleotide at a 3' terminus is phosphorylated.

**34.** A kit for altering a target nucleotide sequence comprising the non-naturally occurring polynucleotide as defined in any one of claims 1 to 33.

**35.** A pharmaceutical composition comprising the non-naturally occurring polynucleotide as defined in any one of claims 1 to 33.

**36.** A method of altering one or more nucleotides comprised in a target nucleotide sequence in an intracellular double-stranded DNA, wherein:

the method comprises introducing a non-naturally occurring polynucleotide comprising one or more mismatch nucleotides against the target nucleotide sequence into a cell,
the alteration of the target nucleotide sequence comprises at least one or more selected from the group consisting of deletion, insertion and
substitution of one or more nucleotides of the target nucleotide sequence, and
the non-naturally occurring polynucleotide is the non-naturally occurring polynucleotide as defined in any one of claims 1 to 33.

**37.** The method according to claim 36, wherein the cell is a prokaryotic cell or a eukaryotic cell.

**38.** The method according to claim 37, wherein the eukaryotic cell is at least one selected from the group consisting of a plant cell, an insect cell and an animal cell.

[FIG. 1]

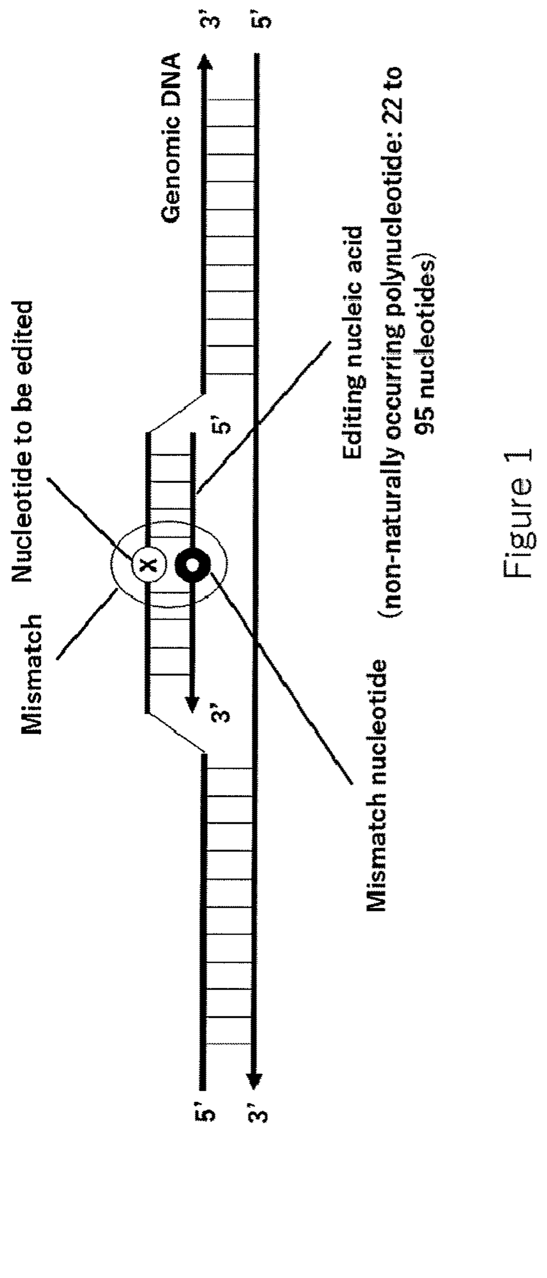
Mismatch
Nucleotide to be edited
Genomic DNA
Editing nucleic acid
(non-naturally occurring polynucleotide: 22 to 95 nucleotides)
Mismatch nucleotide
X
5'
3'
5'
3'
5'
3'
Figure 1

[FIG. 2]

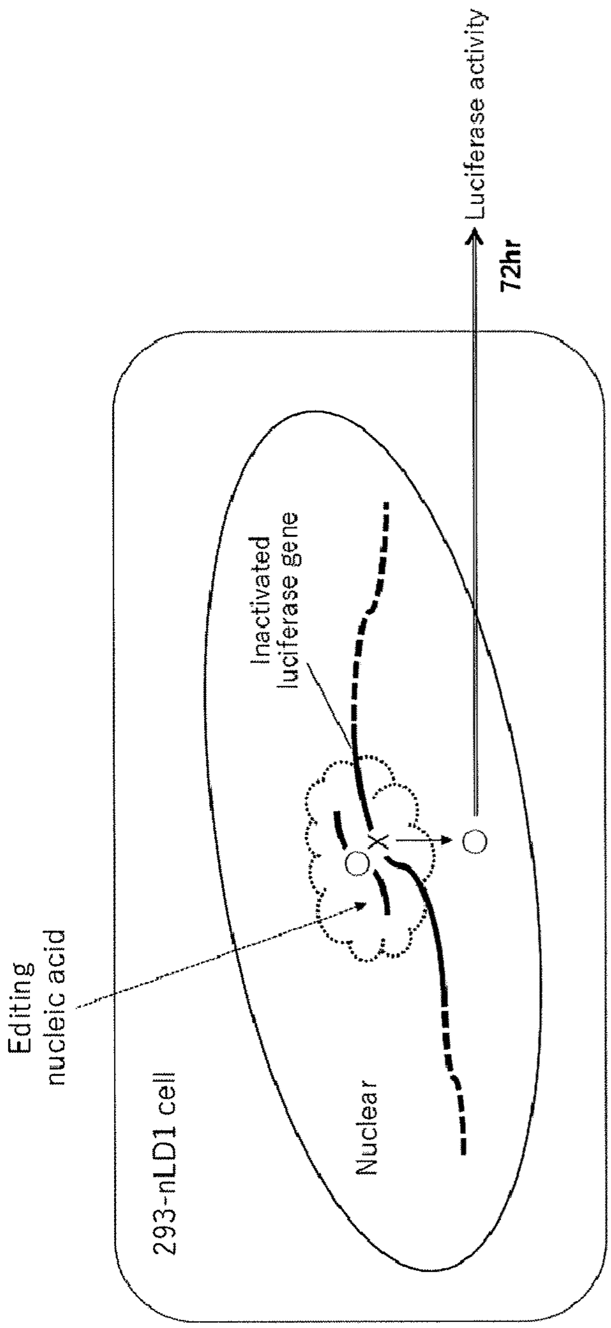


Figure 2

[FIG. 3A]

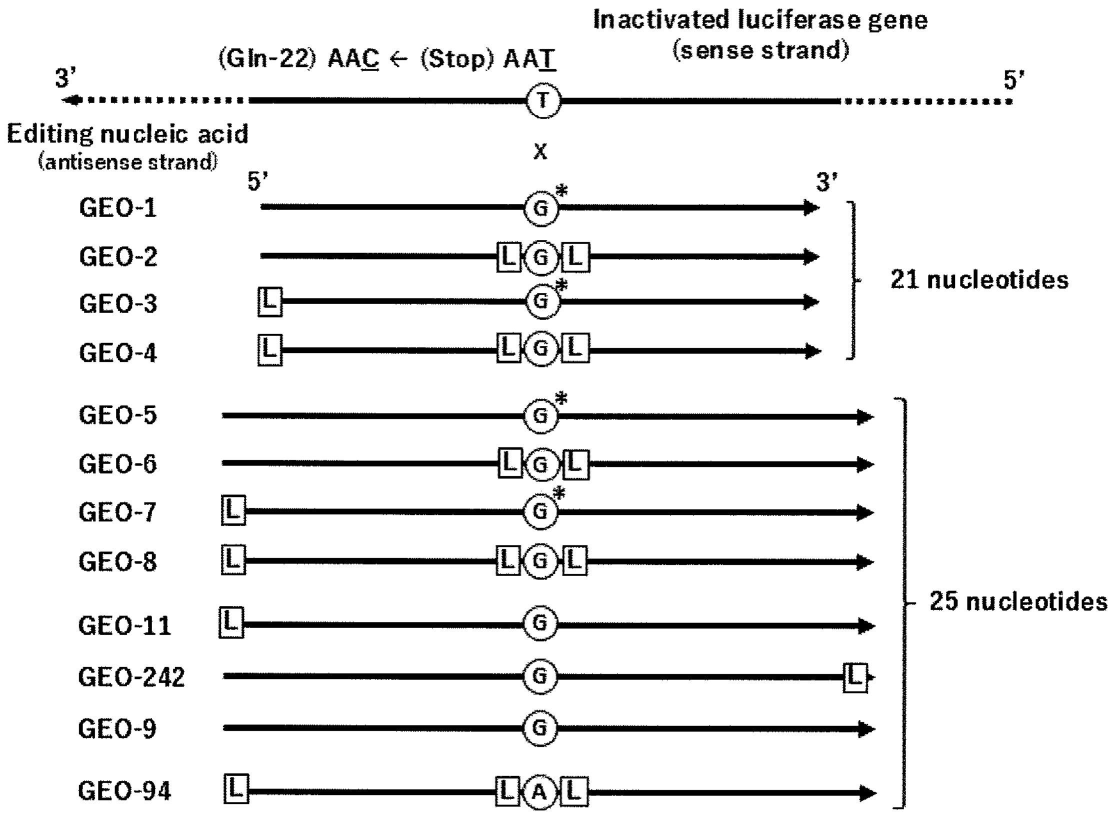


Figure 3A

[FIG. 3B]

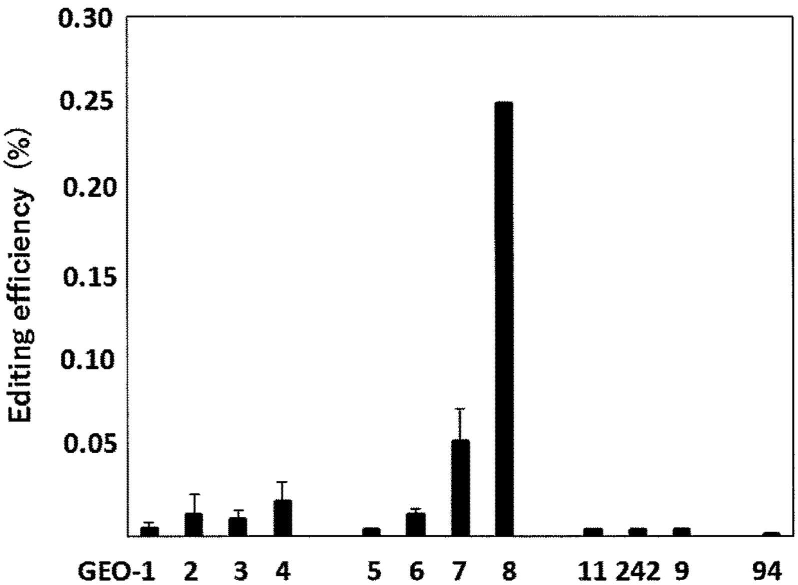


Figure 3B

[FIG. 4]

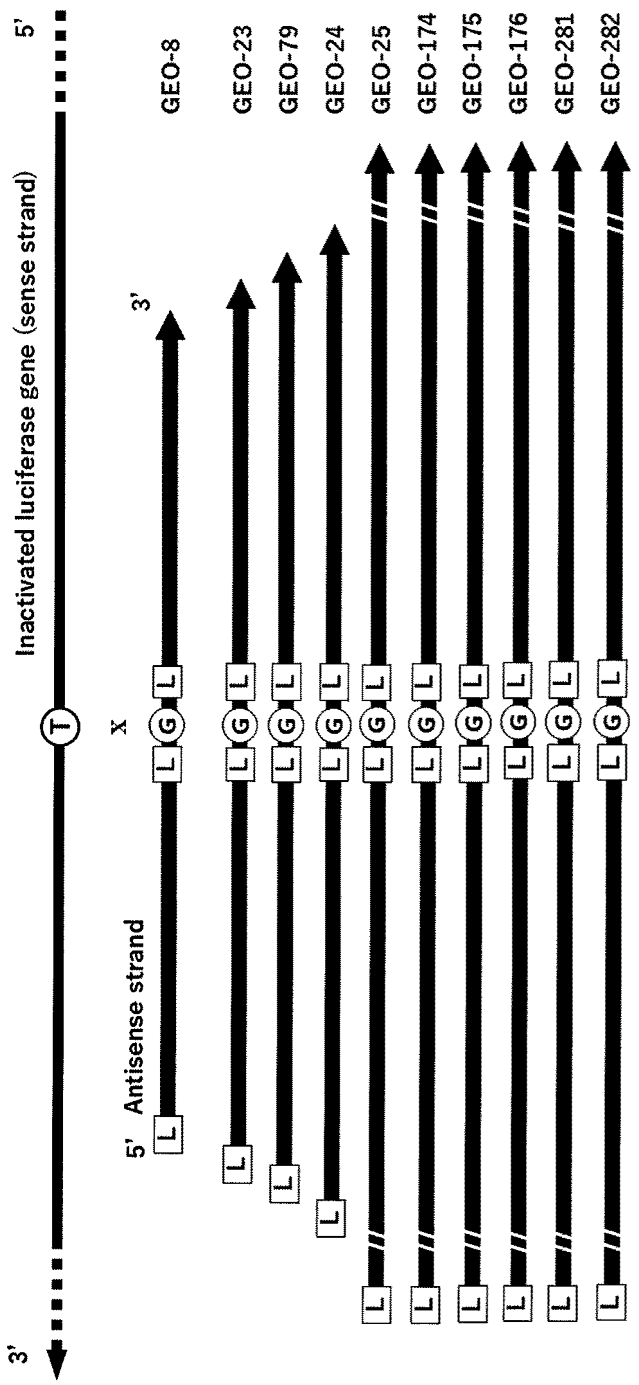


Figure 4

[FIG. 5]

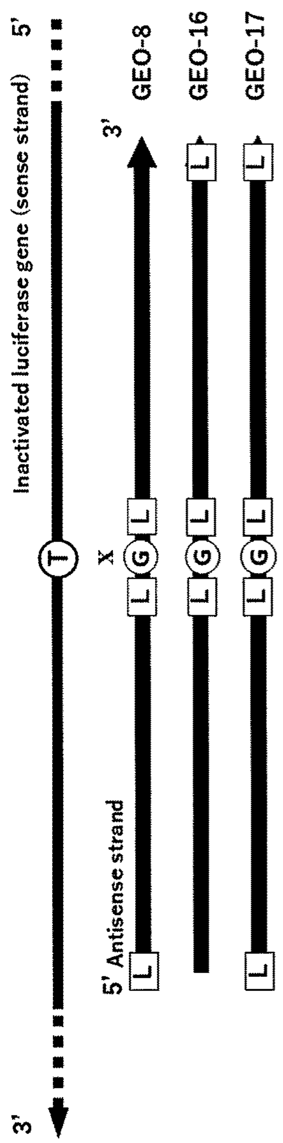


Figure 5

[FIG. 6]

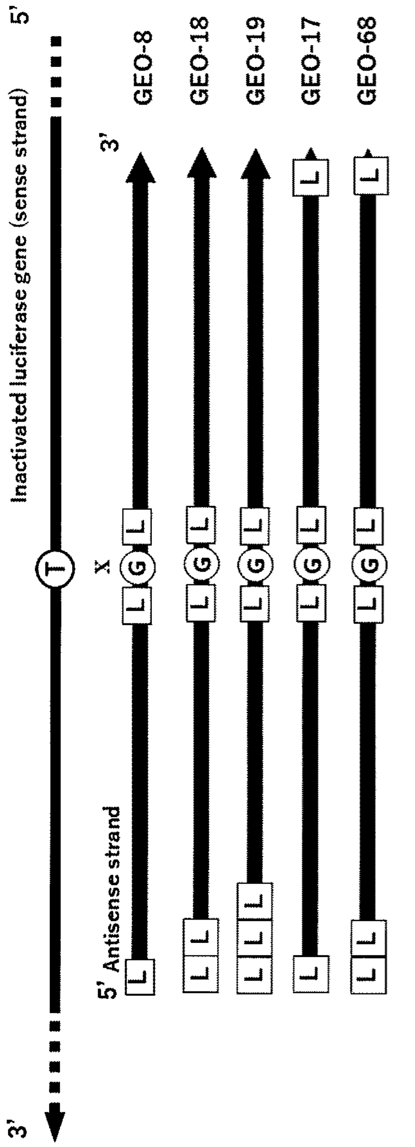


Figure 6

[FIG. 7]

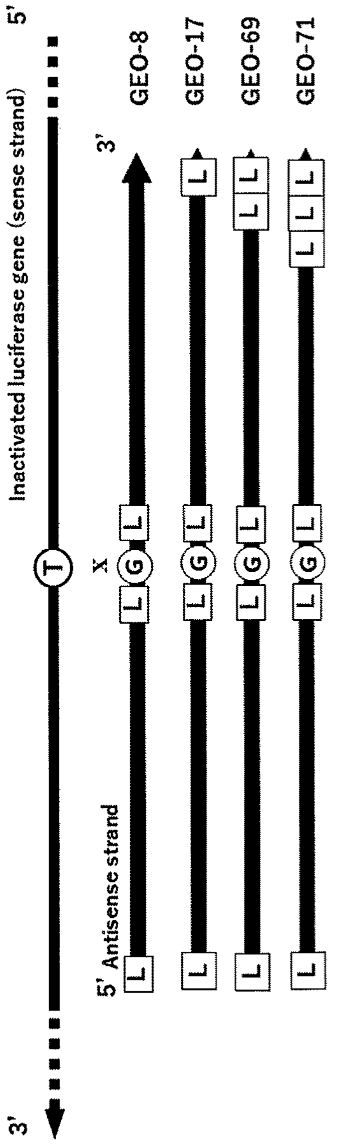


Figure 7

[FIG. 8A]

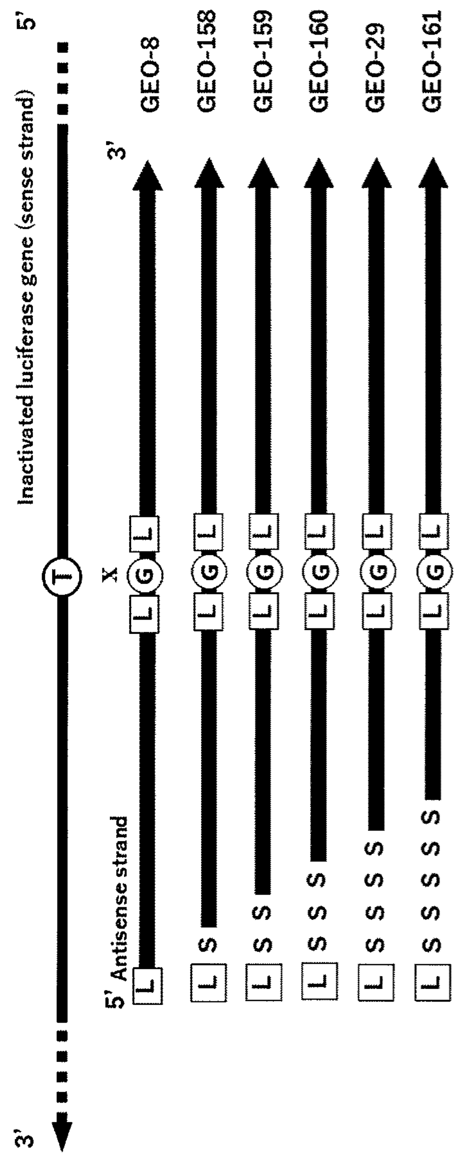


Figure 8A

[FIG. 8B]

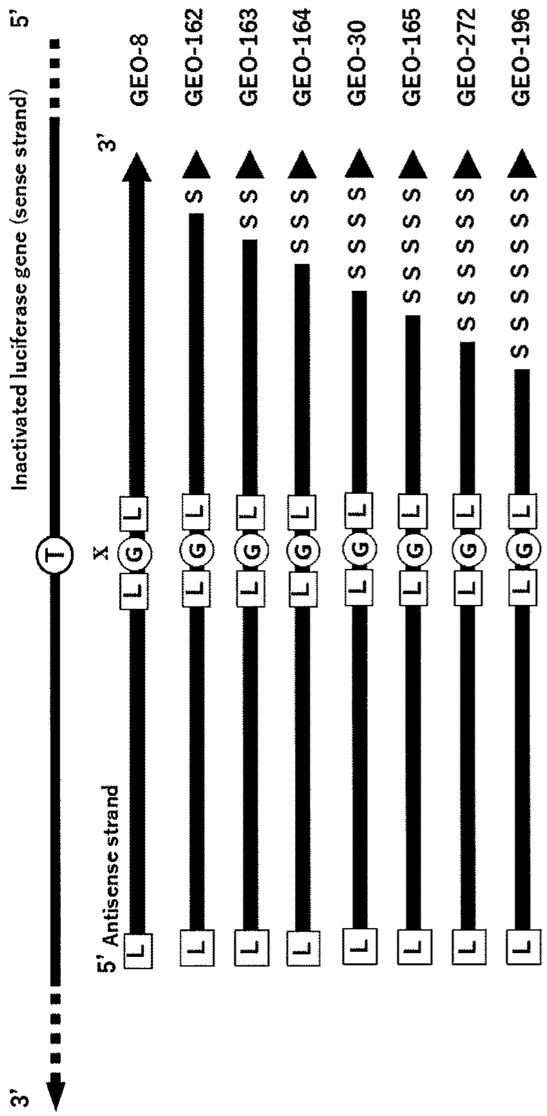


Figure 8B

[FIG. 8C]

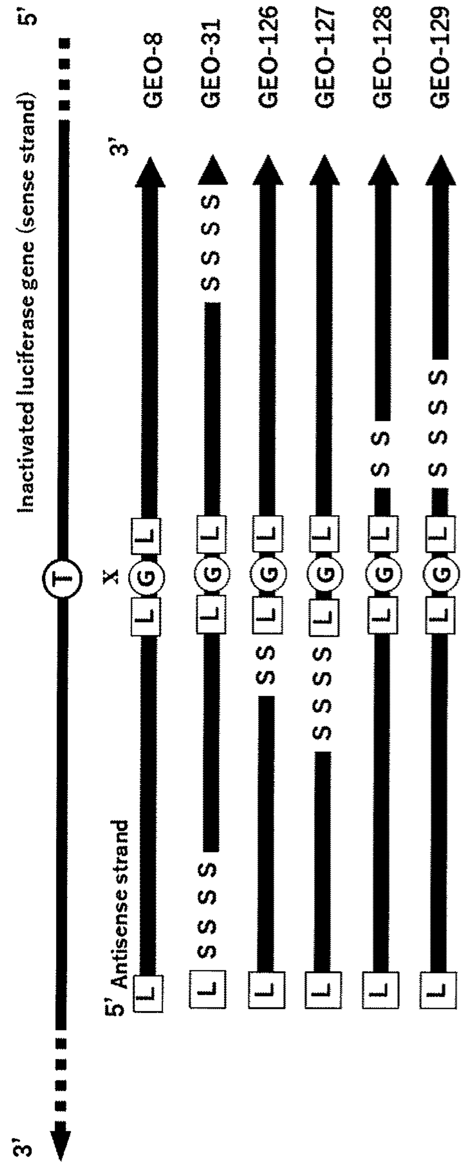


Figure 8C

[FIG. 9]

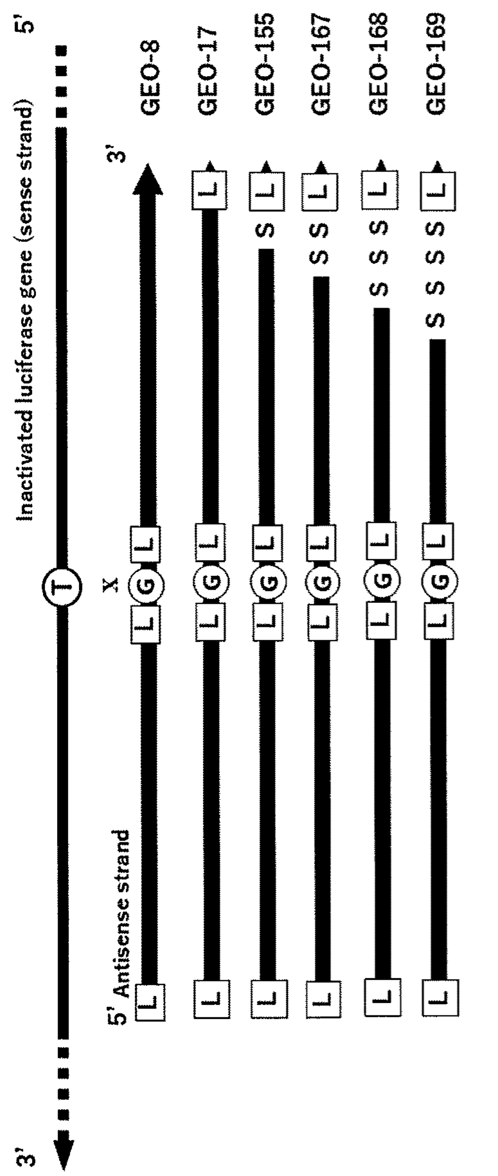


Figure 9

[FIG. 10A]

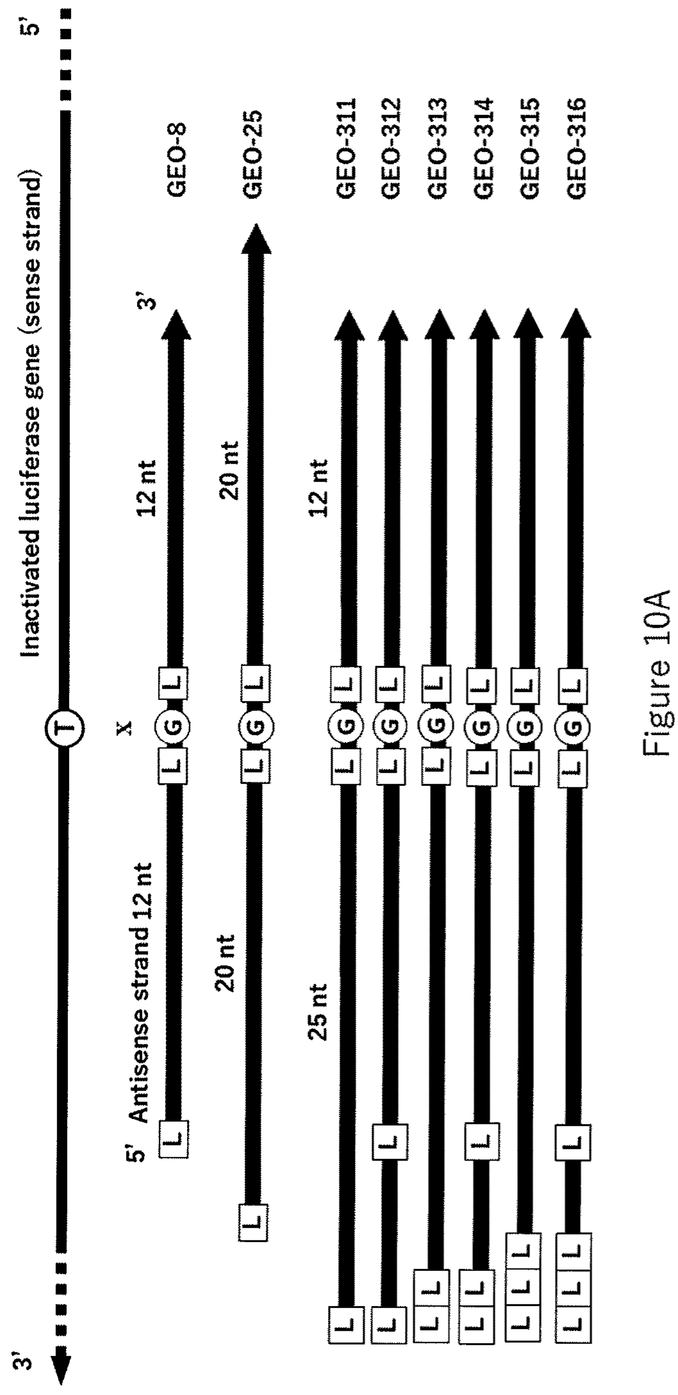
Inactivated luciferase gene (sense strand)
5'
3'
T
X
5' Antisense strand 12 nt
12 nt
3'
GEO-8
20 nt
20 nt
GEO-25
25 nt
12 nt
GEO-311
GEO-312
GEO-313
GEO-314
GEO-315
GEO-316
L
G
Figure 10A

[FIG. 10B]

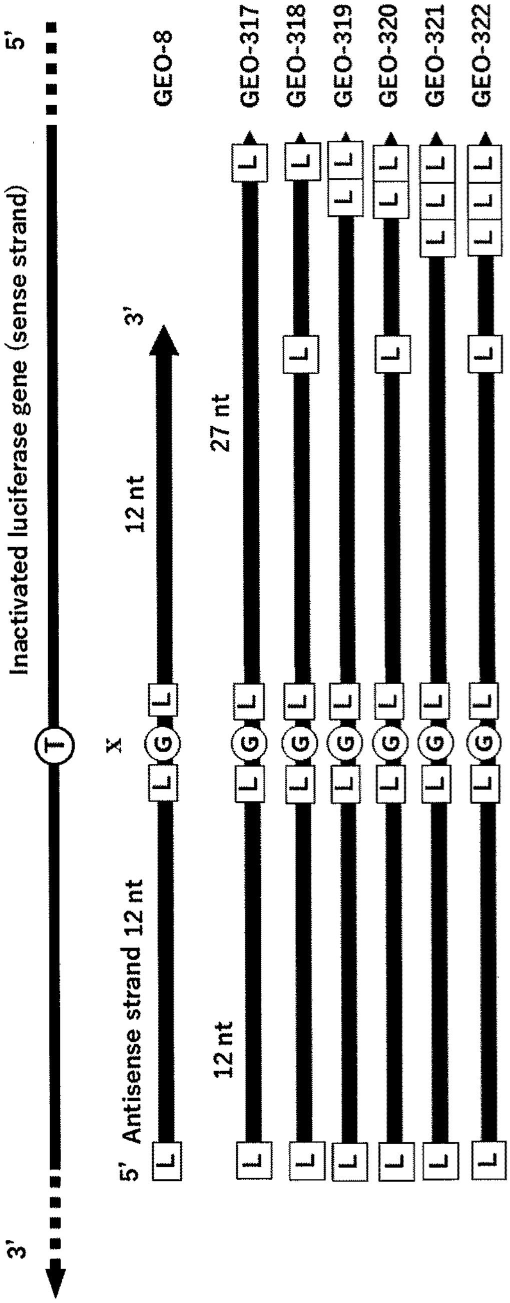


Figure 10B

[FIG. 11]

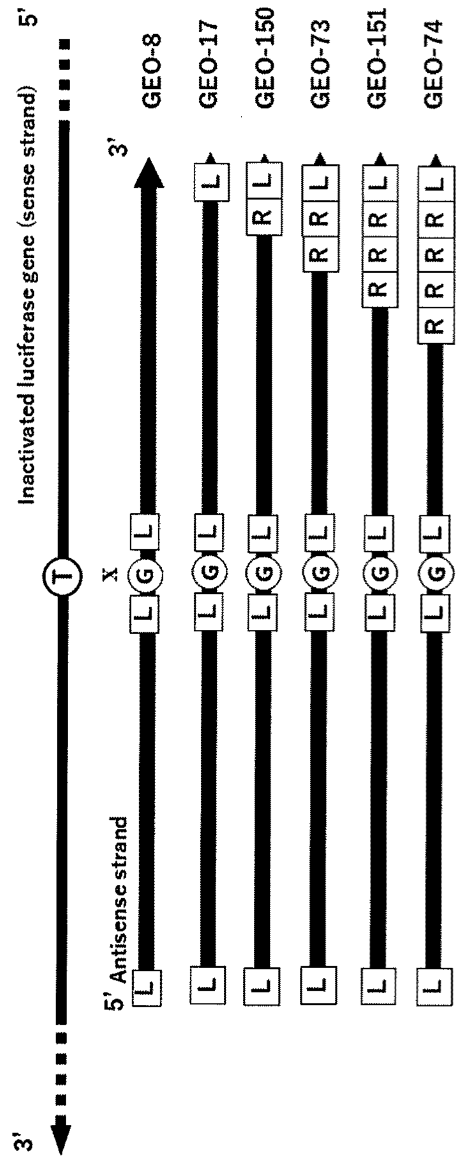


Figure 11

[FIG. 12]

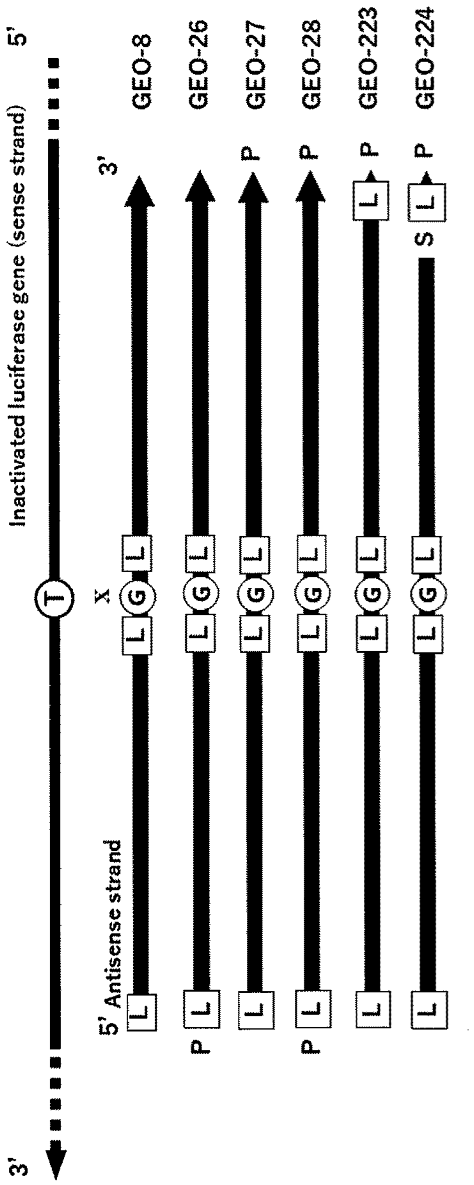


Figure 12

[FIG. 13]

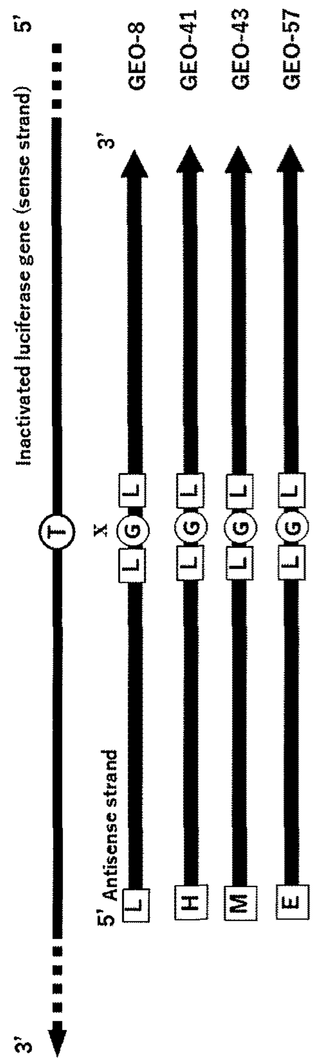


Figure 13

[FIG. 14]

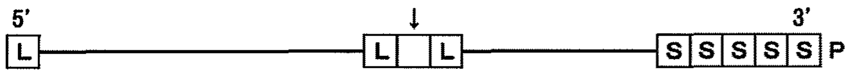


GEO−226 (chain lngth: 25nt)

Figure 14

[FIG. 15]

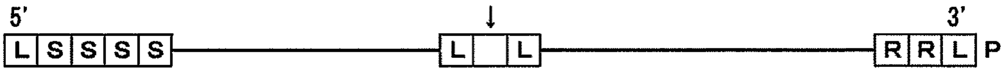


GEO−172 (chain length: 29nt)

Figure 15

[FIG. 16A]

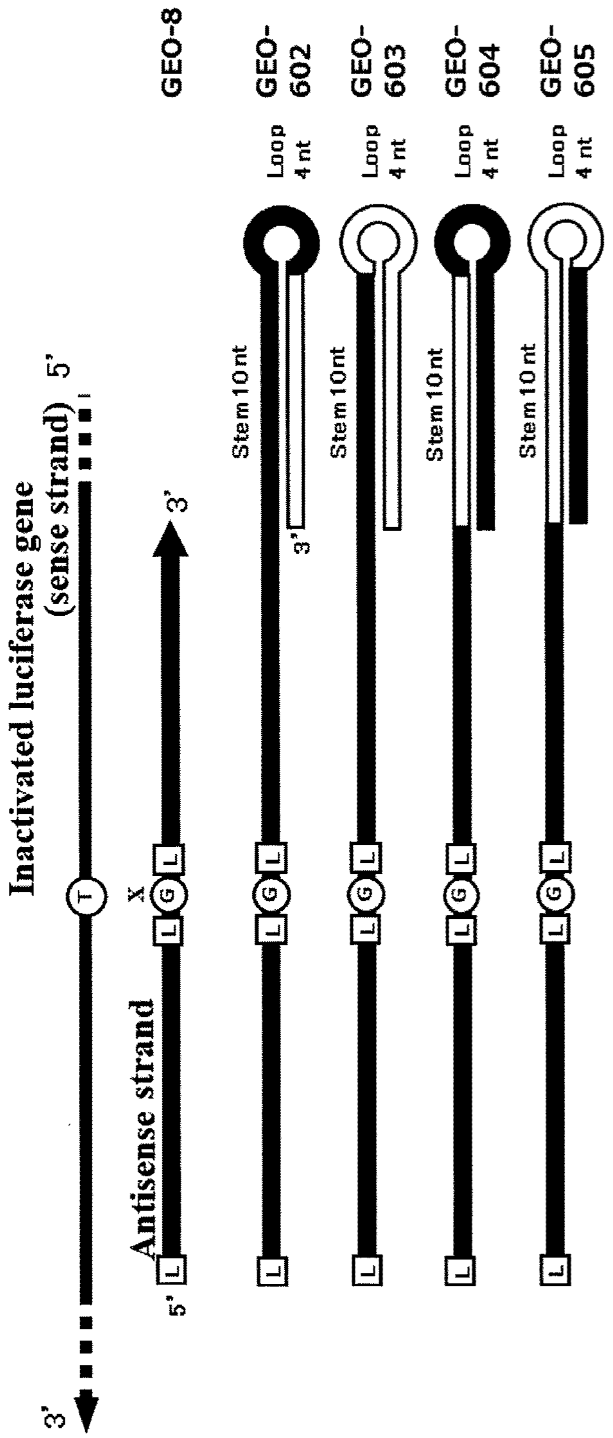


Figure 16A

[FIG. 16B]

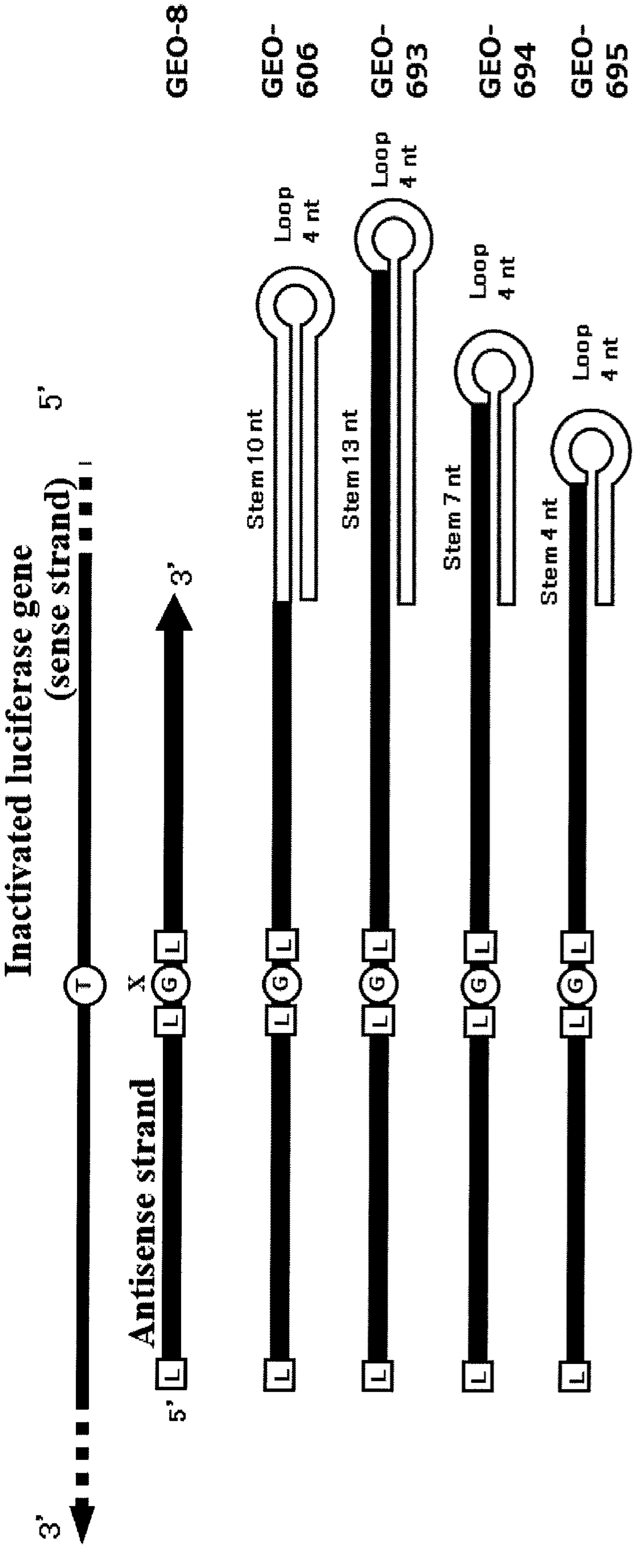


Figure 16B

[FIG. 17A]

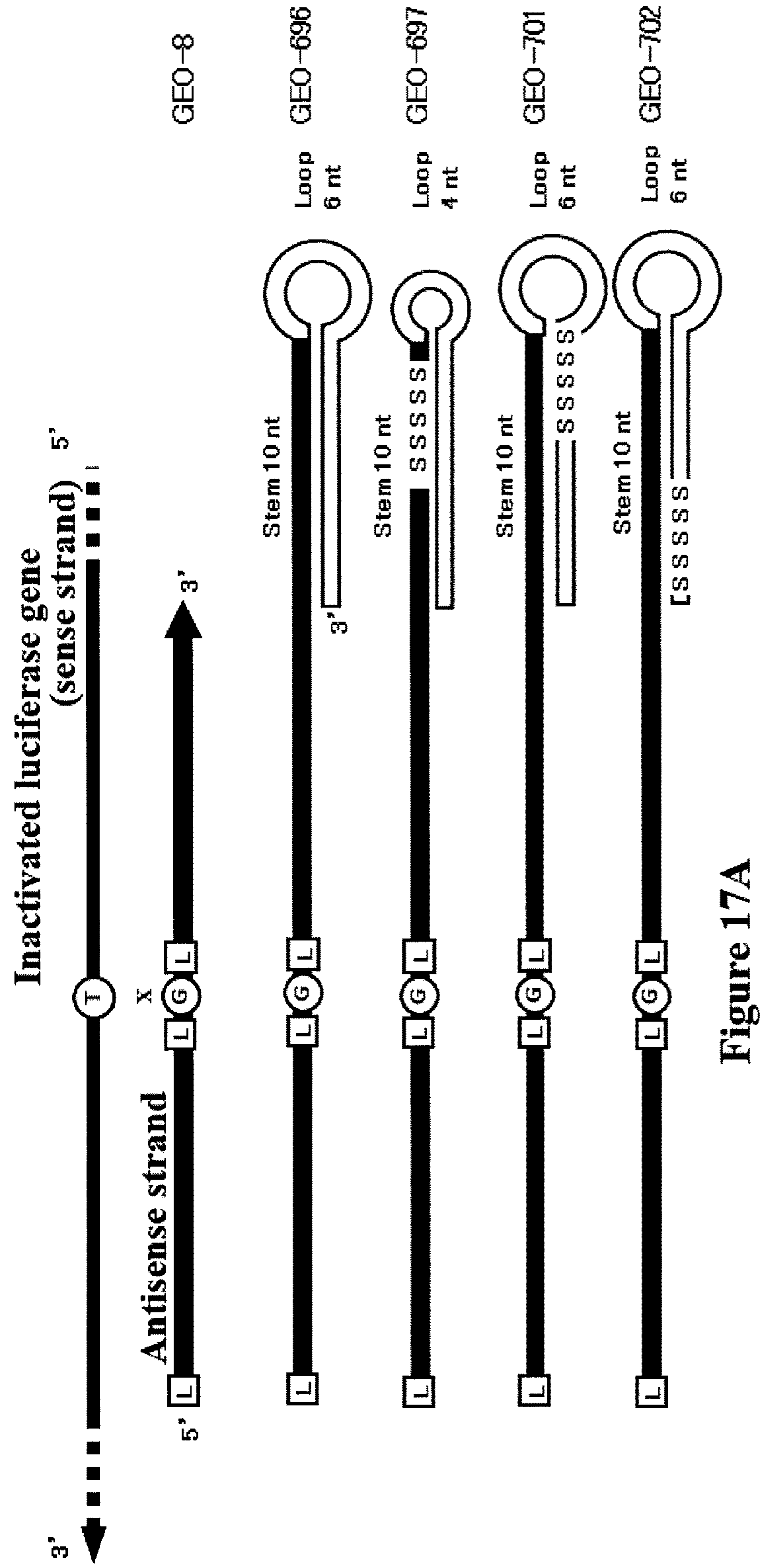


Figure 17A

[FIG. 17B]

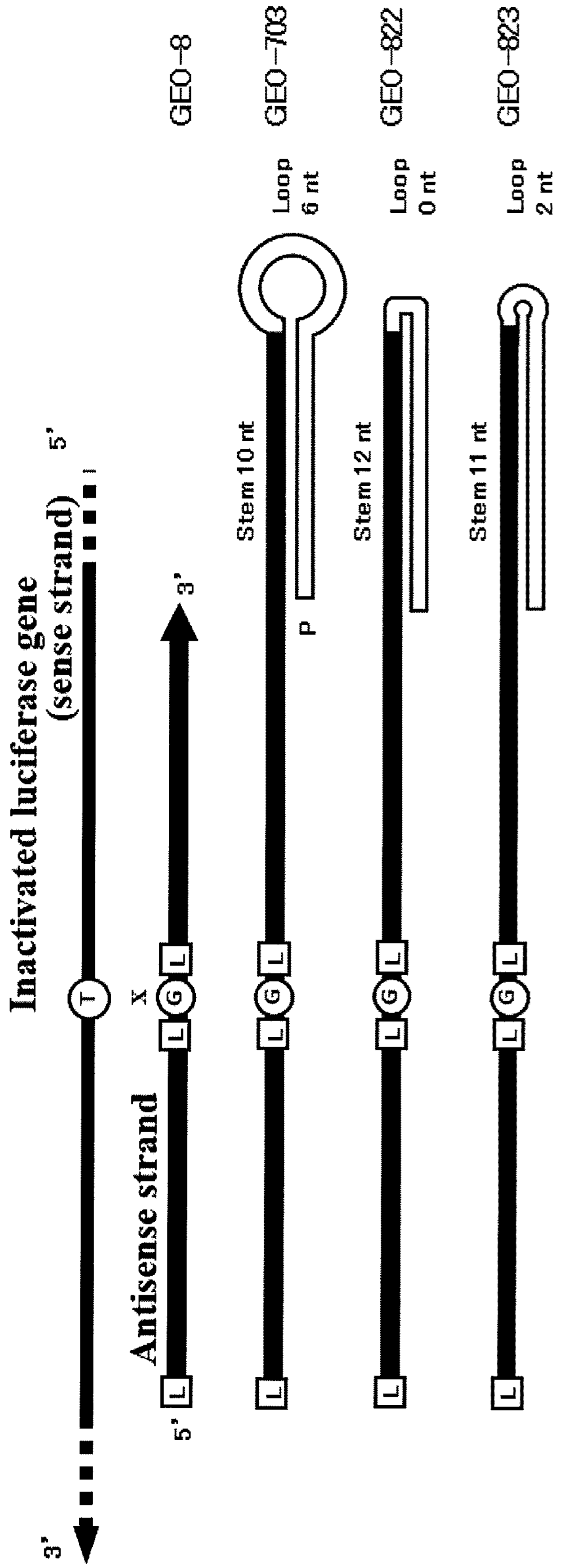


Figure 17B

## INTERNATIONAL SEARCH REPORT

International application No.
**PCT/JP2024/016395**

**A. CLASSIFICATION OF SUBJECT MATTER**

***C12N 15/11***(2006.01)i; ***A61K 31/712***(2006.01)i; ***A61K 31/7125***(2006.01)i; ***A61K 48/00***(2006.01)i; ***C12N 15/10***(2006.01)i
FI: C12N15/11 Z ZNA; C12N15/10 230Z; A61K48/00; A61K31/712; A61K31/7125

According to International Patent Classification (IPC) or to both national classification and IPC

**B. FIELDS SEARCHED**

Minimum documentation searched (classification system followed by classification symbols)

C12N15/11; A61K31/712; A61K31/7125; A61K48/00; C12N15/10

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2009-520495 A (KEYGENE N.V.) 28 May 2009 (2009-05-28) claims, paragraphs [0040], [0097] | 1-9, 11-14, 22, 23, 28-32, 34-38 |
| Y | claims, paragraphs [0040], [0097] | 10-22, 24-28, 33-38 |
| Y | JP 2011-062207 A (NATIONAL INSTITUTE OF AGROBIOLOGICAL SCIENCES) 31 March 2011 (2011-03-31) paragraphs [0077]-[0078] | 10-22, 24-28, 33-38 |
| Y | JP 2020-500035 A (BIO-RAD LABORATORIES, INC.) 09 January 2020 (2020-01-09) paragraph [0092] | 10-22, 24-28, 33-38 |
| Y | JP 2016-517273 A (SOMALOGIC, INC.) 16 June 2016 (2016-06-16) paragraph [0190] | 15-22, 24-28, 34-38 |
| Y | WO 2022/256440 A2 (ARBOR BIOTECHNOLOGIES, INC.) 08 December 2022 (2022-12-08) p. 71 | 15-22, 24-28, 34-38 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 June 2024** | **02 July 2024** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (July 2022)

**INTERNATIONAL SEARCH REPORT**

International application No.
**PCT/JP2024/016395**

**C. DOCUMENTS CONSIDERED TO BE RELEVANT**

| Category* | Citation of document, with indication, where appropriate, of the relevant passages | Relevant to claim No. |
|---|---|---|
| X | JP 2010-530750 A (KEYGENE N.V.) 16 September 2010 (2010-09-16) table 2, paragraph [0003], examples | 1, 4, 7, 8, 11, 22, 29, 30, 34 |
| Y | paragraph [0046] | 15-22, 24-28, 34-38 |
| X | US 2010/0068712 A1 (PRCHAL, Josef T.) 18 March 2010 (2010-03-18) tables 1, 7, 9 | 29, 30 |
| X | JP 2009-520499 A (KEYGENE N.V.) 28 May 2009 (2009-05-28) table 2, examples, paragraph [0004] | 1, 4, 7, 8, 11, 22, 29, 30, 34 |
| A | VAN RAVESTEYN, T. W. et al. LNA modification of single-stranded DNA oligonucleotides allows subtle gene modification in mismatch-repair-proficient cells. PNAS. 2016, vol. 113, no. 15, pp. 4122-4127 fig. 1 | 1-38 |

Form PCT/ISA/210 (second sheet) (July 2022)

**INTERNATIONAL SEARCH REPORT**

International application No.
**PCT/JP2024/016395**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

INTERNATIONAL SEARCH REPORT

International application No.
PCT/JP2024/016395

**Box No. III Observations where unity of invention is lacking (Continuation of item 3 of first sheet)**

This International Searching Authority found multiple inventions in this international application, as follows:

Document 1. JP 2009-520495 A (KEYGENE N.V.) 28 May 2009 (2009-05-28) claims, paragraphs [0040], [0097] & US 2009/0307805 A1 claims, paragraphs [0041], [0106]-[0107] & WO 2007/073154 A1 & EP 2333115 A1

(Invention 1) Claims 1-22 and 34-38

The invention in claim 1 of the present application discloses a "non-natural polynucleotide capable of specifically binding to a target nucleotide sequence for modifying one or a plurality of nucleotides contained in a target nucleotide sequence in double-stranded DNA in a cell, the non-natural polynucleotide comprising:

one or a plurality of mismatched nucleotides with respect to the target nucleotide sequence and

a crosslinked nucleic acid represented by at least any of (A) and (B) below, wherein the non-natural polynucleotide has the features of (C) and (D),

(A) the one or a plurality of nucleotides adjacent to the 5' upstream side of the mismatched nucleotide is a crosslinked nucleic acid

(B) the one or a plurality of nucleotides adjacent to the 3' downstream side of the mismatched nucleotide is a cross-linked nucleic acid

(C) the nucleotide at the 5' end is a cross-linked nucleic acid

(D) the strand length is 22-95 nucleotides" and there is a wording "for modifying one or a plurality of nucleotides contained in a target nucleotide sequence in double-stranded DNA in a cell" for specifying the use of non-natural polynucleotide. However, in general, in the invention of compounds such as polynucleotides, a limitation of use merely indicates the utility of the compound, and therefore the invention in the claims of the present application is understood as the invention of a polynucleotide with no use limitation. Document 1 discloses oligonucleotides for target modification of a double-stranded acceptor DNA sequence, comprising combining a donor oligonucleotide with a double-stranded acceptor DNA sequence.

The modification is also described as being in a cell, such as a plant cell, rodent cell, primate cell, human cell, or the like (claims). Also, document 1 discloses an oligonucleotide comprising an αL-LNA of sequence number 17 (considered as having each of the features set forth in each of claims 1, 2, 7, 8, 11, 22, 29, and 30 in the present invention) (paragraph [0097]).

Accordingly, claims 1 and 2 lack novelty in light of document 1, and thus do not have a special technical feature.

However, claim 3 dependent on claim 1 has a special technical feature of "a non-natural polynucleotide comprising one or a plurality of mismatched nucleotides with respect to a target nucleotide sequence, and

a crosslinked nucleic acid represented by at least any of (A) and (B) below, wherein the non-natural polynucleotide has the features of (C), (D), and (F).

(A) the one or a plurality of nucleotides adjacent to the 5' upstream side of the mismatched nucleotide is a crosslinked nucleic acid

(B) the one or a plurality of nucleotides adjacent to the 3' downstream side of the mismatched nucleotide is a cross-linked nucleic acid

(C) the nucleotide at the 5' end is a cross-linked nucleic acid

(D) the strand length is 22-95 nucleotides

(F) one or a plurality of nucleotides adjacent to a nucleotide at the 5' end is cross-linked nucleic acid." Therefore, claims 1-3 are classified as invention 1.

In addition, the invention in claims 4-22 and the parts referring to claims 1-22 in claims 34-38 are dependent on claim 1 and are inventively related to claim 1, and are thus classified as invention 1.

(Invention 1) Claims 23-28 and 34-38

It cannot be said that the invention in claims 23-28 and the parts referring to claims 23-28 in claims 34-38 have a special technical feature identical or corresponding to that of claim 3 classified as invention 1.

In addition, the invention in claims 23-28 and the parts referring to claims 23-28 in claims 34-38 are not dependent on claim 1. Furthermore, the invention in claims 23-28 and the parts referring to claims 23-28 in claims 34-38 are not substantially identical or equivalent to any of the claims classified as invention 1.

Thus, the invention in claims 23-28 and the parts referring to claims 23-28 in claims 34-38 cannot be classified as invention 1.

The invention in claims 23-28 and the parts referring to claims 23-28 in claims 34-38 has a special technical feature of "a non-natural polynucleotide comprising one or a plurality of mismatched nucleotides with respect to target nucleotide sequence and

**INTERNATIONAL SEARCH REPORT**

International application No. **PCT/JP2024/016395**

**Box No. III Observations where unity of invention is lacking (Continuation of item 3 of first sheet)**

a crosslinked nucleic acid represented by at least any of (A) and (B) below, wherein the non-natural polynucleotide has the features of (D), and at least one feature selected from the group consisting of (H2), (Y1), and (Y3).

(A) the one or a plurality of nucleotides adjacent to the 5' upstream side of said mismatched nucleotide is a crosslinked nucleic acid

(B) the one or a plurality of nucleotides adjacent to the 3' downstream side of said mismatched nucleotide is a cross-linked nucleic acid

(D) the strand length is 22-95 nucleotides

(H2) a phosphate diester bond between a nucleotide at the 5' end and one or a plurality of nucleotides adjacent to the nucleotide at the 5' end is replaced by a phosphate moiety bond

(Y1) a nucleotide at the 5' end is a mismatched nucleotide, and one or a plurality of nucleotides adjacent to the nucleotide at the 5' end are also mismatched nucleotides.

(Y3) an adapter is attached to the 5' end, and thus classified as invention 2.

Similarly, the invention in claims 29 and 30, and the parts referring to claims 29 and 30 in claims 34-38 are classified as invention 3, and the invention in claims 31-33 and the parts referring to claims 31-33 in claims 34-38 are classified as invention 4.

1. ☑ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☑ No protest accompanied the payment of additional search fees.

**INTERNATIONAL SEARCH REPORT**
**Information on patent family members**

International application No.
**PCT/JP2024/016395**

| Patent document cited in search report | Publication date (day/month/year) | Patent family member(s) | Publication date (day/month/year) |
|---|---|---|---|
| JP 2009-520495 A | 28 May 2009 | US 2009/0307805 A1<br>claims, paragraphs [0041], [0106]-[0107]<br>WO 2007/073154 A1<br>EP 2333115 A1 | |
| JP 2011-062207 A | 31 March 2011 | WO 2006/003638 A2<br>p. 51 | |
| JP 2020-500035 A | 09 January 2020 | US 2019/0071711 A1<br>paragraph [0113]<br>WO 2018/089860 A1<br>EP 3559231 A1 | |
| JP 2016-517273 A | 16 June 2016 | US 2014/0315986 A1<br>examples<br>WO 2014/159669 A2<br>EP 2970979 A2 | |
| WO 2022/256440 A2 | 08 December 2022 | US 2023/0023791 A1<br>EP 4347818 A2 | |
| JP 2010-530750 A | 16 September 2010 | US 2010/0223691 A1<br>paragraph [0048], examples<br>WO 2009/002150 A1<br>EP 2167660 A1 | |
| US 2010/0068712 A1 | 18 March 2010 | WO 2008/070370 A2 | |
| JP 2009-520499 A | 28 May 2009 | WO 2007/073170 A1<br>examples | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 6343605 B
- JP 6480647 B
- JP 6206893 B
- JP 5405121 B


### Non-patent literature cited in the description


- **THOMAS W. VAN RAVESTEYN** ; **MARLEEN DEKKER** ; **ALEXANDER FISH** ; **TITIA K. SIXMA** ; **ASTRID WOLTERS** ; **ROB J. DEKKER** ; **HEIN P. J. TE RIELE**. LNA modification of single-stranded DNA oligonucleotides allows subtle gene modification in mismatch-repair-proficient cells. *PNAS*, 2016, vol. 113 (15), 4122-4127
- **THOMAS W. VAN RAVESTEYN** ; **MARCOS ARRANZ DOLS** ; **WIETSKE PIETERS** ; **MARLEEN DEKKER** ; **HEIN TE RIELE**. Extensive trimming of short single-stranded DNA oligonucleotides during replication-coupled gene editing in mammalian cells.. *PLoS Genet*, 2020, vol. 16 (10), e1009041, https://doi.org/10.1371/journal.pgen.1009041